# EUROPEAN PATENT APPLICATION

(11) **EP 2 746 279 A1**
(43) Date of publication of application: **25.06.2014**
(21) Application number: 13196134.4
(22) Date of filing: 09.12.2013
(51) Int. Cl.: C07D 405/06, A01N 43/50, A01N 43/653

(54) **Fungicidal imidazolyl and triazolyl compounds**

(30) Priority: 19.12.2012 EP 12198116; 04.01.2013 EP 13150202
(71) Applicant: BASF SE, 67056 Ludwigshafen (DE)
(72) Inventor: Grammenos, Wassilios, 67071 Ludwigshafen (DE); Craig, Ian Robert, 67063 Ludwigshafen (DE); Boudet, Nadege, 69502 Hemsbach (DE); Müller, Bernd, 67227 Frankenthal (DE); Dietz, Jochen, 76227 Karlsruhe (DE); Lauterwasser, Erica May Wilson, 68199 Mannheim (DE); Lohmann, Jan Klaas, 67245 Lambsheim (DE); Grote, Thomas, 67157 Wachenheim (DE); Haden, Egon, 67346 Speyer (DE); Escribano Cuesta, Ana, 68167 Mannheim (DE)

(57) **Abstract**

The present invention relates to fungicidal imidazolyl and triazolyl compounds I, agrochemical compositions comprising them, to their use and to methods for combating phytopathogenic fungi. The present invention also relates to seeds treated with at least one such compound. Furthermore the invention relates to processes for preparing compounds of formula I as well as to specific intermediates that are obtained during the reaction sequence.

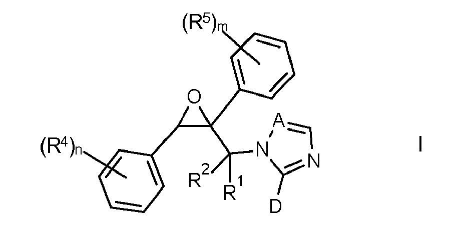

## Description

The present invention relates to fungicidal imidazolyl and triazolyl compounds, agrochemical compositions comprising them, to their use and to methods for combating phytopathogenic fungi. The present invention also relates to seeds treated with at least one such compound. Furthermore the invention relates to processes for preparing compounds of formula I as well as to specific intermediates that are obtained during the reaction sequence.

EP 352673 A2 relates to 1-halogen-1-azolylpropene and -methyloxiranes and fungicides comprising them. EP 3888871 A1 describes azolylmethyloxiranes as herbicides and plant growth regulators. EP 394843 A2 is directed at 1-halogenvinyl-1-azoles, fungicides comprising them and plant growth regulators. EP 391311 A2 relates to 1-alkoxy-1-azolylmethyloxiranes, processes for their preparation and their use as agrochemicals. EP 474045 A1 is directed at azolylmethyloxiranes and fungicides comprising them.

In many cases, in particular at low application rates, the fungicidal activity of known fungicidal compounds is unsatisfactory. Based on this, it was an object of the present invention to provide compounds having improved activity and/or a broader activity spectrum against phytopathogenic fungi. This objective is achieved by the use of substituted imidazolyl and triazolyl compounds of formula I having good fungicidal activity against phytopathogenic fungi.

The compounds according to the present invention differ from those described in the abovementioned publications in that the divalent methylene group adjacent to the triazole or imidazole ring carries at least one further substituent.

Accordingly, the present invention relates to compounds of the formula I 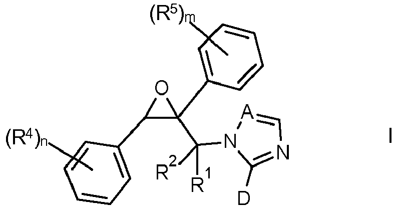 wherein:
- A: is N or CH;
- D: is hydrogen, halogen, SR^{D}; wherein
- R^{D}: is hydrogen, CN, C₁-C₆-alkyl, C₁-C₆-haloalkyl, C₂-C₆-alkenyl, C₂-C₆-haloalkenyl, C₂-C₆-alkynyl or C₂-C₆-haloalkynyl;
- R¹: is halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R¹ are unsubstituted or substituted by one, two, three or four or up to the maximum possible number of independently selected substituents R^{1a}; wherein
- R^{1a}: is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl;
- R²: is hydrogen, halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-Ce-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl group in the last-mentioned groups is 5- or 6-membered; and wherein the aliphatic, alicyclic and aromatic moieties of R² are unsubstituted or substituted by one, two, three or four or up to the maximum possible number of independently selected substituents R^{2a}; wherein
- R^{2a}: is halogen, CN, NO₂, OH, SH, NH₂, C₁-C₆-alkyl, C₁-C₆-haloalkyl, C₁-C₆-alk-oxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl;
or R¹ and R² together with the carbon atom to which they are bound (denominated as C*) form a vinyl group C*=CR¹¹R²², wherein
- R¹¹ and R²²: are independently selected from the group consisting of hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
or R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle includes beside carbon atoms one, two, three or four heteroatoms independently selected from the group consisting of N, O and S, and wherein the carbo- and heterocycle are unsubstituted or carry one, two, three or four independently selected substituents R¹², wherein one or two CH₂ groups of the carbo-or heterocycle may be replaced by one or two groups independently selected from the group of C(=O) and C(=S);
wherein
- R¹²: is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl or phenoxy; wherein the phenyl moieties of R¹² are unsubstituted or substituted by one, two, three or four independently selected substituents R^{12a}; wherein
- R^{12a}: is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl;
- R⁴ and R⁵: are independently selected from the group consisting of halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, phenyl, phenoxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic and alicyclic moieties of R⁴ are unsubstituted or substituted by one, two, three or four or up to the maximum possible number of independently selected substituents R^{4a}; wherein
- R^{4a}: is halogen, CN, NO₂, OH, C₁-C₄-alkyl, C₁-C₄-haloalkyl, C₃-C₈-cycloalkyl, C₁-C₄-alkoxy or C₁-C₄-halogenalkoxy;
- n: is 0, 1, 2, 3 or 4;
- m: is 0, 1, 2, 3 or 4;
and the N-oxides and the agriculturally acceptable salts of the compounds of formula I.

The present invention also provides a use of compounds I and/or their agriculturally useful salts for controlling phytopathogenic fungi and relates to methods for combating phytopathogenic fungi. The invention further provides compositions comprising these compounds I and/or their agriculturally acceptable salts. The present invention also relates to seeds treated with at least one such compound. Furthermore the invention relates to processes for preparing compounds of formula I and to intermediates such as compounds of formulae IV and V.

Compounds I can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, e.g. a racemate, individual stereoisomers, or as an optically active form.

Compounds I can be present in different crystal modifications whose biological activity may differ. They also form part of the subject matter of the present invention. The compounds of formula I can be present in atropisomers arising from restricted rotation about a single bond of asymmetric groups. They also form part of the subject matter of the present invention.

In respect of the variables, the embodiments of the intermediates correspond to the embodiments of the compounds of formula I. The term "compounds I" refers to compounds of formula I.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, for example methyl, ethyl, propyl, 1-methylethyl, butyl, 1-methylpropyl, 2-methylpropyl, and 1,1-dimethylethyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "C₁-C₆-alkoxy" refers to a straight-chain or branched alkyl group having 1 to 6 carbon atoms (as defined above) which is bonded via an oxygen, at any position in the alkyl group, for example methoxy, ethoxy, n-propoxy, 1-methylethoxy, butoxy, 1-methyl¬propoxy, 2-methylpropoxy or 1,1-dimethylethoxy.

The term "C₁-C₆-hydroxyalkyl" refers to a straight-chained or branched alkyl group having 1 to 6 carbon atoms (as defined above), wherein one hydrogen atom in these groups may be replaced by one hydroxy group, for example hydroxymethyl, 2-hydroxyethyl, 3-hydroxy--propyl, 4-hydroxy-butyl.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromo¬ethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to alkyl having 1 to 4 carbon atoms (as defined above), wherein one hydrogen atom of the alkyl radical is replaced by a phenyl or hetereoaryl radical respectively.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₆-alkoxy-C₁-C₄-alkyl" refers to alkyl having 1 to 4 carbon atoms (as defined above), wherein one hydrogen atom of the alkyl radical is replaced by a C₁-C₆-alkoxy group (as defined above).

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₆-alkylsulfinyl" refers to straight-chain or branched alkyl groups having 1 to 6 carbon atoms (as defined above) bonded through a -S(=O)- moiety, at any position in the alkyl group, for example methylsulfinyl and ethylsulfinyl, and the like. Accordingly, the term "C₁-C₆-haloalkylsulfinyl" refers to straight-chain or branched haloalkyl group having 1 to 6 carbon atoms (as defined above), bonded through a -S(=O)- moiety, at any position in the haloalkyl group.

The term "C₁-C₆-alkylsulfonyl" refers to straight-chain or branched alkyl groups having 1 to 6 carbon atoms (as defined above), bonded through a -S(=O)₂- moiety, at any position in the alkyl group, for example methylsulfonyl. Accordingly, the term "C₁-C₆-haloalkylsulfonyl" refers to straight-chain or branched haloalkyl group having 1 to 6 carbon atoms (as defined above), bonded through a -S(=O)₂- moiety, at any position in the haloalkyl group.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₃-C₈-cycloalkyl" refers to monocyclic saturated hydrocarbon radicals having 3 to 8 carbon ring members such as cyclopropyl (C₃H₅), cyclobutyl, cyclopentyl, cyclohexyl, cycloheptyl or cyclooctyl.

The term "C₃-C₈-cycloalkyl-C₁-C₄-alkyl" refers to a cycloalkyl radical having 3 to 8 carbon atoms (as defined above), which is bonded via a C₁-C₄-alkyl group (as defined above).

The term "C₃-C₈-cycloalkyl-C₃-C₈-cycloalkyl" refers to a cycloalkyl radical having 3 to 8 carbon atoms (as defined above), which is substituted by a further cycloalkyl radical having 3 to 8 carbon atoms.

The term "C₃-C₈-cycloalkyloxy" refers to a cycloalkyl radical having 3 to 8 carbon atoms (as defined above), which is bonded via an oxygen.

The term "C(=O)-C₁-C₄-alkyl" refers to a radical which is attached through the carbon atom of the group C(=O) as indicated by the number valence of the carbon atom. The number of valence of carbon is 4, that of nitrogen is 3. Likewise the following terms are to be construed: NH(C₁-C₄-alkyl), N(C₁-C₄-alkyl)₂, NH(C₃-C₆-cycloalkyl), N(C₃-C₆-cycloalkyl)₂, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂.

The term "saturated or partially unsaturated 3-, 4- 5-, 6- or 7-membered carbocycle" is to be understood as meaning both saturated or partially unsaturated carbocycles having 3, 4, 5, 6 or 7 ring members. Examples include cyclopropyl, cyclopentyl, cyclopentenyl, cyclopentadienyl, cyclohexyl, cyclohexenyl, cyclohexadienyl, cycloheptyl, cycloheptenyl, cycloheptadienyl, and the like.

The term "saturated or partially unsaturated 3-, 4-, 5-, 6-, or 7-membered heterocycle, wherein the ring member atoms of the heterocycle include besides carbon atoms 1, 2, 3 or 4 heteroatoms independently selected from the group of N, O and S", is to be understood as meaning both saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine; and
a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra-and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals; and

The term "5-or 6-membered heteroaryl" refers to aromatic ring systems incuding besides carbon atoms, 1, 2, 3 or 4 heteroatoms independently selected from the group consisting of N, O and S, for example,
a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

The inventive compounds I can be prepared by various routes in analogy to known processes described in the prior art, and, advantageously, by the synthesis shown in the following schemes and in the experimental part of this application.

Compounds of the formula I, wherein D is hydrogen, can be prepared starting from compounds II, wherein X is a leaving group, for example, chlorine, bromine, iodine or an alkylsulfonate. Compounds II are already known from EP 394843 A1, EP 388871 A1 and DE 3825841 A1. They are accessible by displacement of X as shown in Scheme 1 and as described in DE 3000244 A1, Indian Journal of Chemistry 2001, Section B: Organic Chemistry Including Medicinal Chemistry, 40B(9), 854-856; Indian Journal of Heterocyclic Chemistry 2006, 16(1), 97-98 or in Journal of Heterocyclic Chemistry2007, 44(1), 75-78. 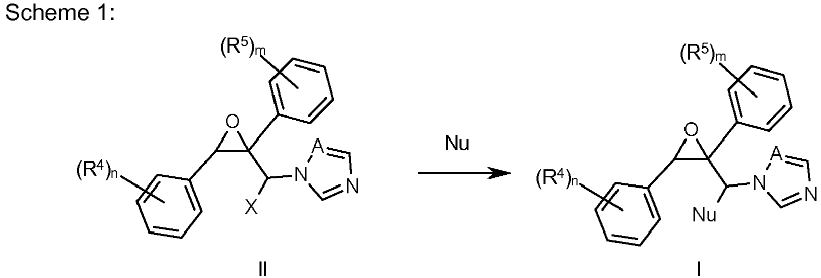 Alternatively compounds of the formula I can be prepared starting from an unsaturated aldehyde of the formula III as shown in Scheme 2. Compounds III are known to be useful intermediates for the synthesis of structurally related compounds I (R¹ and R² being hydrogen) and their preparation is described in EP 391311 A2 and DE 3825841 A1. Addition of a metal organic reagent to the aldehyde portion in compounds III leads to the formation of secondary alcohol IV (R² is hydrogen), which can be transferred to compounds I as shown in Scheme 2. The alcohol IV can then be activated by way of introduction of a leaving group LG. LG can be introduced, for example, by halogenation (LG meaning, for example, chlorine, bromine or iodine) or sulfonation (LG meaning, for example, methylsulfonate or tosylsulfonate). The leaving group can then be replaced by an azole or imidazole residue upon treatment with triazole or imidazole in the presence of a base to yield compounds of the formula I wherein D is hydrogen. 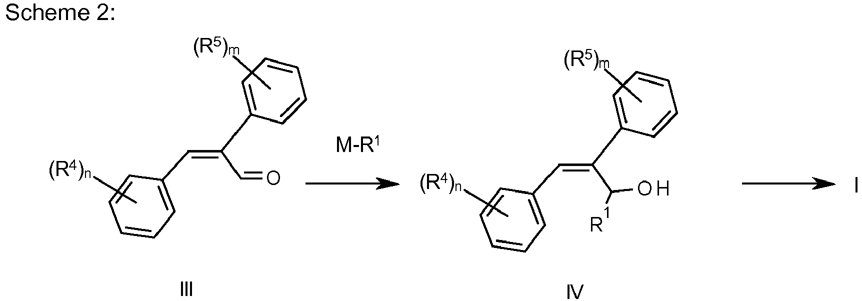 Oxidation of secondary alcohol IV affords an intermediate ketone and subsequent reaction of said ketone with a metal organic compound leads to the formation of a tertiary alcohol of the formula V (Scheme 3), wherein R¹ and R² are not hydrogen. Activation of the hydroxyl group of compound V, i.e. by way of introduction of a leaving group LG as described above, followed by treatment with a triazole or imidazole as described above furnishes compounds of the formula I wherein D is hydrogen. 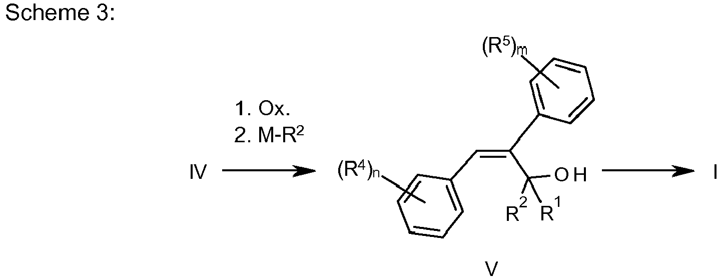 Compounds of the formula I wherein D is SH or halogen can be prepared starting from compounds I wherein D is hydrogen upon reaction with a base, for example butyl lithium, lithium diisopropylamide, lithium hexamethyldisilazide, potassium hexamethyldisilazide, lithium 2,2,6,6-tertramethylpiperidide or zink 2,2,6,6-tertramethylpiperidide, at low temperature in a suitable solvent such as Et₂O, MTBE or THF, followed by addition of a suitable electrophile, for example S₈, CH₃-S-S-CH₃, I₂, ICl, C₂F₄Br₂, to obtain correspondingly substituted azole compounds as has been described in Tetrahedron Letters 2011, 52(36), 4590-4594; WO 2006/102194 A1, Journal of Organic Chemistry 2009, 74(21), 8309-8313; WO 2011/113820 A1; WO 2012/025506 A1, WO 9616048 A1 or in Synthesis 1999, 1, 100-106.
Compounds I wherein D is SCN can be obtained by treating a compound I wherei D is SH with cyanogen halide such as Br-CN or CL-CN and a base (organic or inorganic base such as K₂CO₃, NaOH, KOH or NEt_{3,} DBU) in a suitable solvent such as acetone, MeCN or THF in analogy to methods reported in: WO 2009/077497 A2 or in Chemical & Pharmaceutical Bulletin 1964, 12(2), 182-191. Compounds I wherein D is SH can be functionalized at the sulfur atom using standard procedures, for example with an alkylation agent such as metyl iodide in the presence of a base (organic or inorganic base such as K₂CO₃, NaOH, KOH or NEt₃, DBU) in a suitable solvent such as acetone, MeCN or THF. These compounds can be synthesized for example in analogy with reported methods in: WO 2012/047762 A2, Heteroatom Chemistry 2010, 20(7), 405-410, Khimiya Geterotsiklicheskikh Soedinenii 1977, (11), 1561-1563, Indian Journal of Heterocyclic Chemistry 1999, 8(4), 341-342 or in WO 2011/113820 A1.

Compounds of the formula I wherein X is OR³ can be obtained from the reaction of compound I wherein X is OH in the presence of an electrophile (e.g. Mel, ethyl bromide, cyclopropyl bromide, 1,4-dibromobutane, propargyl bromide, methyl chloroformate, allyl bromide, acetylene, cyclohexene, cyclopentene, phenol) and a base (e.g. NaH, KH, *t*-BuOK, NaH, KOH, Et₃N, LDA, imidazole, K₂CO₃, Cs₂CO₃) and in an inert organic solvent preferably (e.g. THF, DME, Et₂O, DMF, NMP, DMSO, toluene, acetonitrile). These compounds can be synthesized in analogy with the procedures described in: Chemische Berichte 1986, 119(12), 3672-3693; Journal of Organic Chemistry 2011, 76(14), 5825-5831; Synlett 2001, 1962-1964; Tetrahedron 1987, 43(10), 2311-2316; Organometallics 2003, 22(19), 3915-3920; Tetrahedron 2007, 63(37), 9071-9081; Tetrahedron 2007, 63(37), 9071-9081 or in Journal of Organometallic Chemistry 1987, 334(1-2), 225-242.

Compounds of the formula I wherein X is CN can be synthesized from compounds I wherein X is OH by transformation of the OH group into a leaving group as described above for compounds V followed by substitution with an appropriate cyanide source (Organic Process Research & Development 2012, 16(8), 1385-1392; WO 2012024150 A1; US 20100056637 A1; Organic Letters 2008, 10(20), 4573-4576). Such nitriles can also be obtained from compounds I wherein X is OH in presence of a reagent, for example cyanuric trichloride, NaCN, tetrabutylammonium cyanide and/or an additive (for example N-tosylimidazole, Bu₄Nl, Bu₄NCl, Bu₄NBr, TMSCI, DDQ, PPh₃) in an inert organic solvent (for example THF, DME, Et₂O, DMF, NMP, DMSO, toluene, acetonitrile). These compounds can be prepared for example in analogy to methods described in: Letters in Organic Chemistry 2005, 2(8), 725-730, Tetrahedron Letters 2007, 48(38), 6779-6784, Journal of Organic Chemistry 2004, 69(7), 2562-2564 or in Organic Chemistry: An Indian Journal 2008, 4(1), 32-35.

If individual compounds I cannot be obtained by the routes described above, they can be prepared by derivatization of other compounds I. The N-oxides may be prepared from the compounds I according to conventional oxidation methods, e. g. by treating compounds I with an organic peracid such as metachloroperbenzoic acid (cf. WO 03/64572 or J. Med. Chem. (1995), 38(11), 1892-1903,); or with inorganic oxidizing agents such as hydrogen peroxide (cf. J. Heterocyc. Chem. (1981), 18 (7), 1305-1308) or oxone (cf. J. Am. Chem. Soc. (2001), 123 (25), 5962-5973). The oxidation may lead to pure mono-N-oxides or to a mixture of different N-oxides, which can be separated by conventional methods such as chromatography.

In one aspect the invention relates to intermediates of formula IV. In another aspect the invention relates to intermediates of formula V.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

The reaction mixtures are worked up in a customary manner, for example by mixing with water, separating the phases and, if appropriate, chromatic purification of the crude products. In some cases, the intermediates and end products are obtained in the form of colorless or slightly viscous oils which can be freed from volatile components or purified under reduced pressure and at moderately elevated temperatures. If the intermediates and end products are obtained as solids, purification can also be carried out by recrystallization or digestion.

Agriculturally useful salts of the compounds I encompass especially the salts of those cations or the acid addition salts of those acids whose cations and anions, respectively, have no adverse effect on the fungicidal action of the compounds I. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium.
Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting a compound I with an acid of the corresponding anion, preferably of hydrochloric acid, hydrobromic acid, sulfuric acid, phosphoric acid or nitric acid.

In the following, the meaning of the substituents of the compounds used according to the invention is further defined. Thereby, in each case the substituents are meant to have the given meanings and preferred meaning on their own or in any combination with the meanings or preferred meanings of any other substituent. The groups mentioned herein in tables A and B, as well as in tables 1 to 96, for a combination of substituents are furthermore, independently of the combination in which they are mentioned, particularly preferred embodiments of the substituent or of the combination of substituents in question.

A according to the invention is N or CH. According to one embodiment A is N. According to a further embodiment A is CH.

D according to the present invention is hydrogen, halogen or SR^{D}, wherein R^{D} is hydrogen, CN, C₁-C₆-alkyl, C₁-C₆-haloalkyl, C₂-C₆-alkenyl, C₂-C₆-haloalkenyl, C₂-C₆-alkynyl or C₂-C₆-haloalkynyl.

In a preferred embodiment D is hydrogen, halogen, SH, SCN or S-CH₂-CH=CH₂ (S-allyl). According to one embodiment D is hydrogen. According to a further embodiment, D is halogen, in particular iodine. According to another preferred embodiment D is SR^{D}. According to a particular embodiment, R^{D} is H. In yet another preferred embodiment R^{D} is CN. In a further preferred embodiment R^{D} is -CH₂-CH=CH₂.

R¹ according to the invention is halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R¹ are unsubstituted or substituted by one, two, three or four or up to the maximum possible number of independently selected substituents R^{1a}; wherein R^{1a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl.

R² according to the invention is hydrogen, halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-Ce-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl group in the last-mentioned groups is 5- or 6-membered; and wherein the aliphatic, alicyclic and aromatic moieties of R² are unsubstituted or substituted by one, two, three or four or up to the maximum possible number of independently selected substituents R^{2a}; wherein R^{2a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl.
According to a preferred embodiment of the invention R² is hydrogen, halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-Ce-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R² are unsubstituted or substituted by one, two, three or four or up to the maximum possible number of independently selected substituents R^{2a}; wherein R^{2a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl.
According to another preferred embodiment of the invention R² is halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-Ce-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R² are unsubstituted or substituted by one, two, three or four or up to the maximum possible number of independently selected substituents R^{2a}; wherein R^{2a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl.
According to a further preferred embodiment R² is hydrogen, halogen, CN, NO₂, OH, SH, C₁-C₆-alkyl, C₁-C₆-hydroxyalkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl, C₂-C₆-haloalkenyl, C₂-C₆-haloalkynyl, C₃-C₈-cycloalkyl, C₃-C₈-cycloalkyloxy, C₃-C₈-cycloalkyl-C₁-C₄-alkyl, NH₂, NH(C₁-C₄-alkyl) or N(C₁-C₄-alkyl)₂. Preferably, R² is halogen, CN, NO₂, OH, NH₂, N(CH₃)₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl or C₃-C₈-cycloalkyl.
According to another preferred embodiment R² is halogen, CN, NO₂, OH, SH, C₁-C₆-alkyl, C₁-C₆-hydroxyalkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl, C₂-C₆-haloalkenyl, C₂-C₆-haloalkynyl, C₃-C₈-cycloalkyl, C₃-C₈-cycloalkyloxy, C₃-C₈-cycloalkyl-C₁-C₄-alkyl, NH₂, NH(C₁-C₄-alkyl) or N(C₁-C₄-alkyl)₂. Preferably, R² is halogen, CN, NO₂, OH, NH₂, N(CH₃)₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl or C₃-C₈-cycloalkyl.
In still a further preferred embodiment R² is hydrogen, halogen, CN, OH, NH₂, N(CH₃)₂, C₁-C₆-alkyl, C₁-C₆-haloalkyl, C₁-C₆-alkoxy, C₁-C₆-haloalkoxy, C₂-C₆-alkenyl, C₂-C₆-alkynyl or C₃-C₈-cycloalkyl.
In yet another preferred embodiment R² is halogen, CN, OH, NH₂, N(CH₃)₂, C₁-C₆-alkyl, C₁-C₆-haloalkyl, C₁-C₆-alkoxy, C₁-C₆-haloalkoxy, C₂-C₆-alkenyl, C₂-C₆-alkynyl or C₃-C₈-cycloalkyl.
In another preferred embodiment R² is hydrogen, halogen, CN, OH, NH₂, N(CH₃)₂, C₁-C₆-alkyl, C₁-C₆-alkoxy, C₂-C₆-alkenyl, C₂-C₆-alkynyl and C₃-C₈-cycloalkyl.
In still another preferred embodiment R² is halogen, CN, OH, NH₂, N(CH₃)₂, C₁-C₆-alkyl, C₁-C₆-alkoxy, C₂-C₆-alkenyl, C₂-C₆-alkynyl and C₃-C₈-cycloalkyl.

According to a further preferred embodiment of the invention, R² is C₃-C₈-cydoalkyl, phenyl or 5- or 6-membered heteroaryl, wherein the alicyclic and aromatic moieties of R² are unsubstituted or substituted by one, two, three or four or up to the maximum possible number of independently selected substituents R^{2a}. In another preferred embodiment R² is C₃-C₈-cycloalkyl, in particular cyclopropyl.
According to a further preferred embodiment of the invention, R² is phenyl or 5- or 6-membered heteroaryl, wherein the aromatic moieties of R² is unsubstituted or substituted by one, two, three or four or up to the maximum possible number of independently selected substituents R^{2a}.
In another preferred embodiment R² is C₂-C₆-alkenyl, in particular 2-propenyl, or C₂-C₆-alkynyl, in particular 2-propynyl and 2-butynyl.
In one further preferred aspect of the invention R² is C₁-C₆-haloalkyl, in particular CF₃ or CHF₂, or C₁-C₆-haloalkoxy, in particular OCF₃ or OCHF₂.
In a preferred embodiment R² is halogen, in particular chlorine or bromine.
According to a further preferred embodiment R² is C₁-C₆-alkyl, in particular C₁-C₄-alkyl, more particularly methyl, ethyl and 1-methylethyl.
Furthermore, in a preferred embodiment R² is C₁-C₆-alkoxy, in particular methoxy, ethoxy or 1-methylethoxy.
In another preferred embodiment R² is C₂-C₆-alkenyl, in particular 2-propenyl, or C₂-C₆-alkynyl, in particular 2-propynyl and 2-butynyl. In one further aspect of the invention R² is C₁-C₆-haloalkyl, in particular CF₃ or CHF₂, or C₁-C₆-haloalkoxy, in particular OCF₃ or OCHF₂. In another preferred embodiment R² is hydrogen.

According to one embodiment of the invention R¹ is halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-_{C}y_{C}loalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R¹ are unsubstituted or substituted by one, two, three or four or up to the maximum possible number of independently selected substituents R^{1a}; wherein R^{1a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl and R² is independently as defined or preferably defined above. According to another embodiment R¹ is halogen, CN, NO₂, OH, SH, C₁-C₆-alkyl, C₁-C₆-hydroxyalkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl, C₂-C₆-haloalkenyl, C₂-C₆-haloalkynyl, C₃-C₈-cydoalkyl, C₃-C₈-cycloalkyloxy, C₃-C₈-cycloalkyl-C₁-C₄-alkyl, NH₂, NH(C₁-C₄-alkyl) or N(C₁-C₄-alkyl)₂ and R² is independently as defined for R¹ or hydrogen. Preferably, R¹ is halogen, CN, NO₂, OH, NH₂, N(CH₃)₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl or C₃-C₈-cycloalkyl and R² is as defined or preferably defined above. In a further preferred embodiment R¹ is halogen, CN, OH, NH₂, N(CH₃)₂, C₁-C₆-alkyl, C₁-C₆-haloalkyl, C₁-C₆-alkoxy, C₁-C₆-haloalkoxy, C₂-C₆-alkenyl, C₂-C₆-alkynyl or C₃-C₈-cycloalkyl and R² is independently as defined or preferably defined above.
In yet another preferred embodiment R¹ is halogen, CN, NO₂, OH, NH₂, N(CH₃)₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl or C₂-C₆-alkynyl or C₃-C₈-cycloalkyl, and R² is a radical as defined for R¹ or hydrogen.
In still another preferred embodiment R¹ is halogen, CN, OH, NH₂, N(CH₃)₂, C₁-C₆-alkyl, C₁-C₆-alkoxy, C₂-C₆-alkenyl, C₂-C₆-alkynyl and C₃-C₈-cycloalkyl and R² is independently as defined or preferably defined above.

In one aspect of the invention R¹ is CN and R² is as defined or preferably defined above. In a further aspect R¹ is CN and R² is CN. In yet another aspect R¹ is CN and R² is hydrogen.
In a further specific aspect of the invention R¹ is OH, SH or NH₂ and R² is as defined or preferably defined above. In still another specific aspect R² is hydrogen and R¹ is OH, SH or NH₂. In a further aspect R² is hydrogen and R¹ is OH, SH or NH₂.
In one preferred embodiment R¹ is C₂-C₆-alkenyl, in particular 2-propenyl, or C₂-C₆-alkynyl, in particular 2-propynyl and 2-butynyl and R² is as defined or preferably defined above. In a preferred embodiment R² is hydrogen and R¹ is C₂-C₆-alkenyl, in particular 2-propenyl, or C₂-C₆-alkynyl, in particular 2-propynyl and 2-butynyl.
In another preferred embodiment R¹ is C₁-C₆-haloalkyl, in particular CF₃ or CHF₂, or C₁-C₆-haloalkoxy and R² is as defined or preferably defined above. In one further aspect of the invention R² is hydrogen and R¹ is C₁-C₆-haloalkyl, in particular CF₃ or CHF₂, or C₁-C₆-haloalkoxy, in particular OCF₃ or OCHF₂.
In a further preferred embodiment R¹ is C₃-C₈-cycloalkyl, phenyl or 5- or 6-membered heteroaryl, wherein the alicyclic and aromatic moieties of R¹ are unsubstituted or substituted by one, two, three or four or up to the maximum possible number of independently selected substituents R^{1a} and R² is as defined or preferably defined above. In still another preferred embodiment R² is hydrogen and R¹ is C₃-C₈-cycloalkyl, phenyl or 5- or 6-membered heteroaryl, wherein the alicyclic and aromatic moieties of R¹ are unsubstituted or substituted by one, two, three or four or up to the maximum possible number of independently selected substituents R^{1a}.
According to a further embodiment of the invention, R¹ is phenyl or 5- or 6-membered heteroaryl, wherein the aromatic moieties of R¹ are unsubstituted or substituted by one, two, three or four or up to the maximum possible number of independently selected substituents R^{1a} and R² is independently as defined or preferably defined above. According to yet another embodiment of the invention R² is hydrogen and R¹ is phenyl or 5- or 6-membered heteroaryl, wherein the aromatic moieties of R¹ are unsubstituted or substituted by one, two, three or four or up to the maximum possible number of independently selected substituents R^{1a}.

In another preferred embodiment R¹ is C₃-C₈-cydoalkyl, in particular cyclopropyl and R² is as defined or preferably defined above. In still a further preferred embodiment R² is hydrogen and R¹ is C₃-C₈-cydoalkyl, in particular cyclopropyl.

According to one embodiment of the invention R¹ and R² together with the carbon atom to which they are bound (denominated as C*) form a vinyl group C*=CR¹¹R²², wherein R¹¹ and R²² are as defined or preferably defined below; or R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle includes beside carbon atoms one, two, three or four heteroatoms independently selected from the group consisting of N, O and S, and wherein the carbo- and heterocycle are unsubstituted or carry one, two, three or four independently selected substituents R¹² as defined and preferably defined below, and wherein one or two CH₂ groups of the carbo- or heterocycle may be replaced by one or two groups independently selected from the group of C(=O) and C(=S).
According to a further embodiment of the invention R¹ and R² together with the carbon atom to which they are bound (denominated as C*) form a vinyl group C*=CR¹¹R²², wherein R¹¹ and R²² are as defined or preferably defined below; or R¹ and R² together with the carbon atom to which they are bound form a saturated or partially unsaturated three-, four-, five-, six- or seven-membered carbocycle, which is unsubstituted or carries one, two, three or four independently selected substituents R¹² as defined and preferably defined below, and wherein one or two CH₂ groups of the carbocycle may be replaced by one or two groups independently selected from the group of C(=O) and C(=S).

According to a further embodiment of the invention R¹ and R² together with the carbon atom to which they are bound (denominated as C*) form a vinyl group C*=CR¹¹R²², wherein R¹¹ and R²² are as defined or preferably defined below.

R¹¹ and R²² according to the invention are independently selected from the group consisting of hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl. In another preferred embodiment R¹ and R² are independently selected from the group consisting of hydrogen, halogen and C₁-C₆-alkyl. In a further preferred embodiment R¹¹ and R²² are hydrogen

In a further embodiment of the invention R¹ and R² together with the carbon atom to which they are bound form a saturated or partially unsaturated three-, four-, five-, six-or seven-membered carbocycle or a saturated or partially unsaturated three-, four-, five-, six- or seven-membered heterocycle, wherein the heterocycle includes beside carbon atoms one, two, three or four heteroatoms independently selected from the group consisting of N, O and S, and wherein the carbo- and heterocycle are unsubstituted or carry one, two, three or four independently selected substituents R¹² as defined and preferably defined below, wherein one or two CH₂ groups of the carbo-or heterocycle may be replaced by one or two groups independently selected from the group of C(=O) and C(=S), such as in, for example, cyclopropanone, cyclopentanone, cyclopropanethione, cyclopentanethione, 5-oxazolone, cyclohexane-1,4-dione, cyclohexane-1,4-dithione.

According to another embodiment of the invention R¹ and R² together with the carbon atom to which they are bound form a saturated or partially unsaturated three-, four-, five-, six- or seven-membered carbocycle which is unsubstituted or carries one, two, three or four independently selected substituents R¹² as defined and preferably defined below.
In one preferred embodiment R¹ and R² together with the carbon atom to which they are bound form a saturated carbocycle, in particular selected from the group consisting of cyclopropyl, cyclobutyl, cyclopentyl, cyclohexyl and cycloheptyl, wherein the carbocycle in each case is unsubstituted or carries one, two, three or four independently selected substituents R¹² as defined and preferably defined below.
In another preferred embodiment R¹ and R² together with the carbon atom to which they are bound form a partially unsaturated carbocycle, in particular selected from the group consisting of cyclopentenyl, cyclopentadienyl and cyclohexenyl and wherein the carbocycle is unsubstituted or carries one, two, three or four independently selected substituents R¹² as defined and preferably defined below.
In a specific embodiment thereof, R¹ and R² together with the carbon atom to which they are bound form a cyclopropyl ring which is unsubstituted or carries one, two, three or four independently selected substituents R¹² as defined and preferably defined below.
According to a further specific embodiment R¹ and R² together with the carbon atom to which they are bound form a cyclobutyl ring which is unsubstituted or carries one, two, three or four independently selected substituents R¹² as defined and preferably defined below.
In still a further specific embodiment of the invention R¹ and R² together with the carbon atom to which they are bound form a cyclopentyl ring which is unsubstituted or carries one, two, three or four independently selected substituents R¹² as defined and preferably defined below.
In yet another preferred embodiment R¹ and R² together with the carbon atom to which they are bound form a cyclohexyl ring which is unsubstituted or carries one, two, three or four independently selected substituents R¹² as defined and preferably defined below.

According to still another embodiment of the invention R¹ and R² together with the carbon atom to which they are bound form a saturated or partially unsaturated three-, four-, five-, six- or seven-membered heterocycle, in particular a three-, four- or five-membered heterocycle, wherein the heterocycle includes beside carbon atoms one, two, three or four, in particular one or two, heteroatoms independently selected from the group consisting of N, O and S, and wherein the heterocycle is unsubstituted or carries one, two, three or four independently selected substituents R¹² as defined and preferably defined below.
In one specific embodiment thereof R¹ and R² form a heterocycle selected from the group consisting of oxirane, aziridine, thiirane, oxetane, azetidine, thiethane, 2-tetrahydrofuranyl, 3-tetrahydrofuranyl, 2-tetrahydrothienyl and 3-tetrahydrothienyl, wherein the heterocycle is unsubstituted or carries one, two, three or four independently selected substituents R¹² as defined and preferably defined below.
In a further specific embodiment R¹ and R² together with the carbon atom to which they are bound form an oxirane which is unsubstituted or carries one, two, three or four independently selected substituents R¹² as defined and preferably defined below.
In still a further specific embodiment of the invention R¹ and R² together with the carbon atom to which they are bound form an oxetane which is unsubstituted or carries one, two, three or four independently selected substituents R¹² as defined and preferably defined below.

R¹² according to the invention is in each case independently selected from the group consisting of halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl or phenoxy; wherein the phenyl moieties of R¹² are unsubstituted or substituted by one, two, three or four independently selected substituents R^{12a}; wherein R^{12a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl.
In a preferred embodiment R¹² is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio or C₁-C₄-alkoxy-C₁-C₄-alkyl.
In another preferred embodiment R¹² is halogen, CN, C₁-C₆-alkyl, C₁-C₆-haloalkyl, C₁-C₆-alkoxy or C₁-C₆-haloalkoxy. In another preferred embodiment R¹² is halogen, CN or C₁-C₆-alkyl. In yet another preferred embodiment R¹² is halogen, in particular fluorine. In a further preferred embodiment R¹² is chlorine. In still another preferred aspect of the invention R¹² is C₁-C₆-alkyl, in particular C₁-C₄-alkyl, even more preferably methyl.

R⁴ and R⁵ according to the present invention are independently selected from the group consisting of halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, phenyl, phenoxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic and alicyclic moieties of R⁴ are unsubstituted or substituted by one, two, three or four or up to the maximum possible number of independently selected substituents R^{4a}; wherein R^{4a} is halogen, CN, NO₂, OH, C₁-C₄-alkyl, C₁-C₄-haloalkyl, C₃-C₈-cydoalkyl, C₁-C₄-alkoxy or C₁-C₄-halogenalkoxy.

In another preferred embodiment R⁴ and R⁵ are independently selected from the group consisting of halogen, CN, NO₂, OH, SH, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl, C₂-C₆-haloalkenyl, C₂-C₆-haloalkynyl, C₃-C₈-cydoalkyl, C₃-C₈-cycloalkyloxy and C₃-C₈-cycloalkyl-C₁-C₄-alkyl.
In one specific embodiment R⁴ and R⁵ are independently selected from the group consisting of halogen, CN, NO₂, OH, C₁-C₆-alkyl, C₁-C₆-haloalkyl, C₁-C₆-alkoxy, C₁-C₆-haloalkoxy, C₂-C₆-alkenyl, C₂-C₆-alkynyl, C₂-C₆-haloalkenyl and C₂-C₆-haloalkynyl.
In another preferred embodiment R⁴ and R⁵ are independently selected from the group consisting of halogen and C₁-C₆-haloalkyl.
In a further preferred embodiment R⁴ and R⁵ are independently selected from the group consisting of halogen, in particular fluorine and chlorine.

In another preferred embodiment of the invention R⁴ is independently selected from the group consisting of halogen, in particular fluorine and chlorine, and R⁵ is independently selected from the group consisting of C₁-C₆-haloalkyl, in particular CF₃ and CHF₂. In another preferred embodiment of the invention R⁵ is independently selected from the group consisting of halogen, in particular fluorine or chlorine, and R⁴ is independently selected from the group consisting of C₁-C₆-haloalkyl, in particular CF₃ or CHF₂.
In a further preferred embodiment of the invention R⁴ is independently selected from the group consisting of halogen, in particular fluorine or chlorine, and R⁵ is CN. In another preferred embodiment of the invention R⁵ is independently selected from the group consisting of halogen, in particular fluorine or chlorine, and R⁴ is CN.
In one preferred embodiment of the invention R⁴ is independently selected from the group consisting of C₁-C₆-haloalkyl, in particular CF₃ or CHF₂ and R⁵ is CN. In still another preferred embodiment of the invention R⁵ is independently selected from the group consisting of C₁-C₆-haloalkyl, in particular CF₃ or CHF₂ and R⁴ is CN. In a further preferred embodiment R⁴ and R⁵ are CF₃.
In still another preferred embodiment R⁴ and R⁵ are CHF₂. In yet another preferred aspect of the invention R⁴ and R⁵ are CN.
In a further embodiment of the invention R⁴ is independently selected from the group consisting of 2-F, 3-F, 4-F, 2-Cl, 3-Cl, 4-Cl, 4-Br, 4-I, 4-CH₃, 4-OCH₃, 2-CF₃, 3-CF₃, 4-CF₃, 2-CHF₂, 3-CHF₂, 4-CHF₂, 2,4-F₂, 2,3-F₂, 2,5-F₂, 2,6-F₂, 3,4-F₂, 3,5-F₂, 2,4-Cl₂, 2,3-Cl₂, 2,5-Cl₂, 2,6-Cl₂, 3,4-Cl₂, 3,5-Cl₂, 2,4-(CH₃)₂, 2,4-(CF₃)₂, 2,4-(CHF₂)₂, 2-F-4-Cl, 2-F-4-CF₃, 2-F-4-CHF₂, 2-Cl-4-F, 2-Cl-4-CF₃, 2-Cl-4-CHF₂, 2-CF₃-4-F, 2-CF₃-4-Cl, 2-CF₃-4-CHF₂, 2-CHF₂-4-F, 2-CHF₂-4-Cl, 2-CHF₂-4-CF₃, 2-CN, 3-CN, 4-CN, 2-F-4-CN, 2-Cl-4-CN, 2-CF₃-4-CN, 2-CHF₂-4-CN and 2-OCHF₂.

In still a further embodiment of the invention R⁴ is independently selected from the group consisting of 2-F, 3-F, 4-F, 2-Cl, 3-Cl, 4-Cl, 2-CF₃, 3-CF₃, 4-CF₃, 2,4-F₂, 2,4-Cl₂, 2,4-(CF₃)₂, 3-CN, 4-CN, 4-C(CH₃)₃.
In a further embodiment of the invention R⁵ is independently selected from the group consisting of 2-F, 3-F, 4-F, 2-Cl, 3-Cl, 4-Cl, 4-Br, 4-I, 4-CH₃, 4-OCH₃, 2-CF₃, 3-CF₃, 4-CF₃, 2-CHF₂, 3-CHF₂, 4-CHF₂, 2,4-F₂, 2,3-F₂, 2,5-F₂, 2,6-F₂, 3,4-F₂, 3,5-F₂, 2,4-Cl₂, 2,3-Cl₂, 2,5-Cl₂, 2,6-Cl₂, 3,4-Cl₂, 3,5-Cl₂, 2,4-(CH₃)₂, 2,4-(CF₃)₂, 2,4-(CHF₂)₂, 2-F-4-Cl, 2-F-4-CF₃, 2-F-4-CHF₂, 2-Cl-4-F, 2-Cl-4-CF₃, 2-Cl-4-CHF₂, 2-CF₃-4-F, 2-CF₃-4-Cl, 2-CF₃-4-CHF₂, 2-CHF₂-4-F, 2-CHF₂-4-Cl, 2-CHF₂-4-CF₃, 2-CN, 3-CN, 4-CN, 2-F-4-CN, 2-Cl-4-CN, 2-CF₃-4-CN, 2-CHF₂-4-CN, 2-OCHF₂, 4-OCF₃, 4-C(CH₃)₃ and 3-Br-4-F.
In still a further embodiment of the invention R⁵ is independently selected from the group consisting of 2-F, 3-F, 4-F, 2-Cl, 3-Cl, 4-Cl, 2-CF₃, 3-CF₃, 4-CF₃, 2,4-F₂, 2,4-Cl₂, 2,4-(CF₃)₂, 3-CN, 4-CN, 4-C(CH₃)₃.

According to the invention n is 0, 1, 2, 3 or 4. In a preferred embodiment n is 1, 2, 3 or 4. In another preferred embodiment n is 0, 1, 2 or 3. In another preferred embodiment n is 1, 2 or 3. In a preferred aspect n is 0, 1 or 2. In still another preferred aspect n is 1 or 2. In a further preferred embodiment n is 1. In yet another preferred embodiment n is 2. In still a further preferred embodiment n is 0.

According to one embodiment of the invention n is 1, 2, 3 or 4, in particular 1, 2 or 3, wherein in each case one radical R⁴ is located in ortho-position of the phenyl ring with respect to the oxirane residue. In another preferred aspect of the invention n is 1, 2, 3 or 4, in particular 1, 2 or 3, wherein in each case one radical R⁴ is located in para-position of the phenyl ring with respect to the oxirane residue. In a preferred embodiment n is 1, 2, 3 or 4, in particular 1, 2 or 3, or particularly 1 or 2, wherein in each case one R⁴ is located in ortho- and one R⁴ is located in para-position of the phenyl ring with respect to the oxirane residue. In still another preferred embodiment, n is 1 and R⁴ is located in ortho-position of the phenyl ring with respect to the oxirane residue. In a further preferred embodiment n is 1 and R⁴ is located in para-position of the phenyl ring with respect to the oxirane residue. In a specific embodiment, n is 1 and R⁴ is chlorine, which is located in ortho-position of the phenyl ring with respect to the oxirane residue. In yet another specific embodiment, n is 1 and R⁴ is CF₃, which is located in ortho-position of the phenyl ring with respect to the oxirane residue.

According to the invention m is 0, 1, 2, 3 or 4. In a preferred embodiment m is 1, 2, 3 or 4. In another preferred embodiment m is 0, 1, 2 or 3. In another preferred embodiment m is 1, 2 or 3. In a preferred aspect m is 0, 1 or 2. In still another preferred aspect m is 1 or 2. In a further preferred embodiment m is 1. In yet another preferred embodiment m is 2. In still a further preferred embodiment m is 0.

According to one embodiment of the invention m is 1, 2, 3 or 4, in particular 1, 2 or 3, wherein in each case one radical R⁵ is located in ortho-position of the phenyl ring with respect to the oxirane residue. In another preferred aspect of the invention m is 1, 2, 3 or 4, in particular 1, 2 or 3, wherein in each case one radical R⁵ is located in para-position of the phenyl ring with respect to the oxirane residue. In a preferred embodiment m is 1, 2, 3 or 4, in particular 1, 2 or 3, or particularly 1 or 2, wherein in each case one R⁵ is located in ortho- and one R⁵ is located in para-position of the phenyl ring with respect to the oxirane residue. In still another preferred embodiment, m is 1 and R⁵ is located in ortho-position of the phenyl ring with respect to the oxirane residue. In a further preferred embodiment m is 1 and R⁵ is located in para-position of the phenyl ring with respect to the oxirane residue. In a specific embodiment, m is 1 and R⁵ is chlorine, which is located in ortho-position of the phenyl ring with respect to the oxirane residue. In yet another specific embodiment, m is 1 and R⁵ is CF₃, which is located in ortho-position of the phenyl ring with respect to the oxirane residue.

According to further preferred embodiments of the invention R¹, R² and D in each case are one of the following combinations in lines A-1 to A-96 in table A.

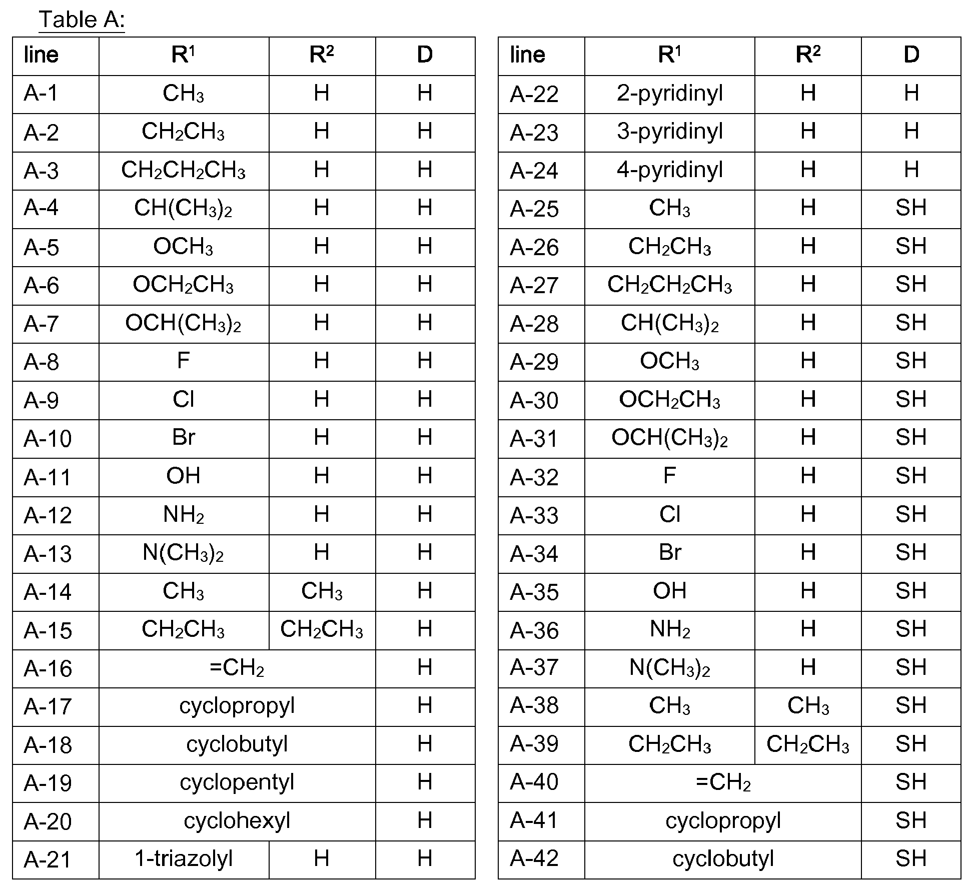

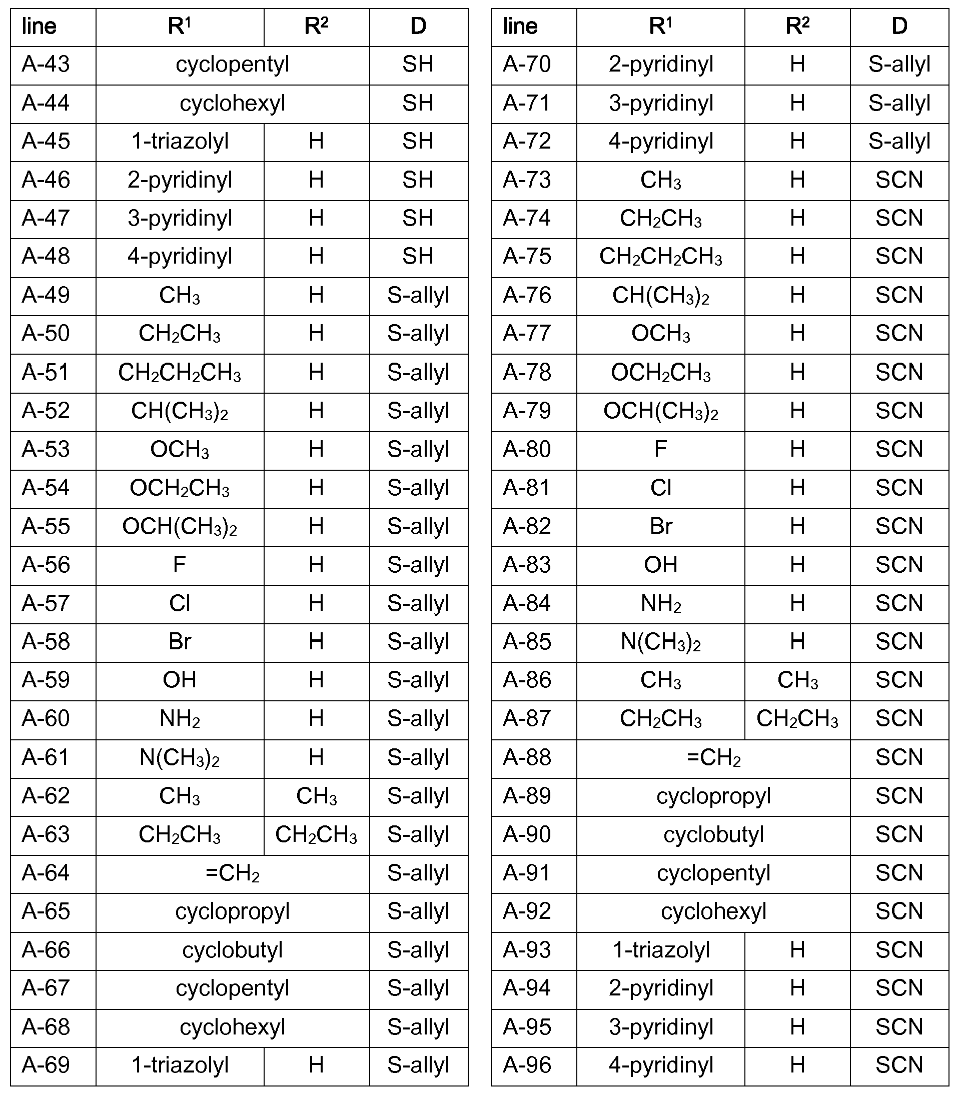

Further preferred embodiments relate to compounds I wherein the combination of (R⁴)ₙ and (R⁵)ₘ in each case has one of the following meanings as defined in lines B-1 to B-2704 in table B.

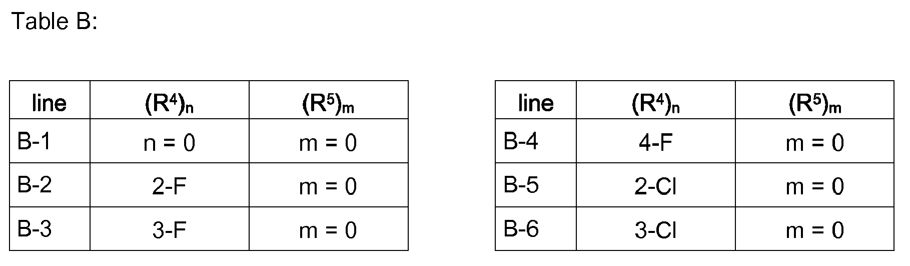

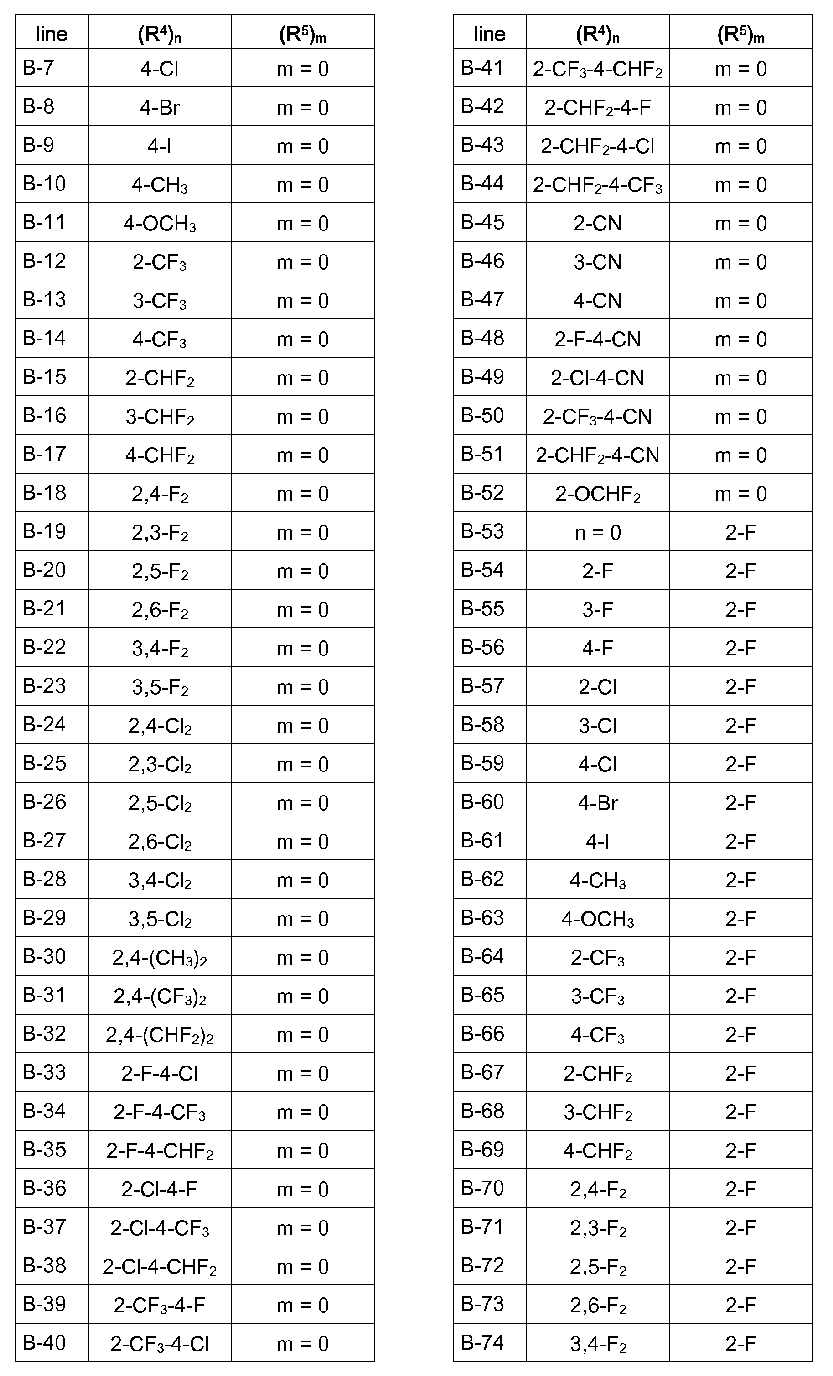

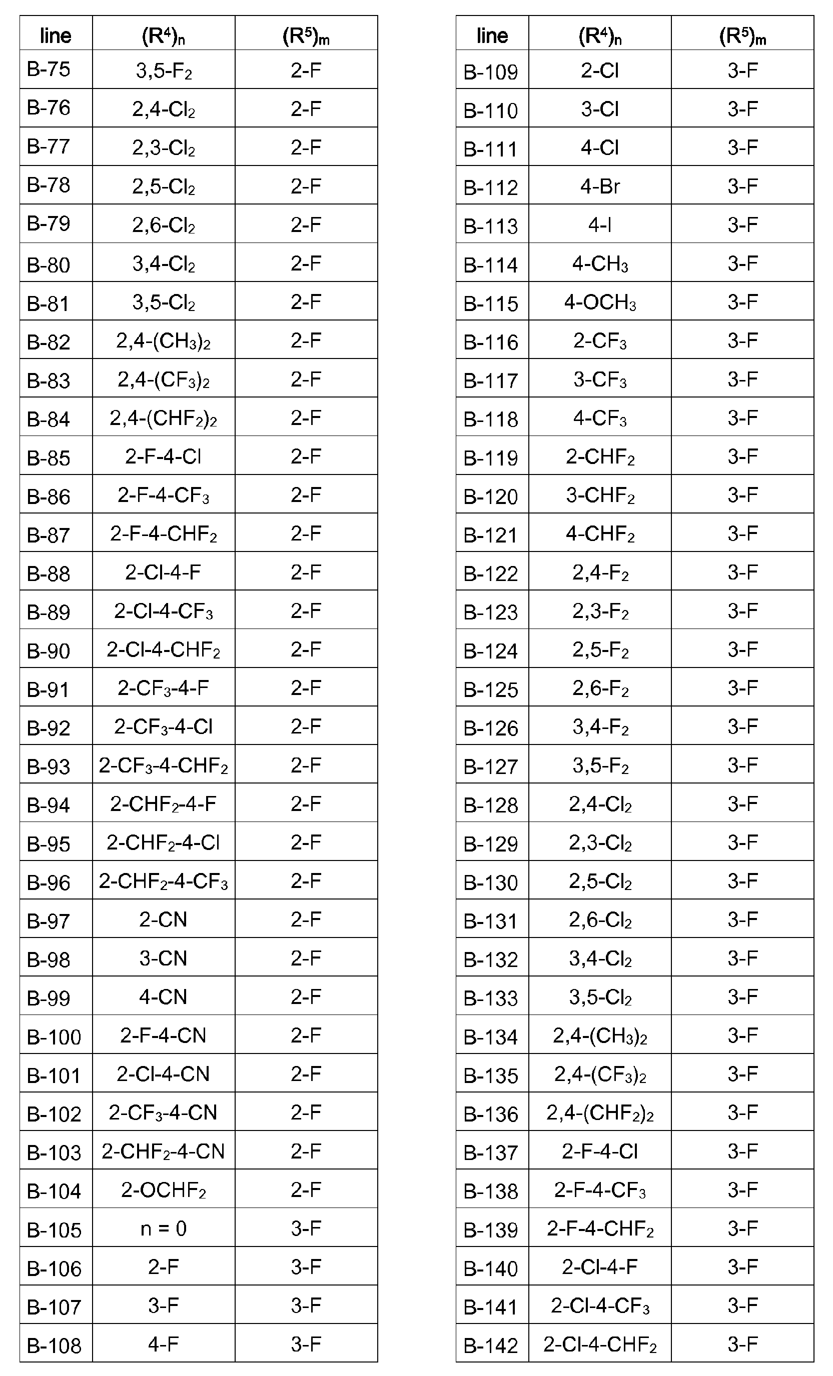

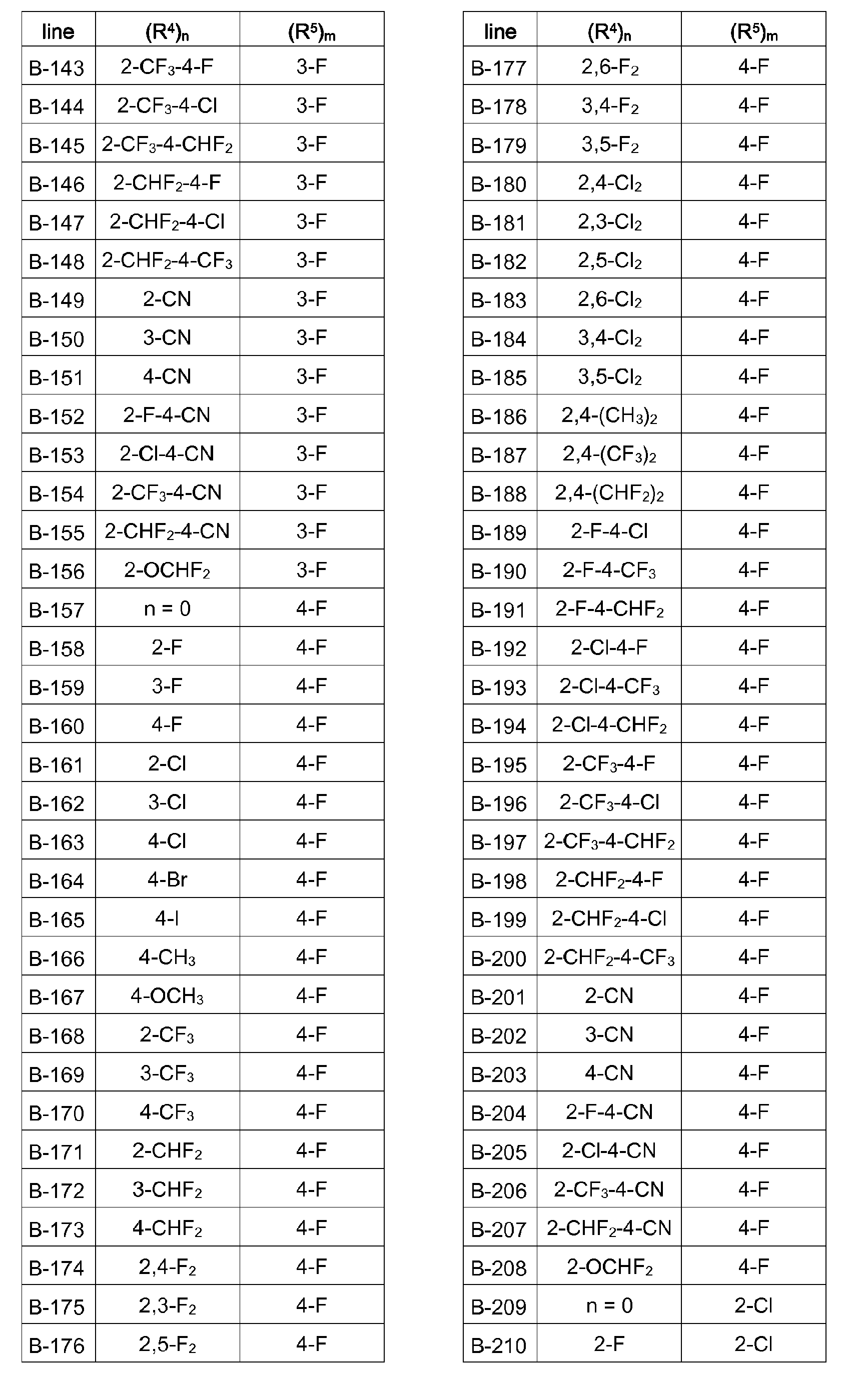

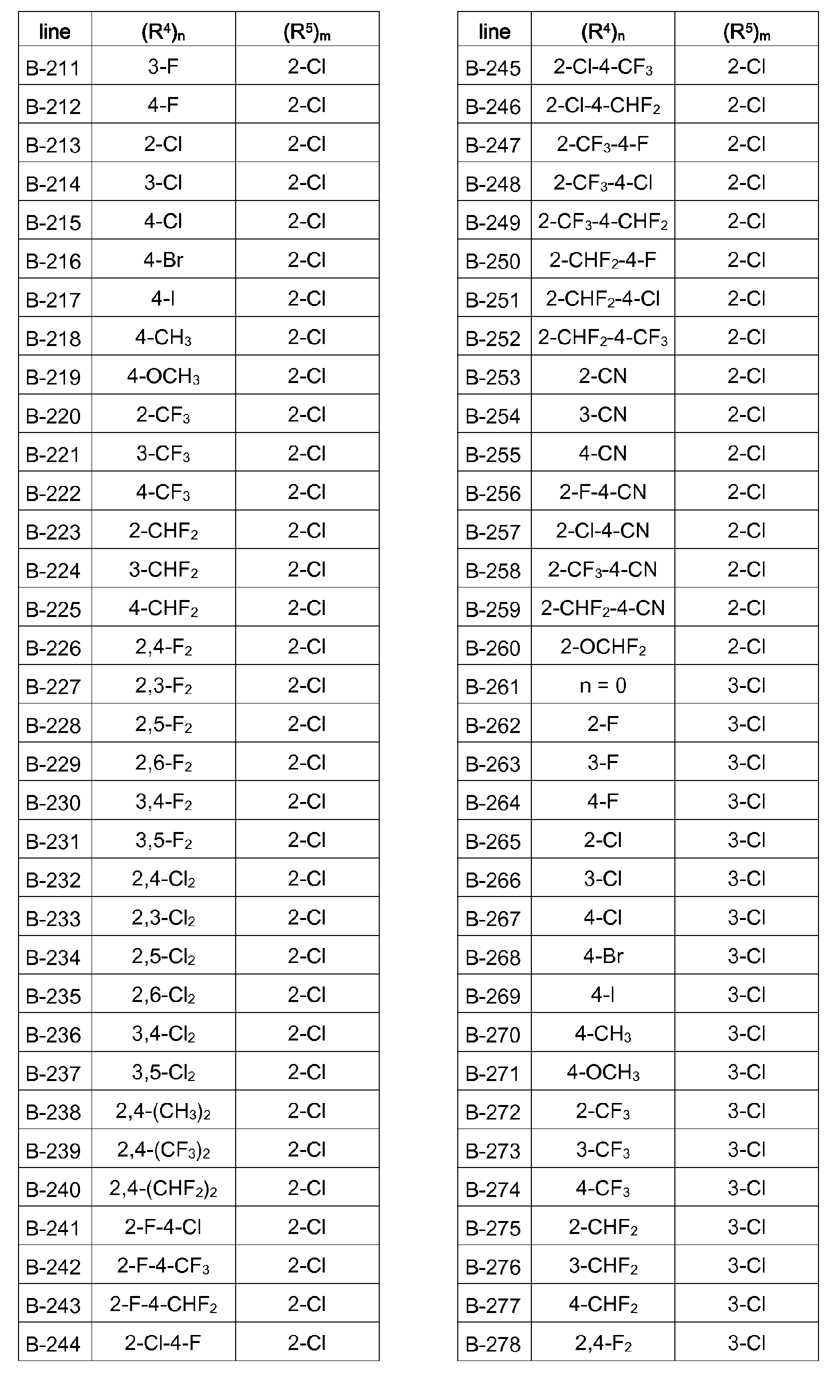

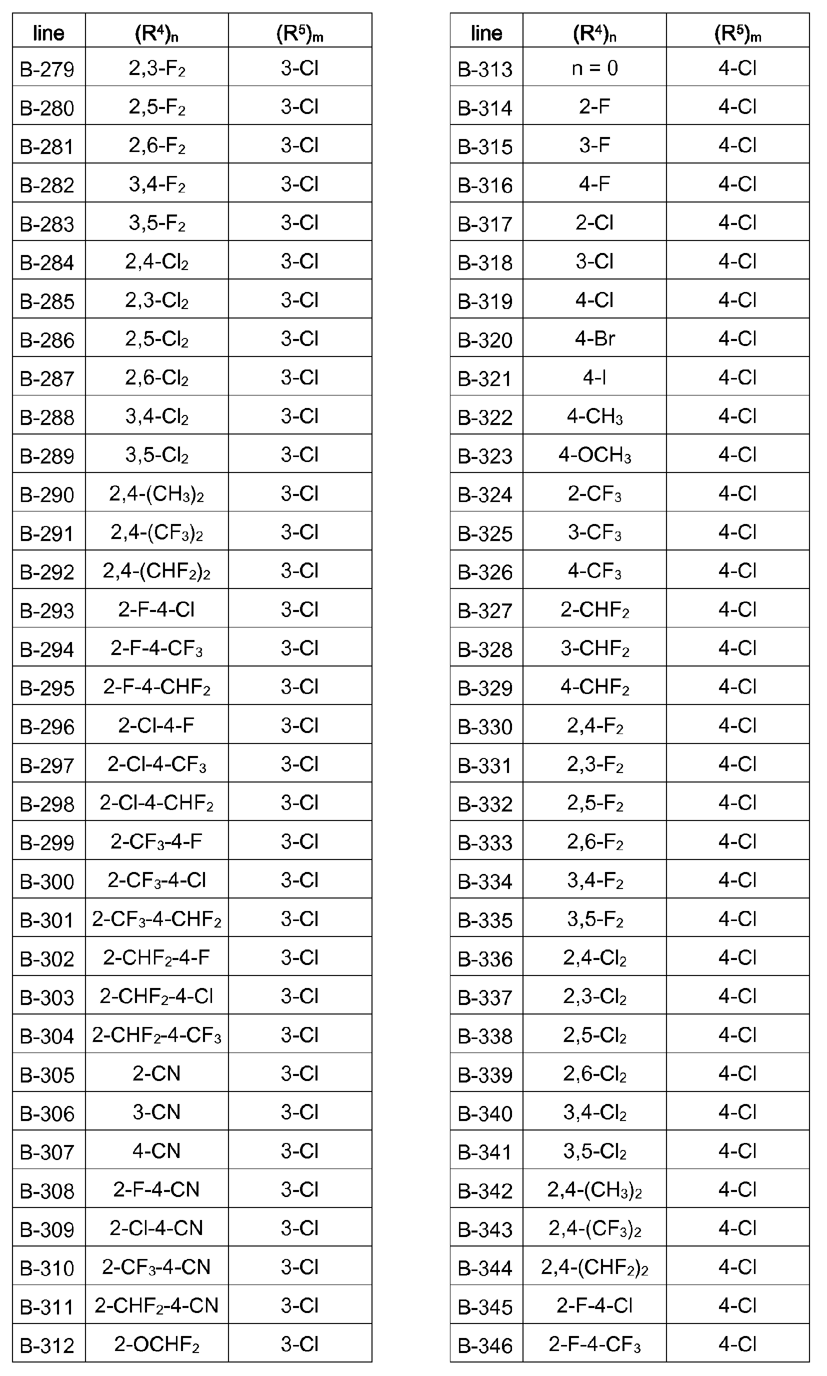

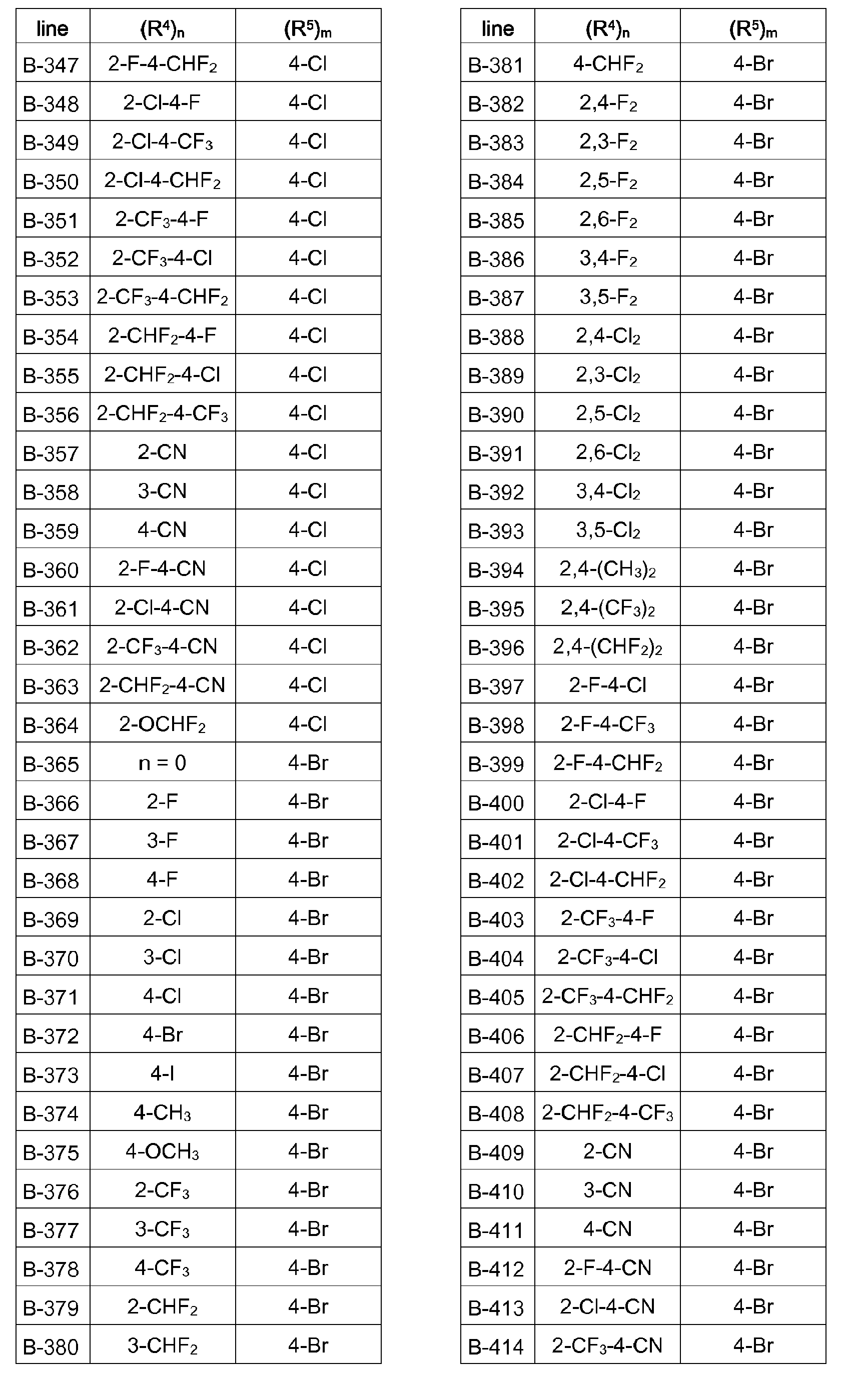

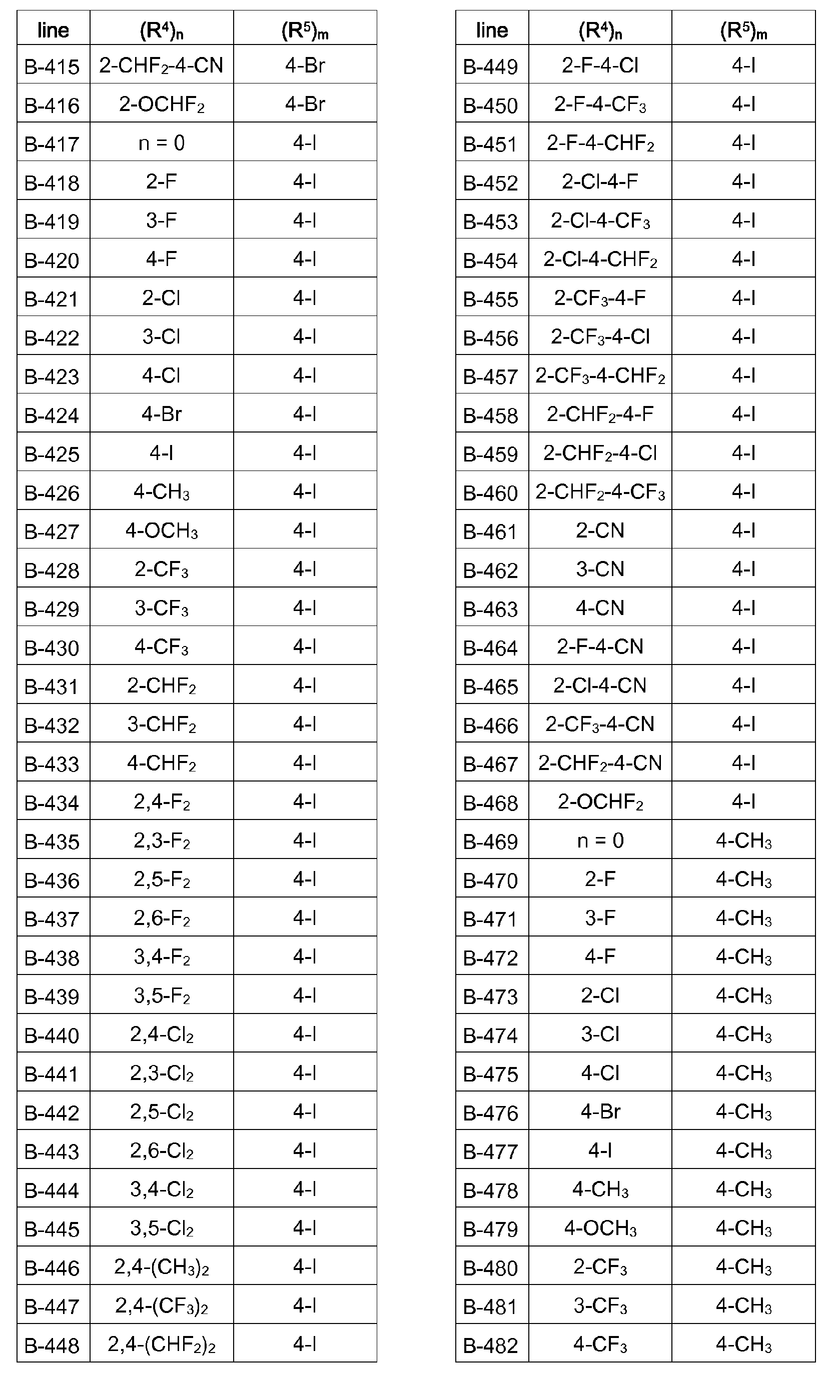

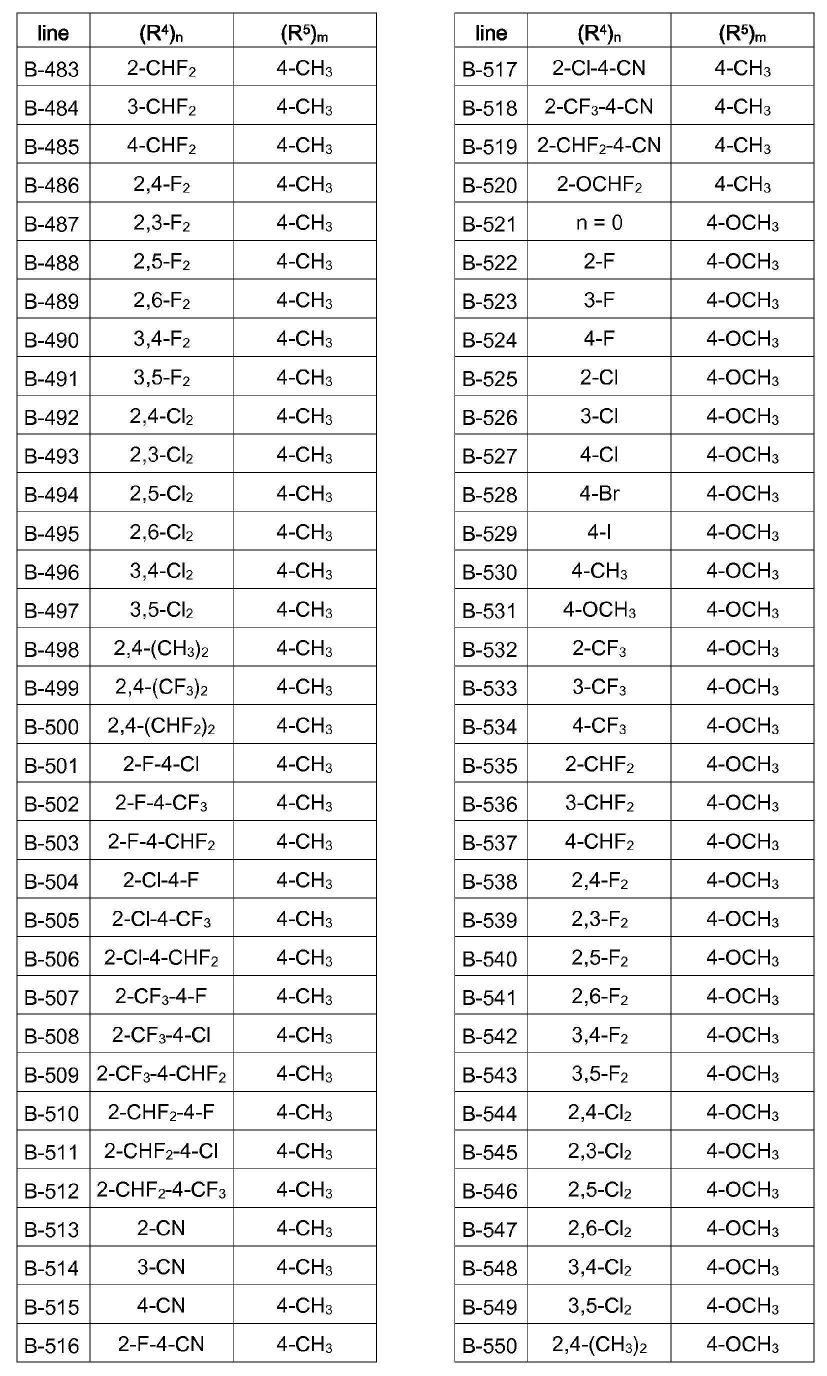

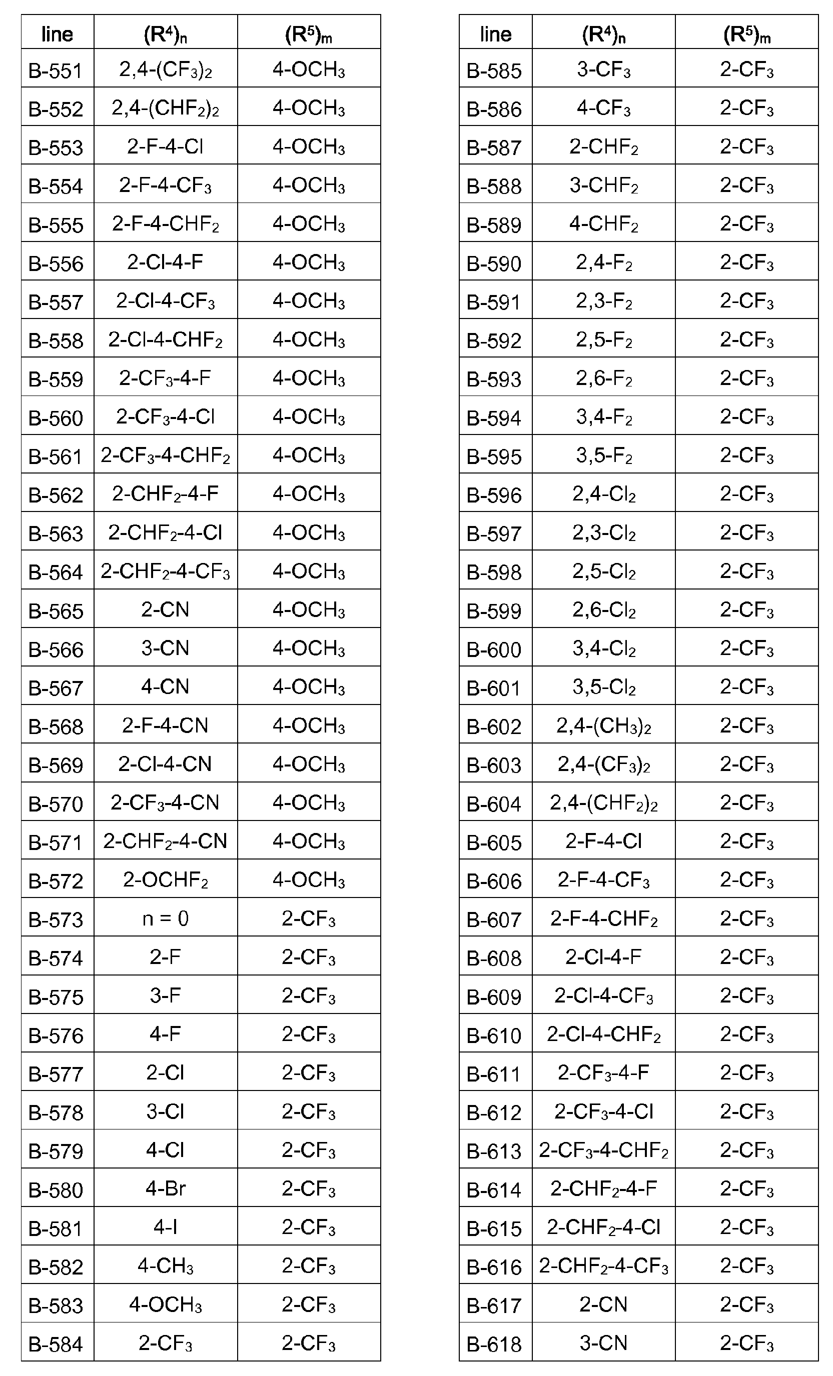

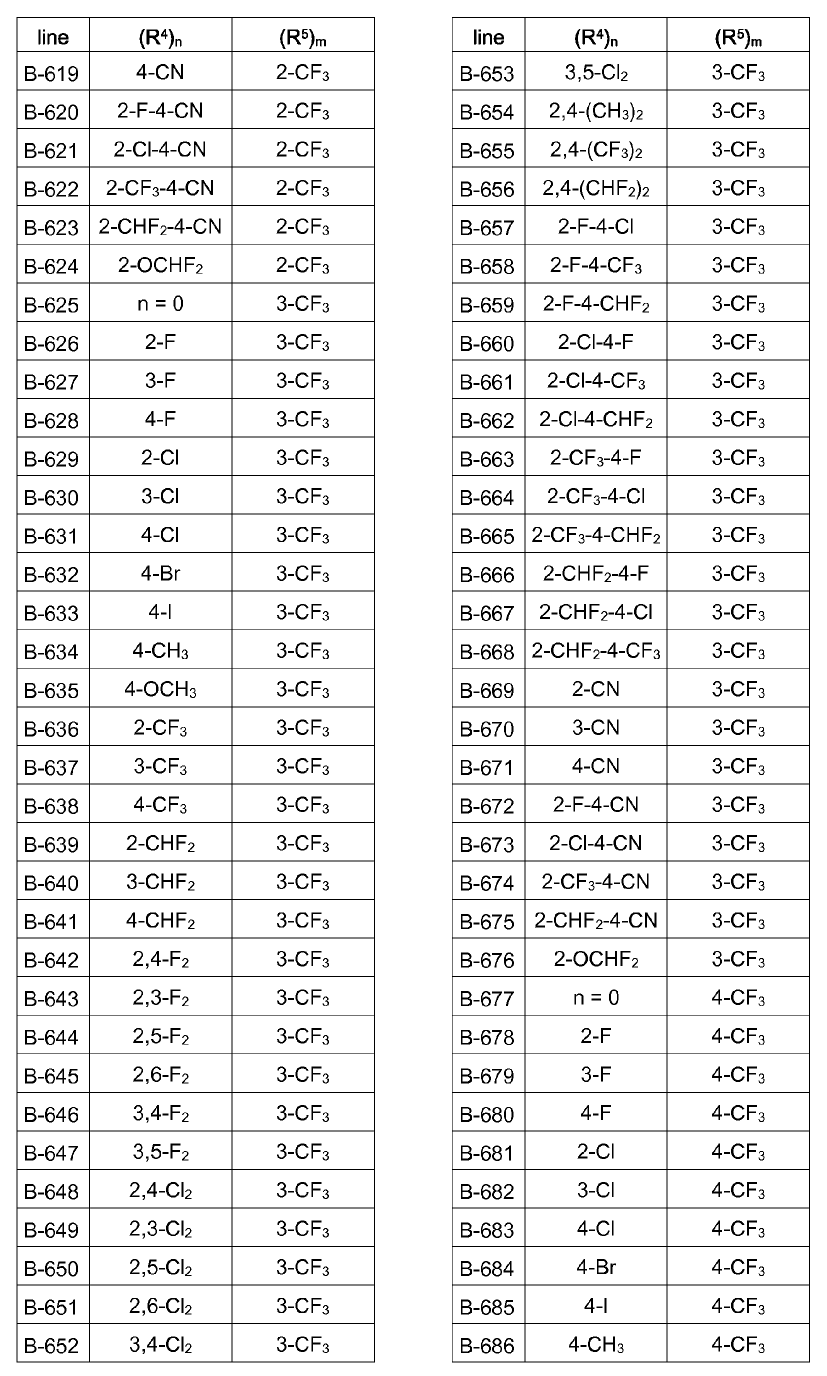

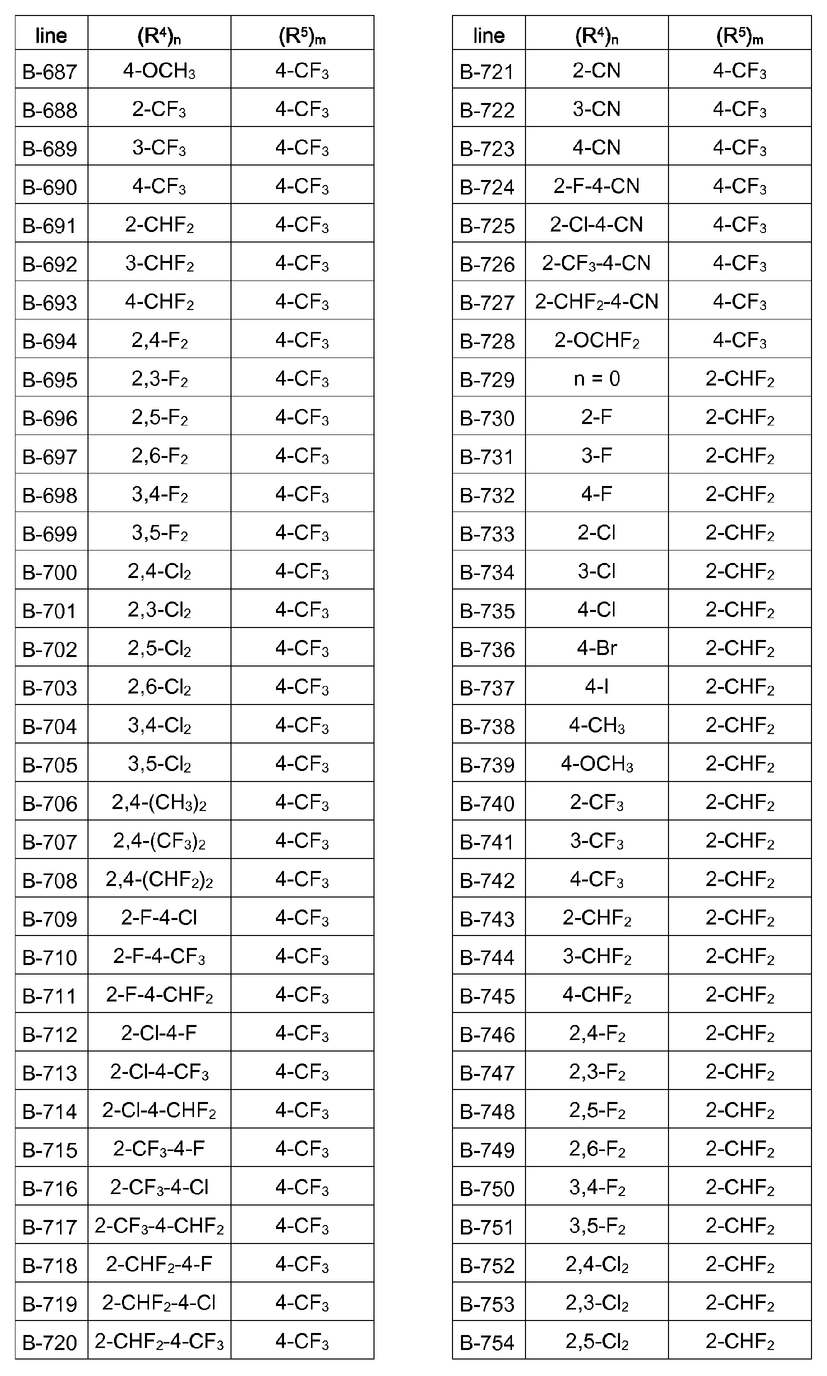

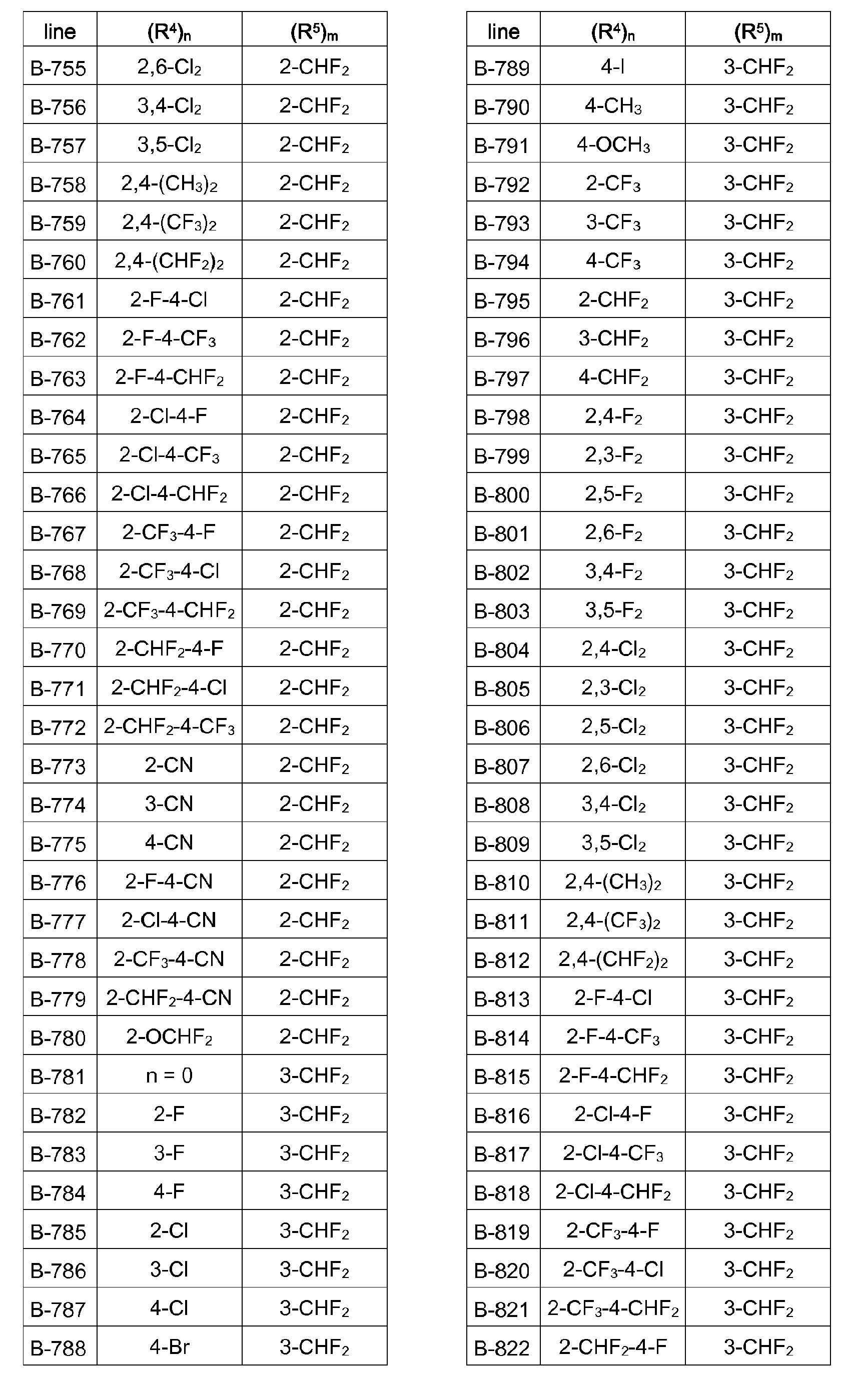

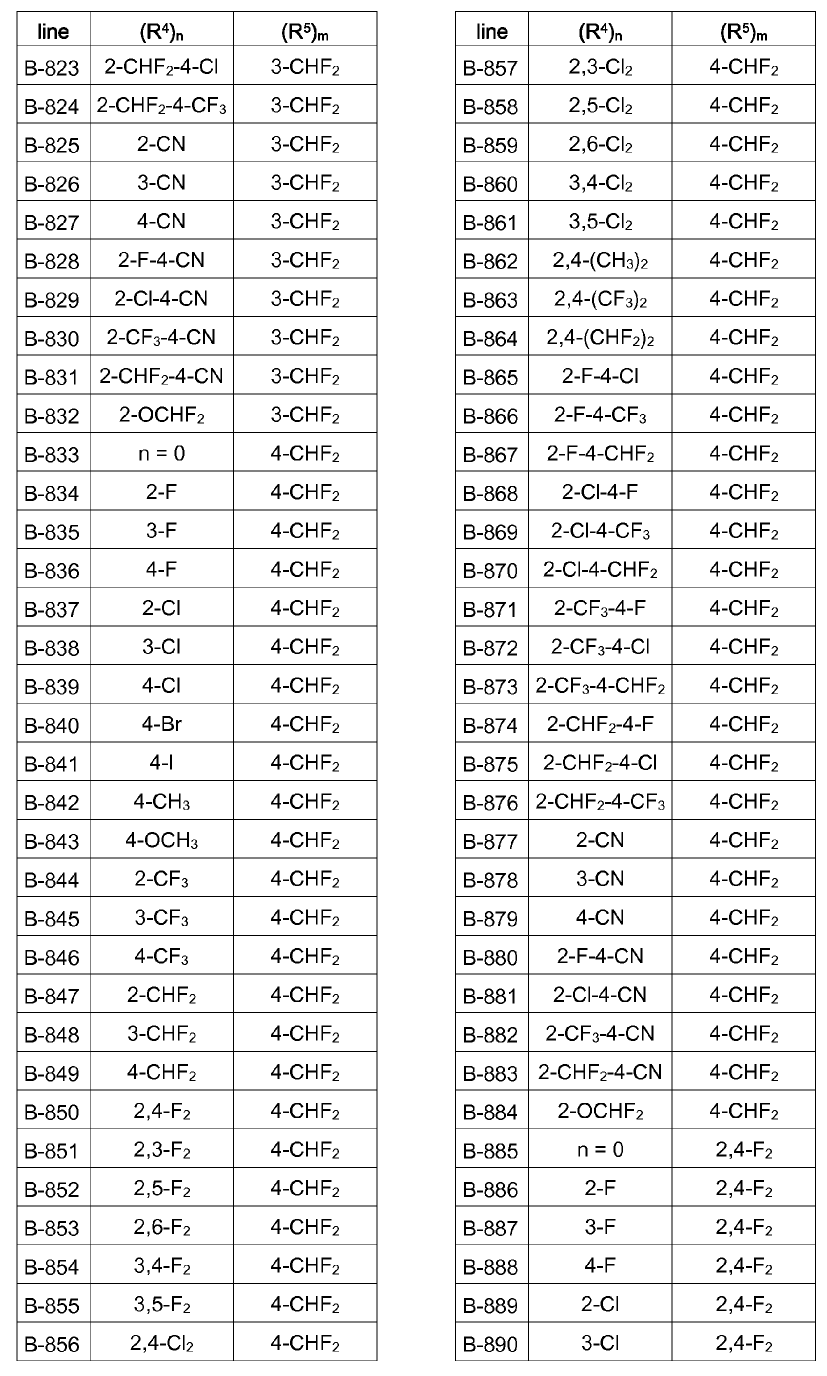

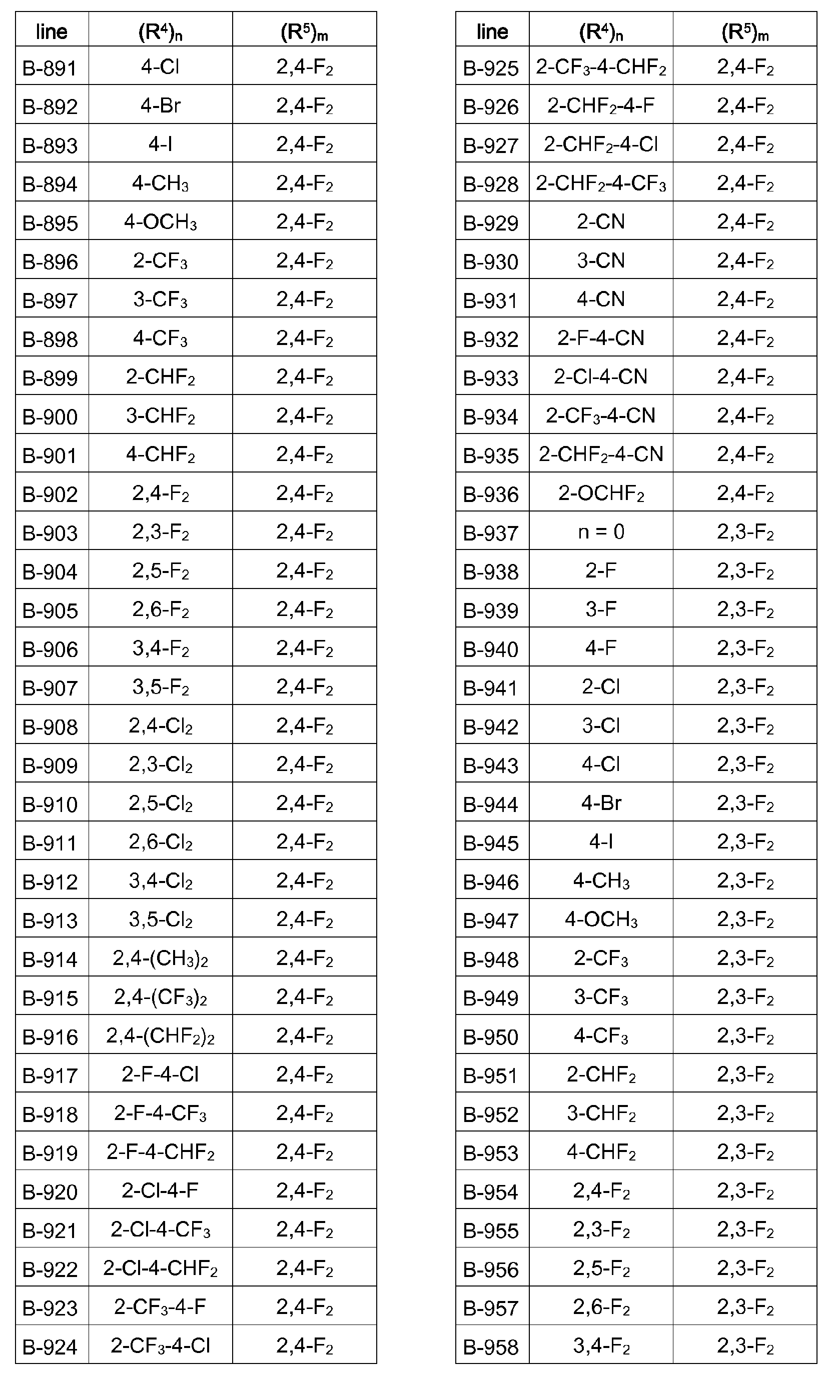

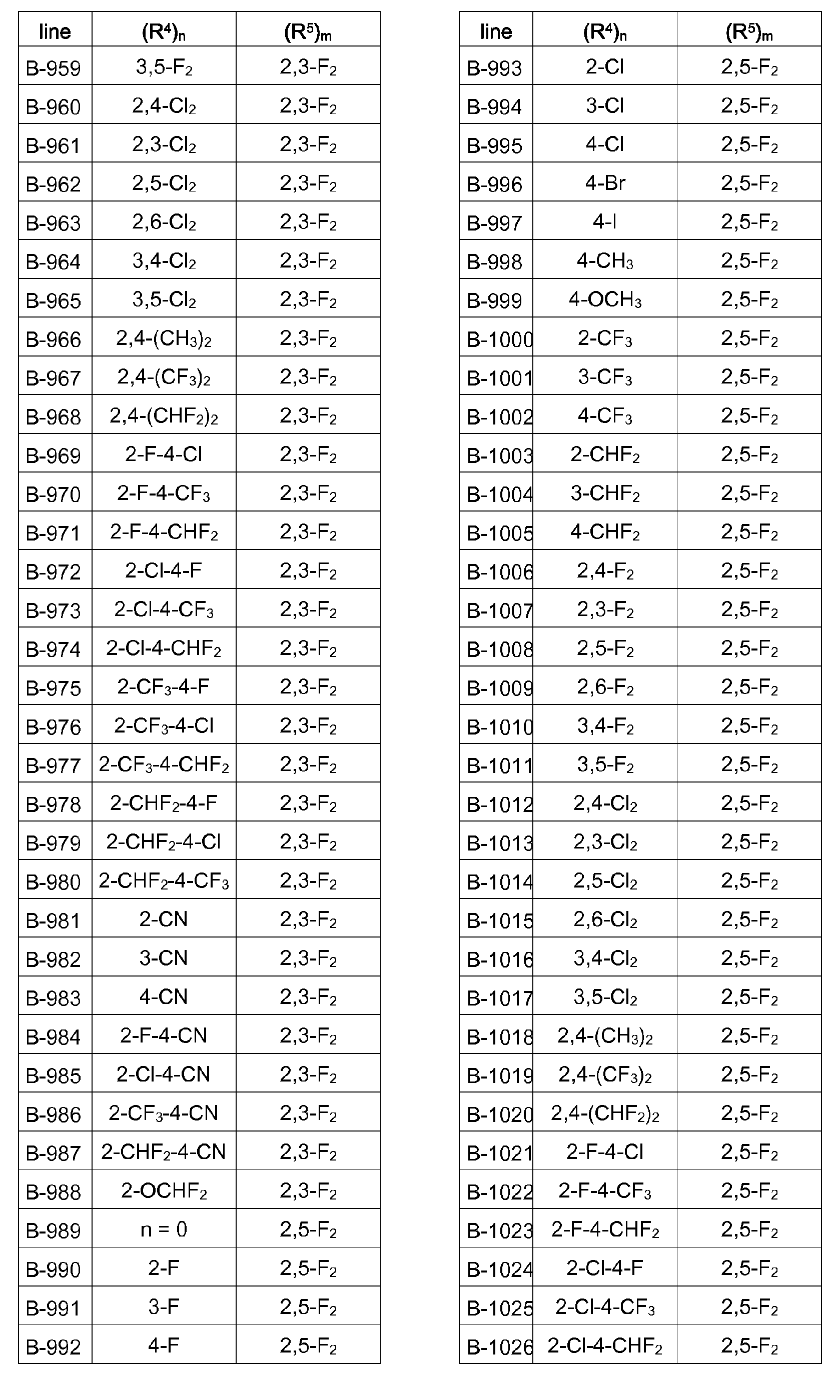

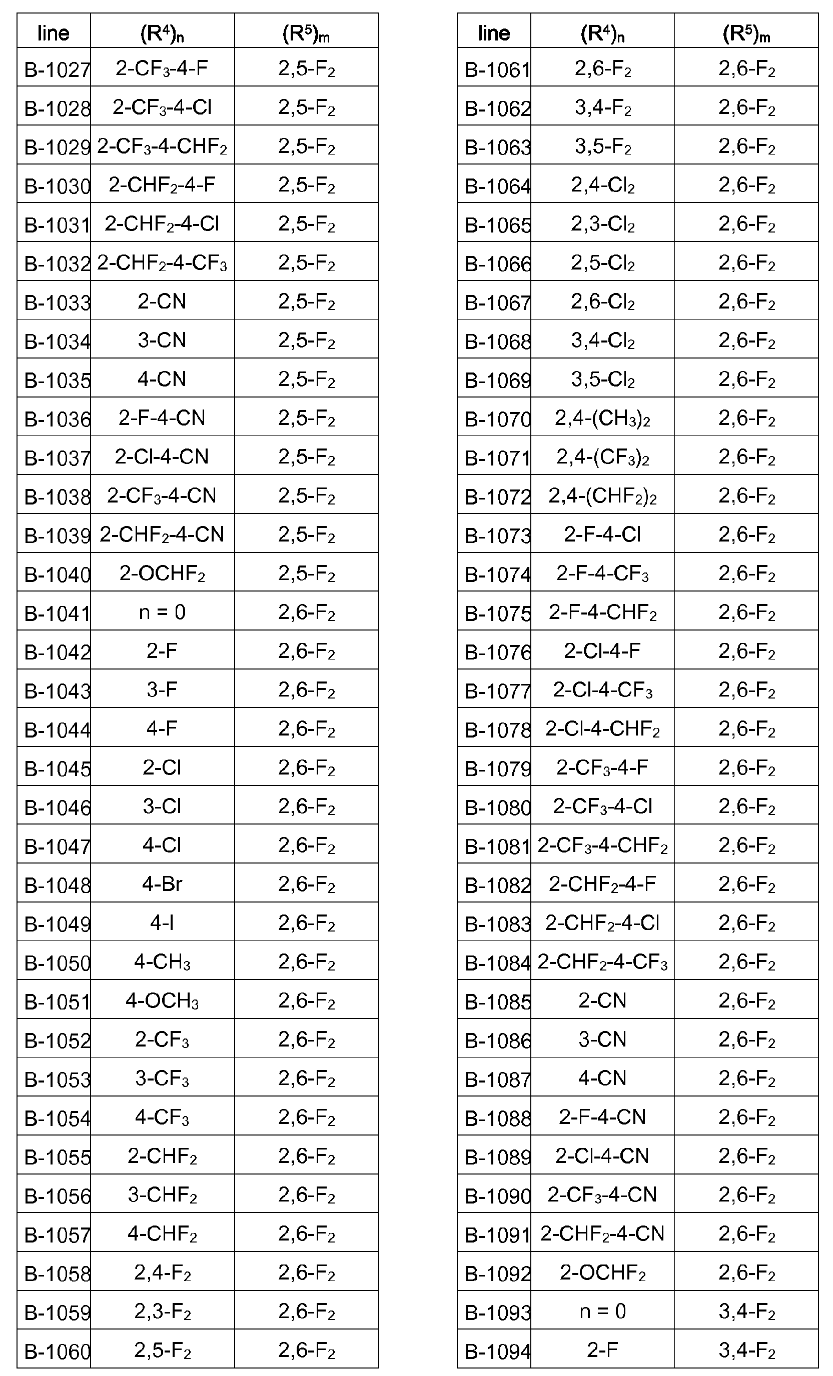

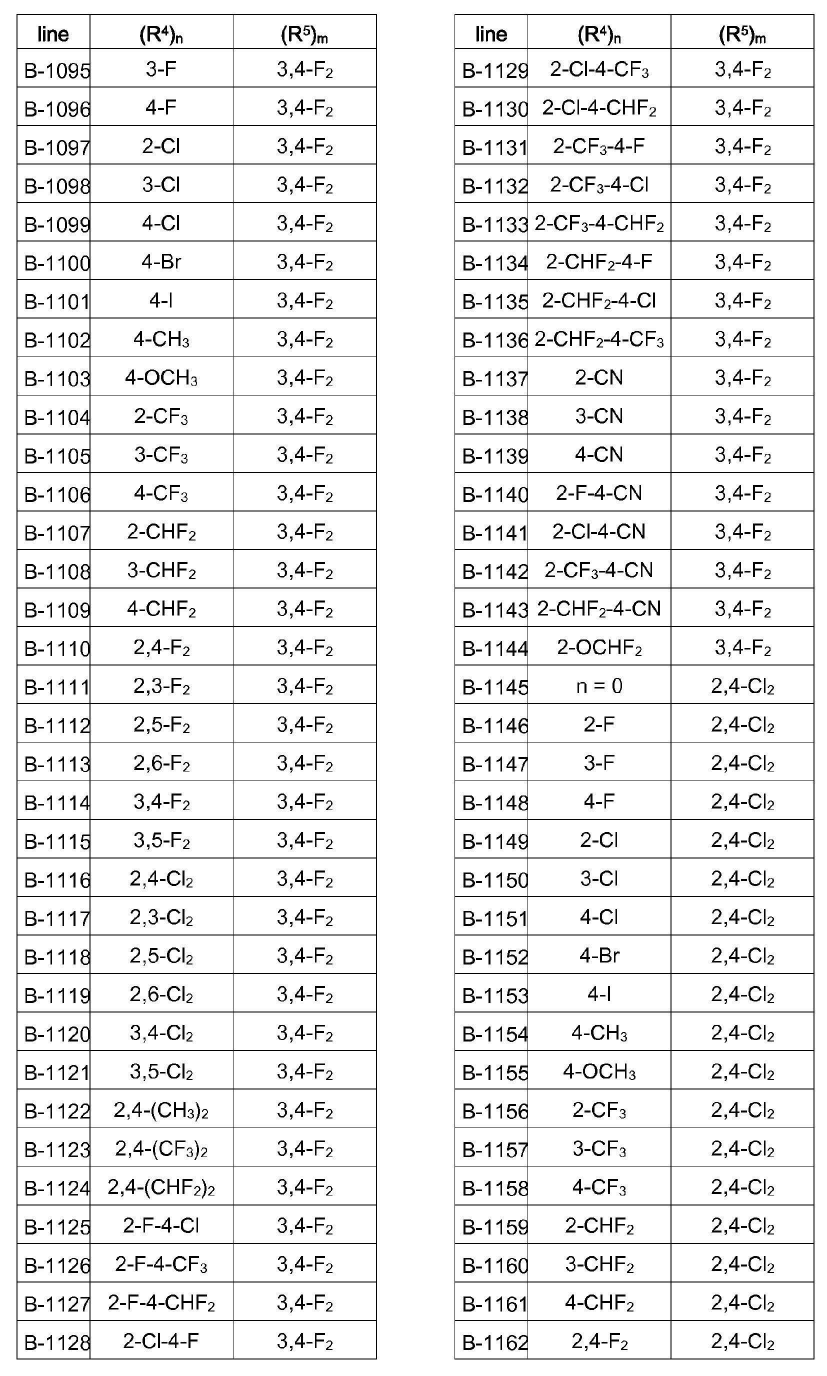

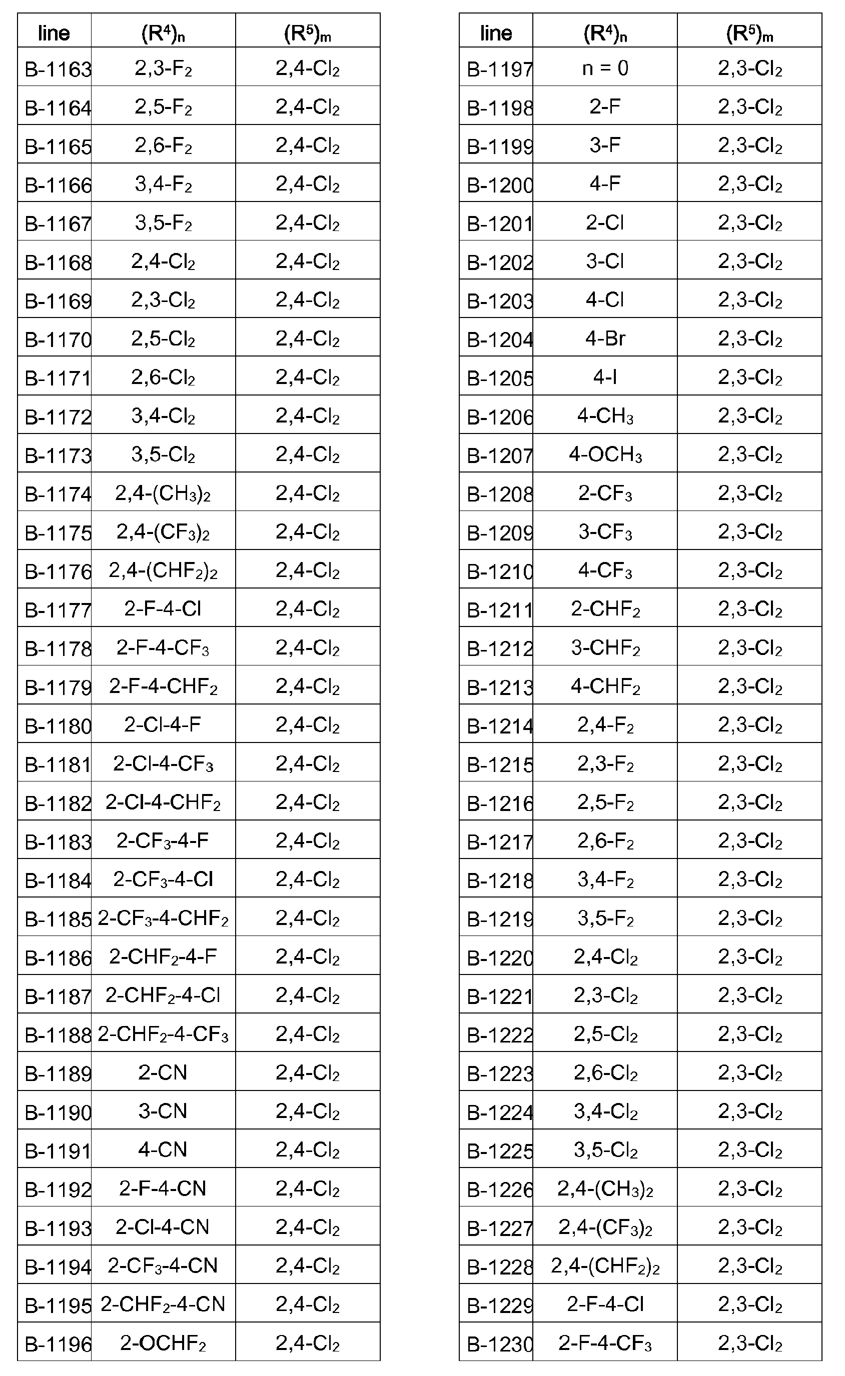

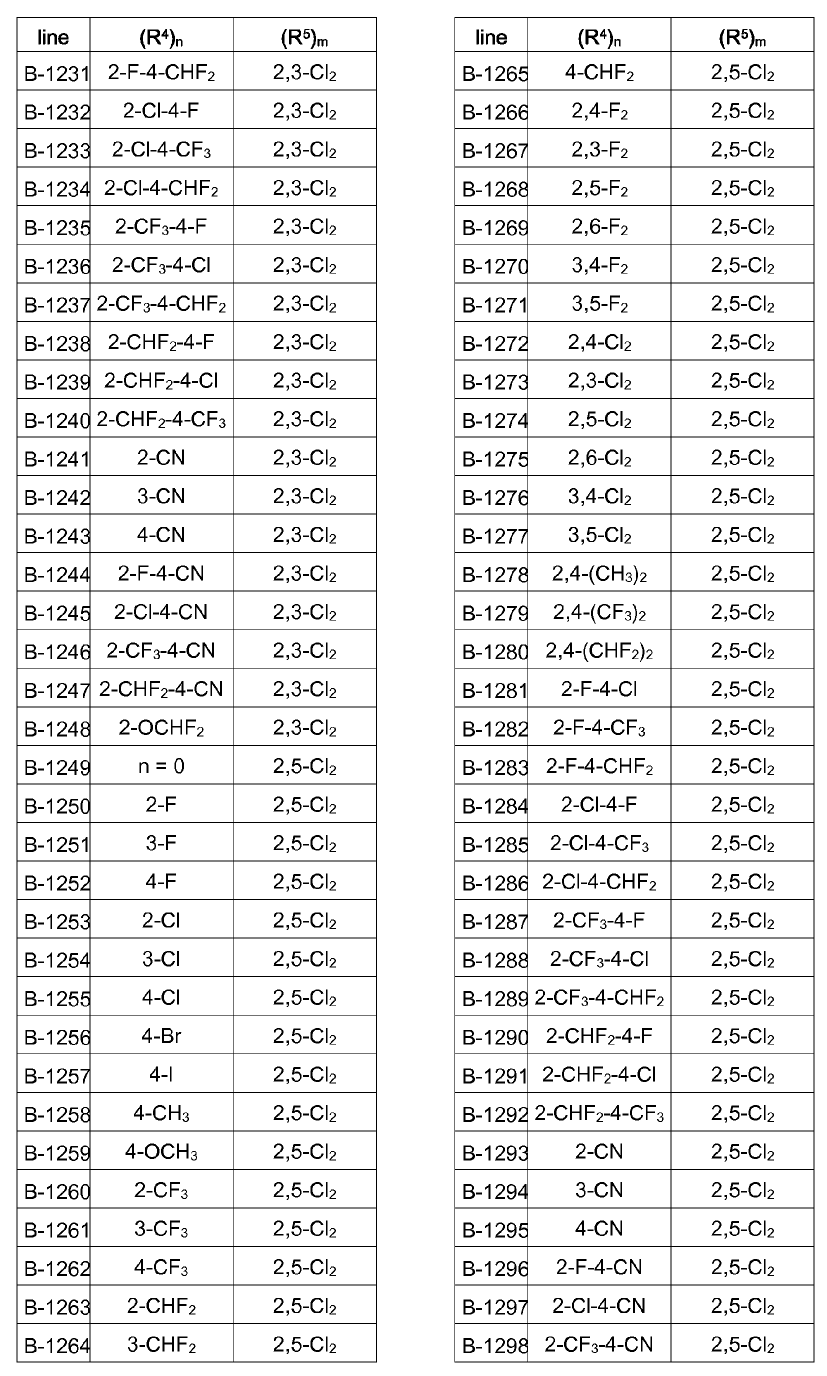

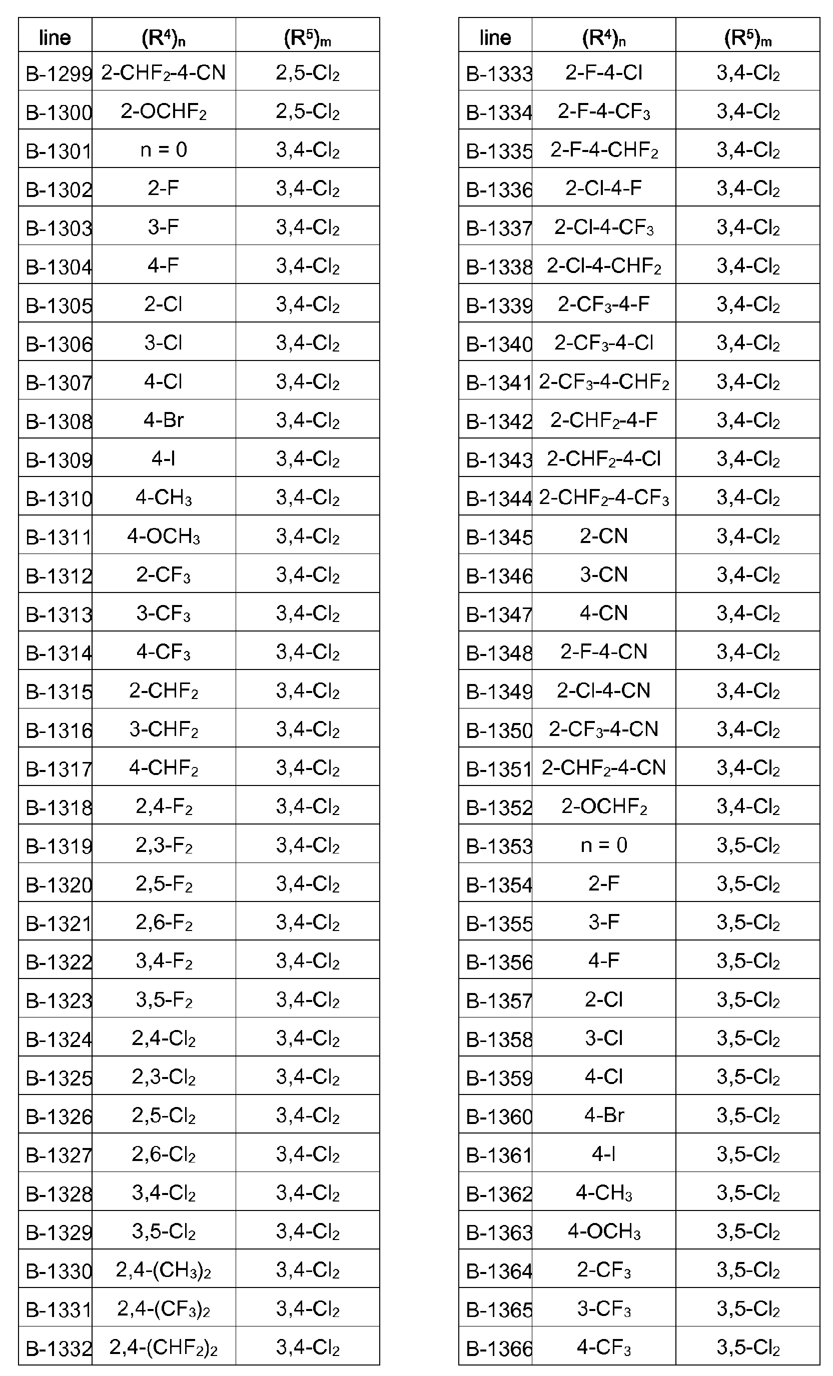

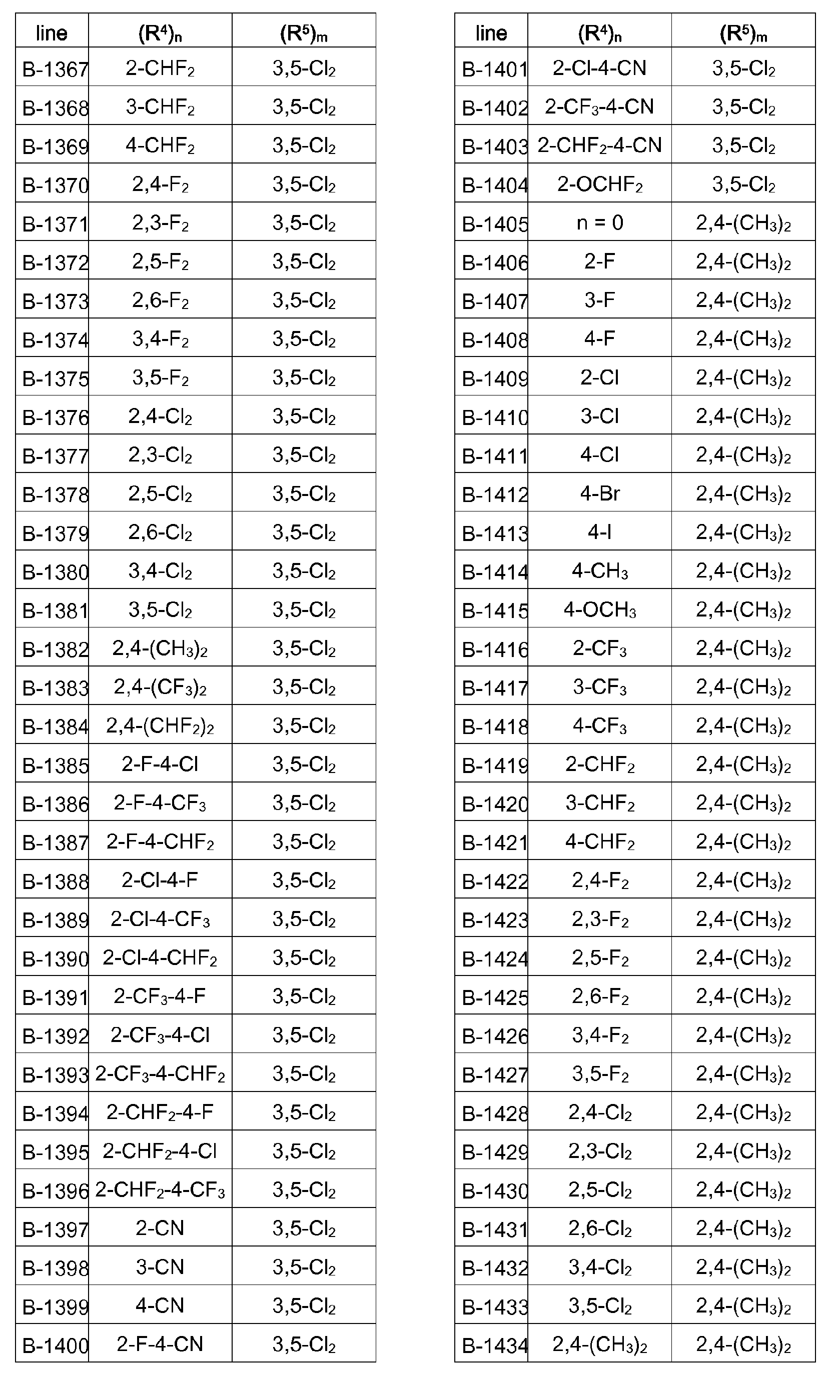

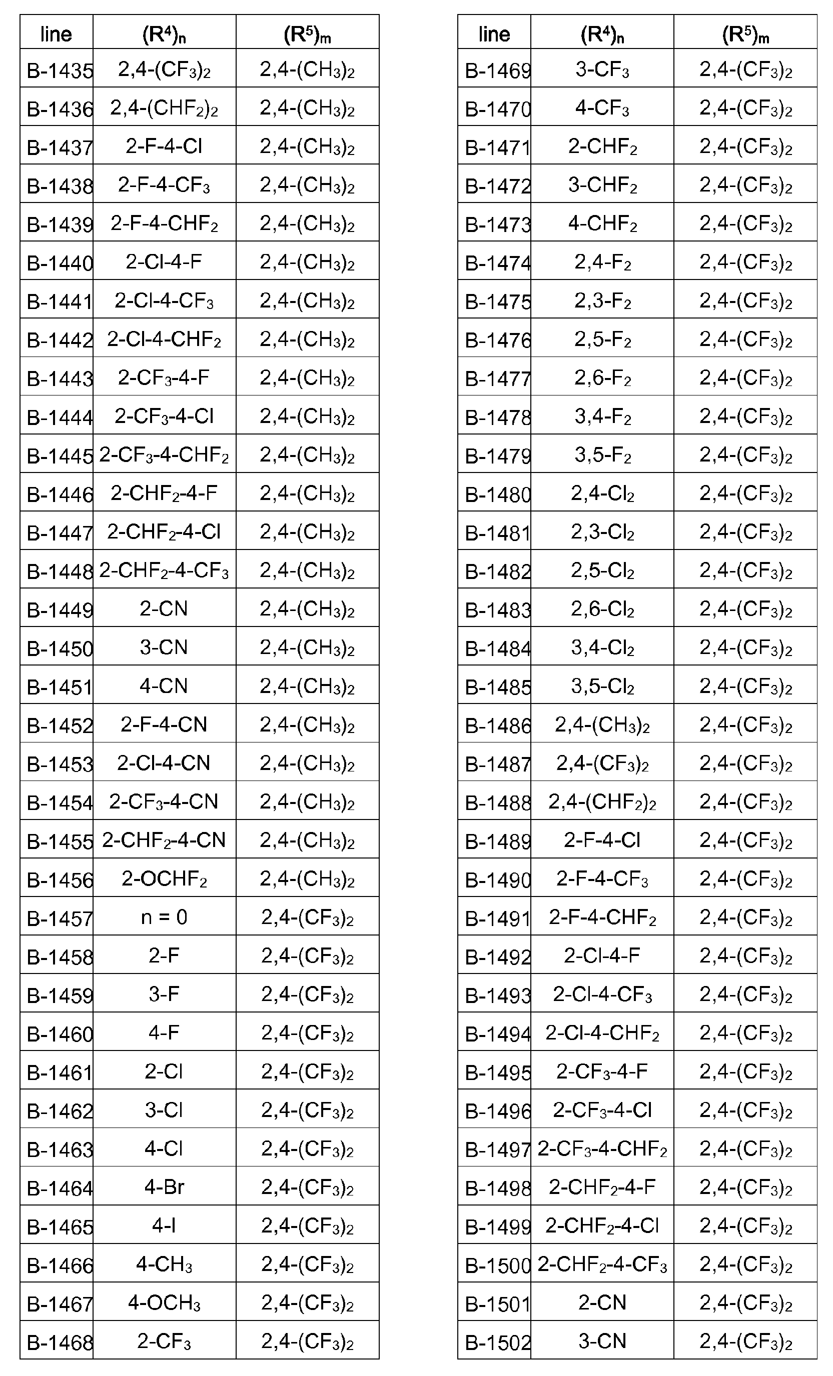

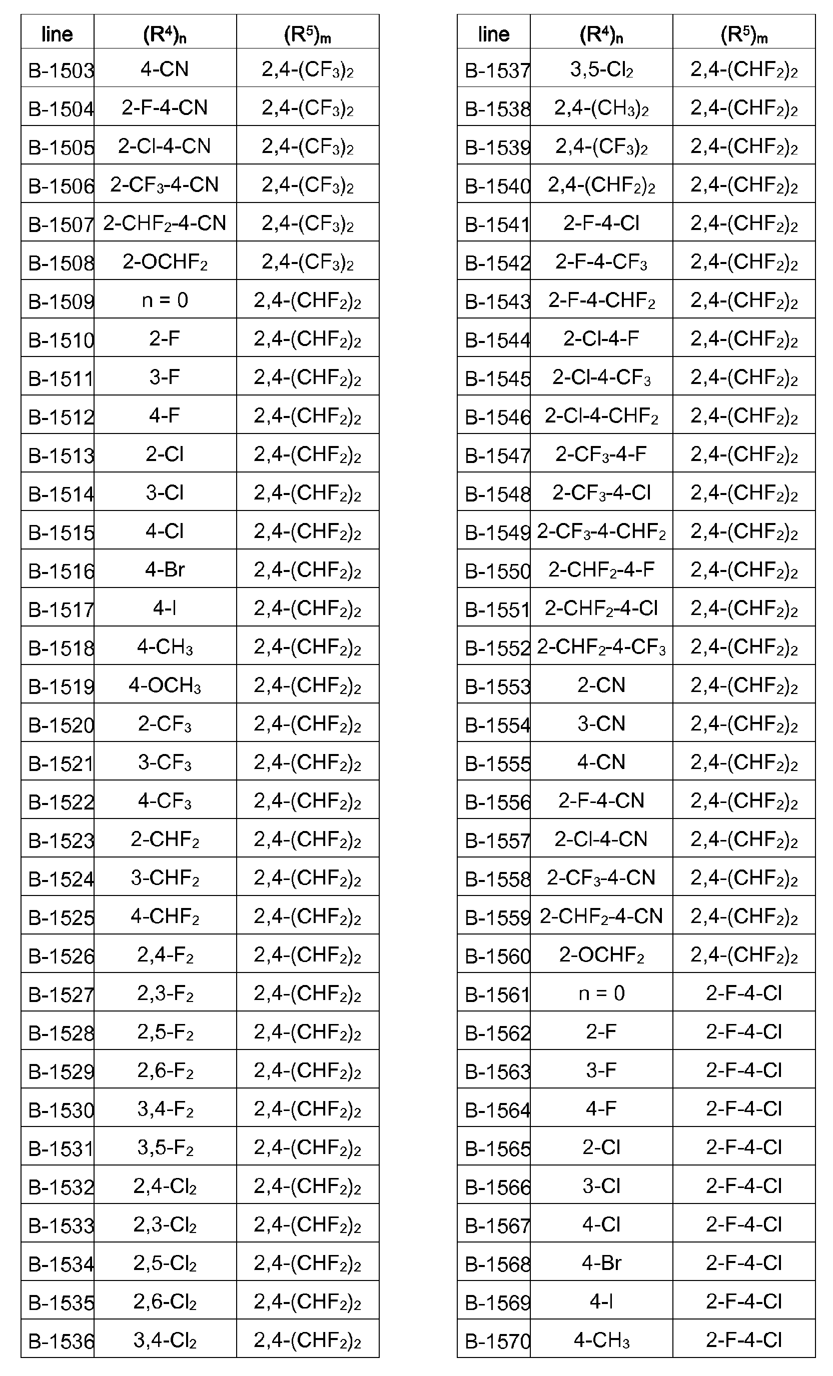

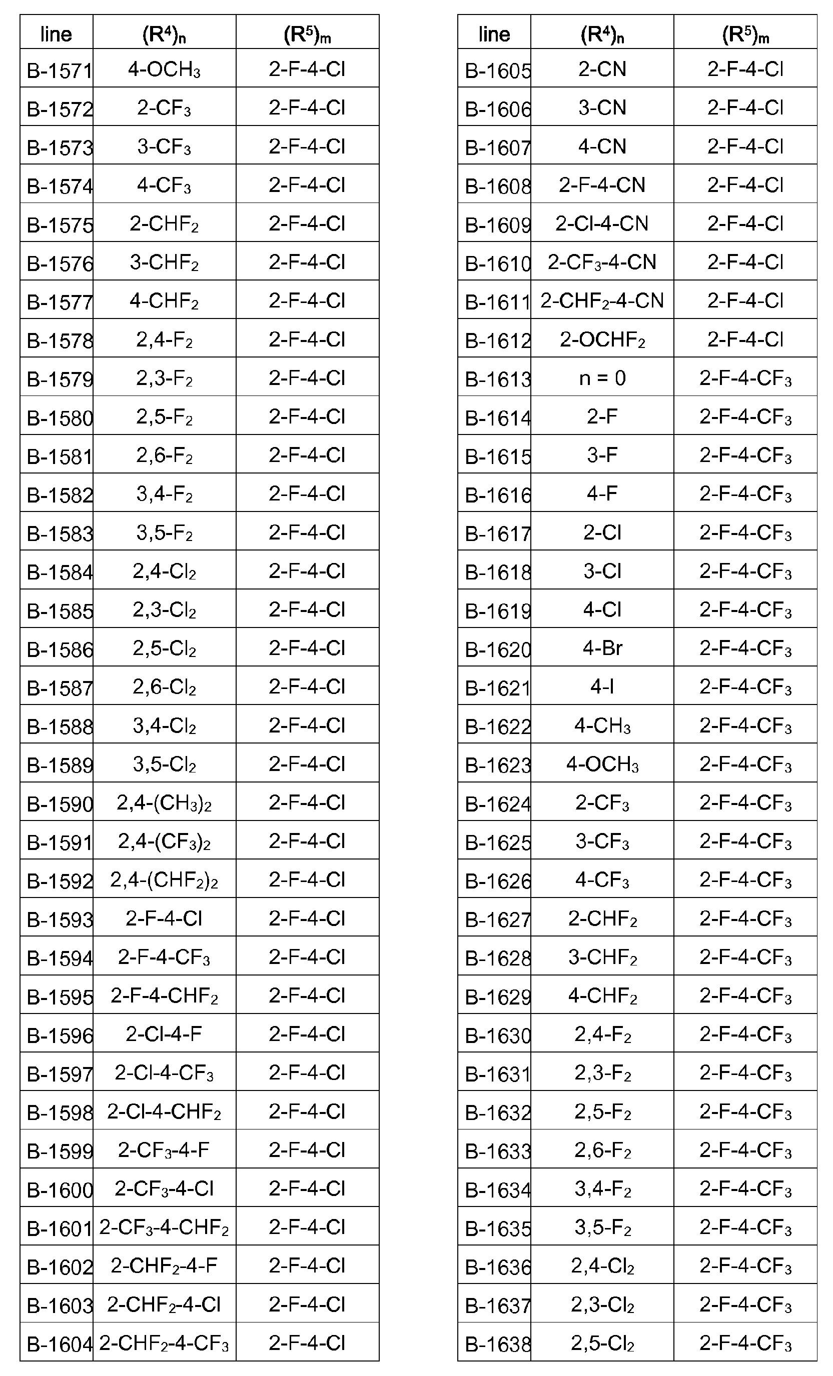

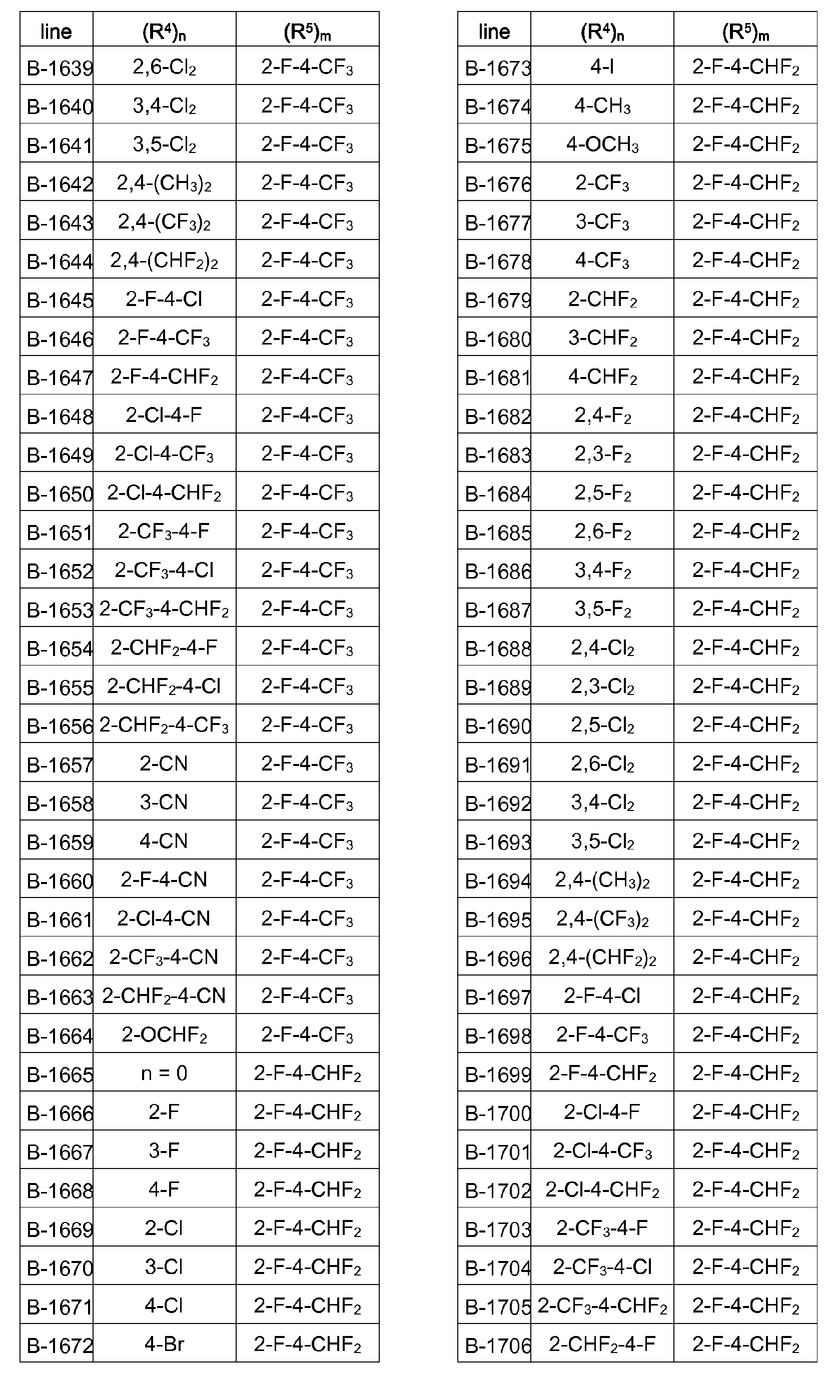

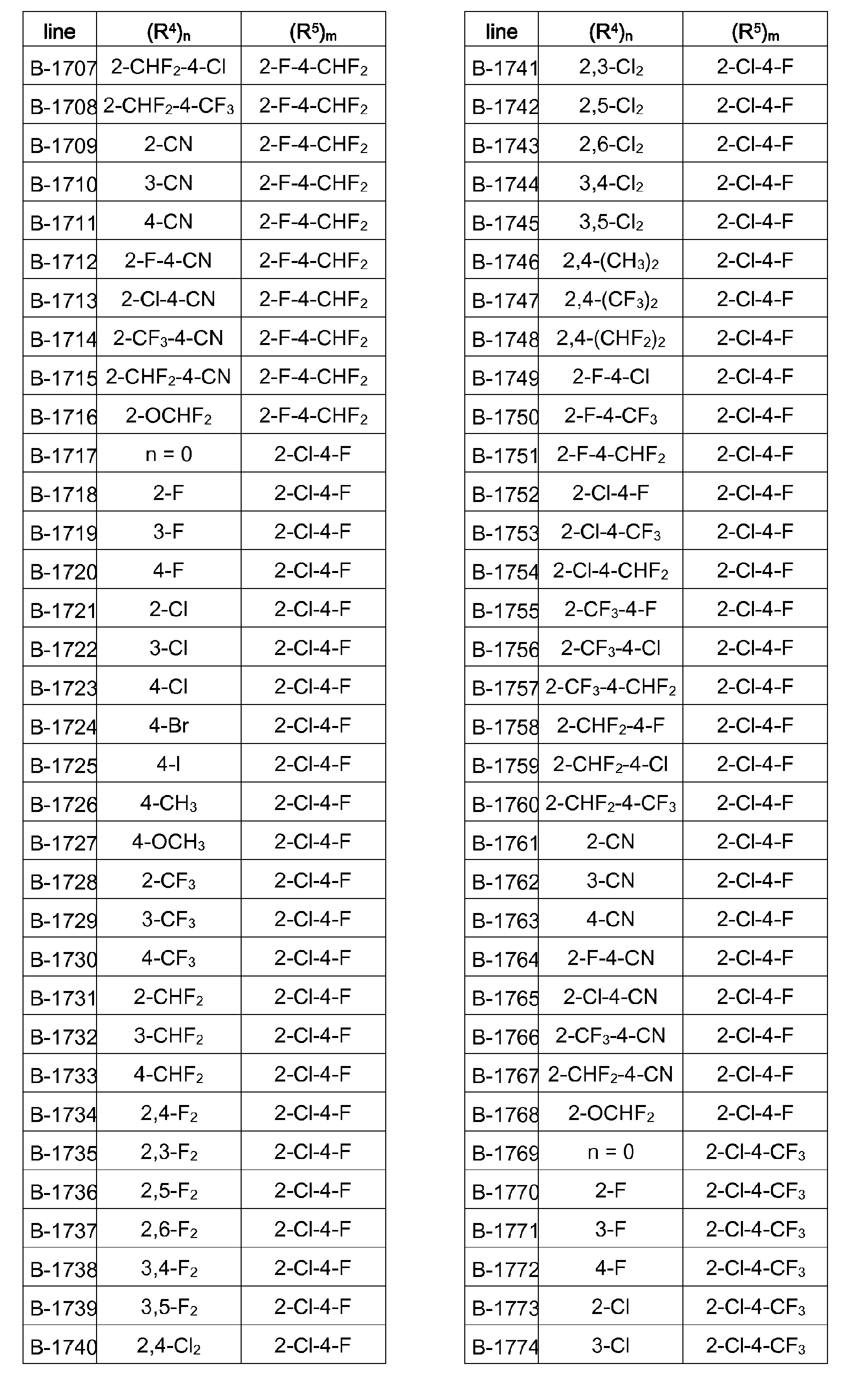

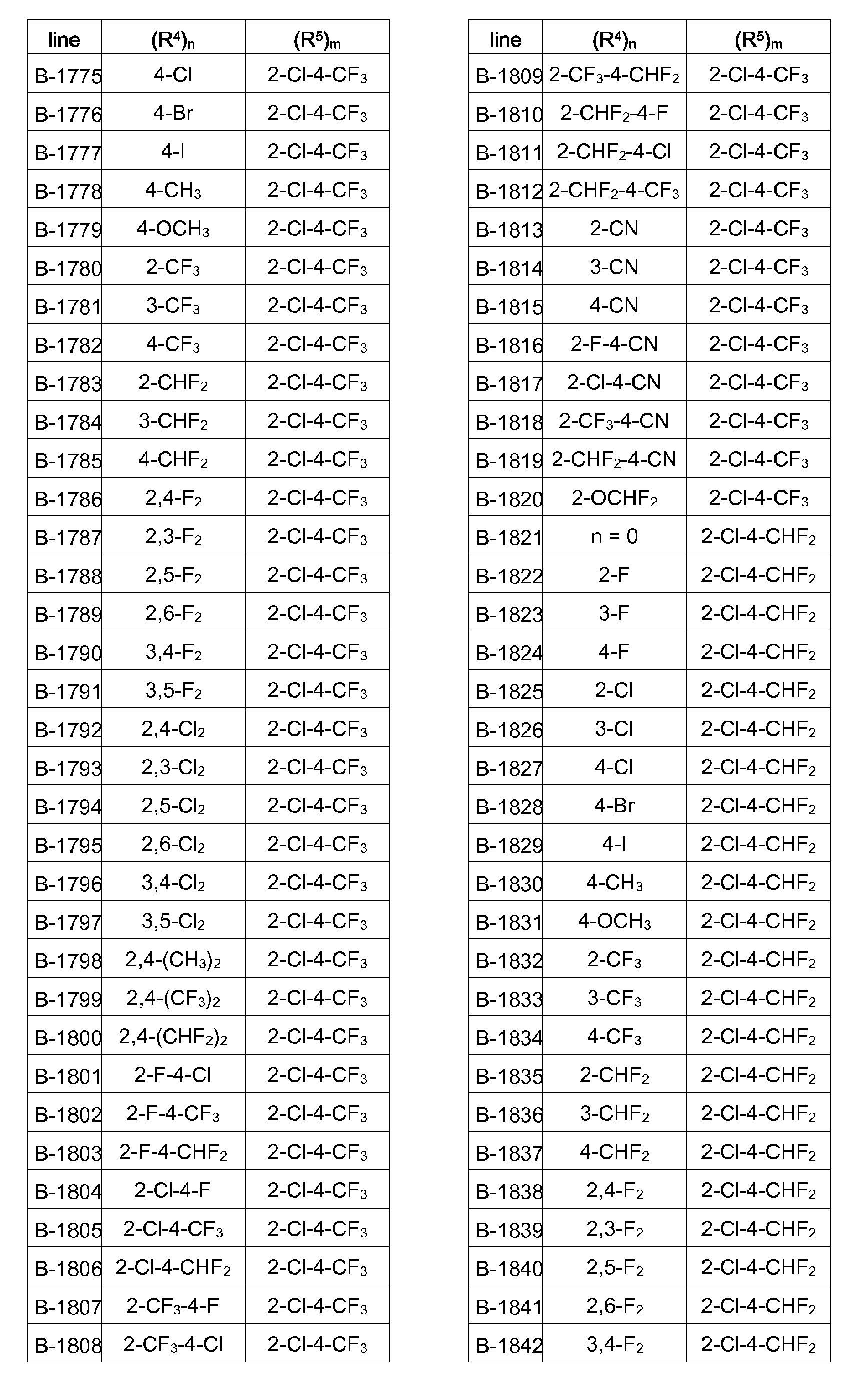

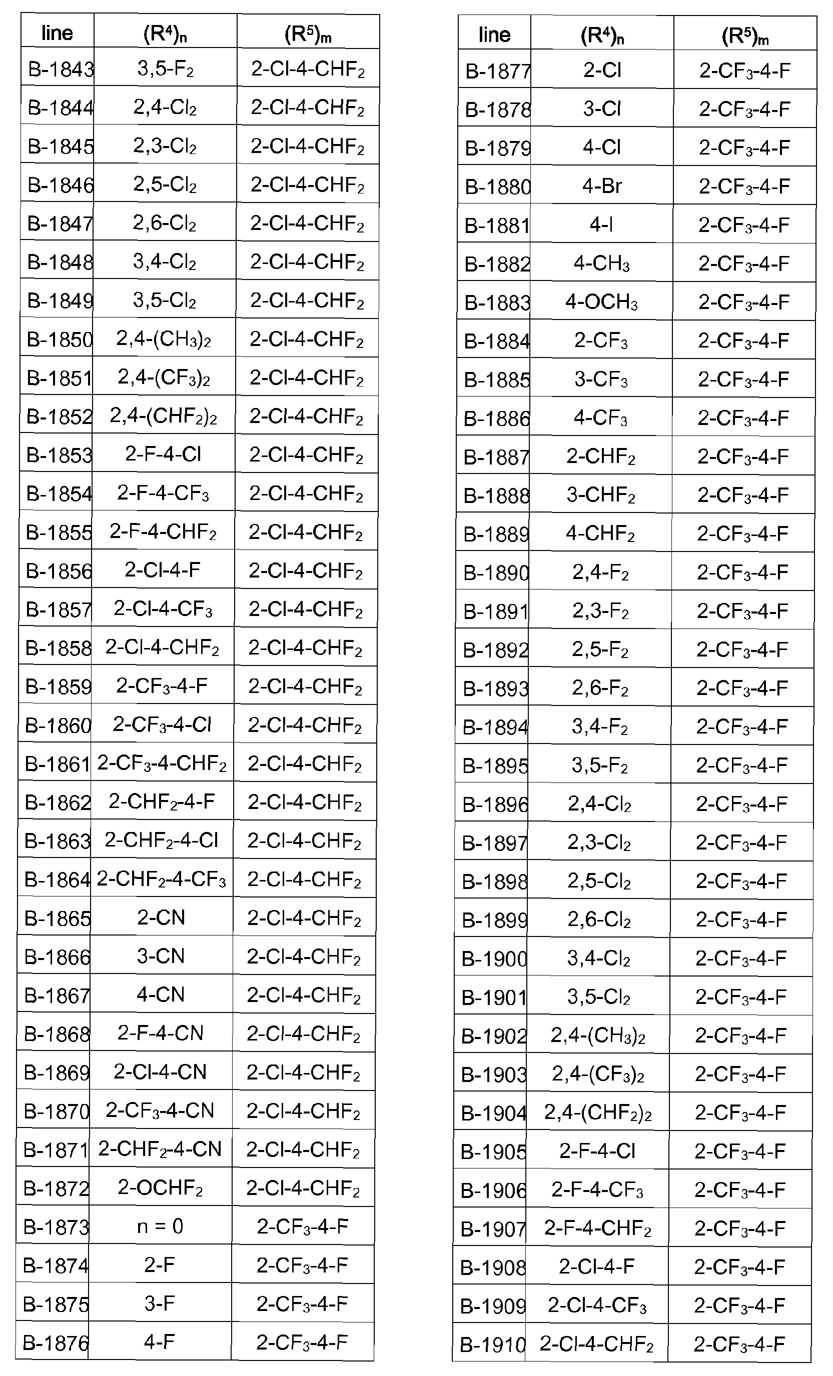

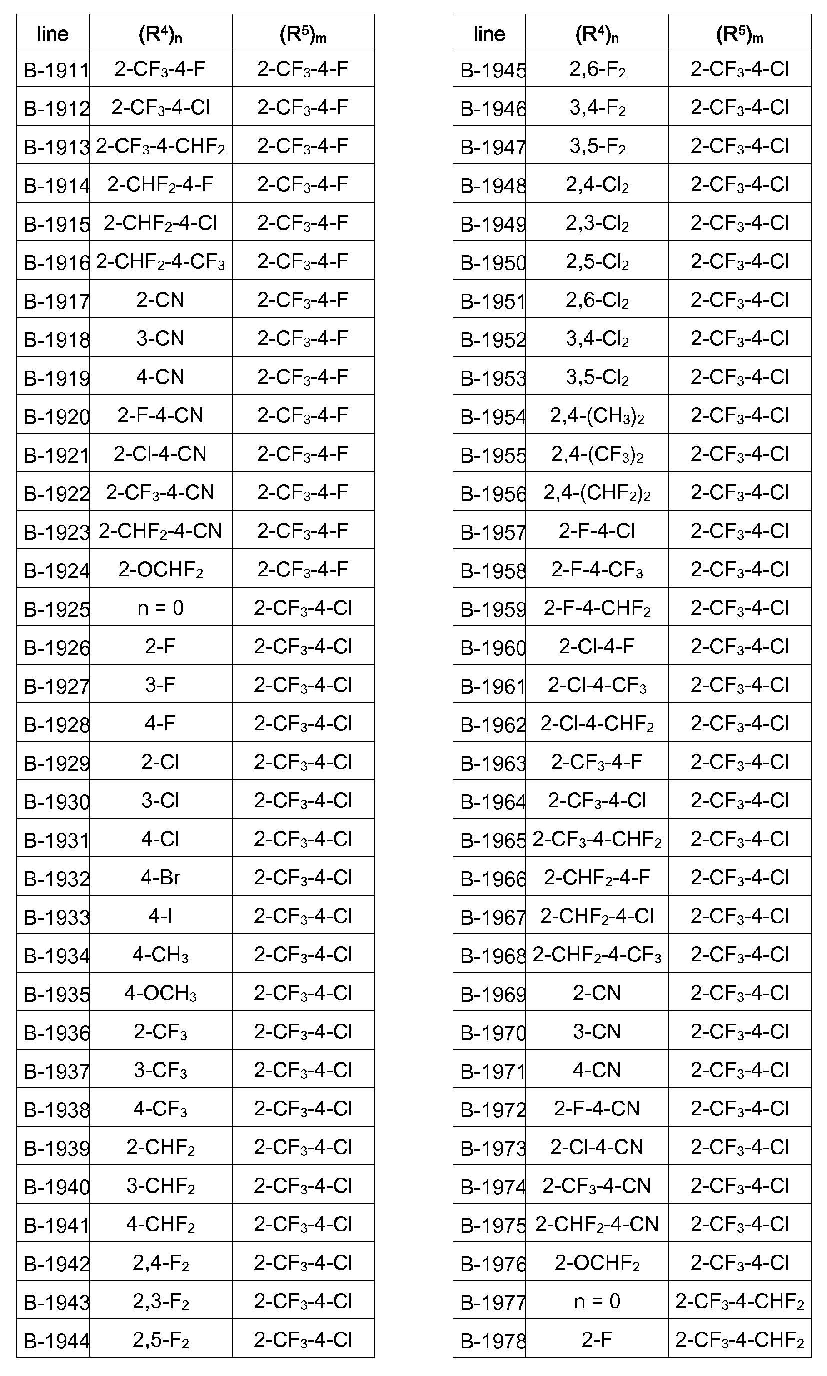

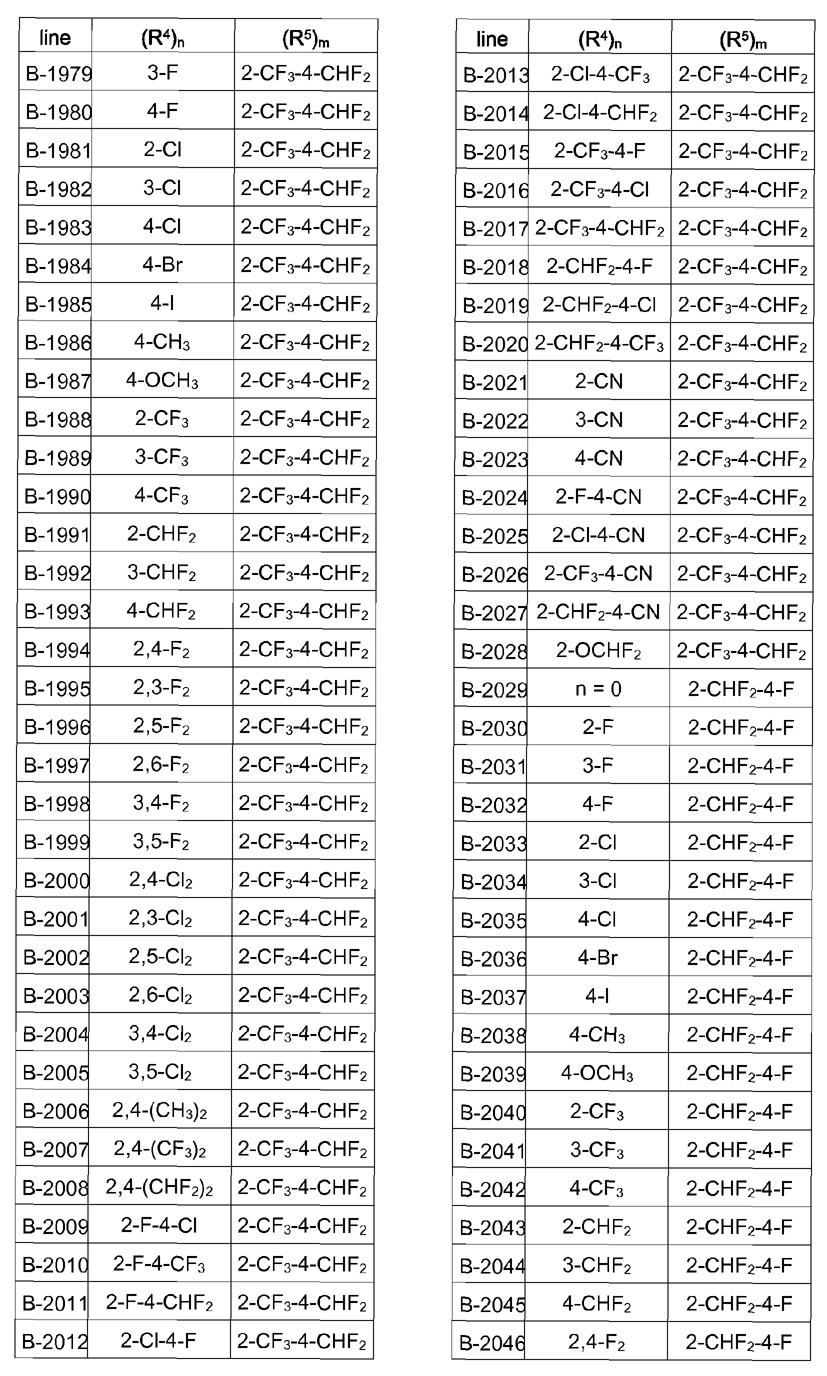

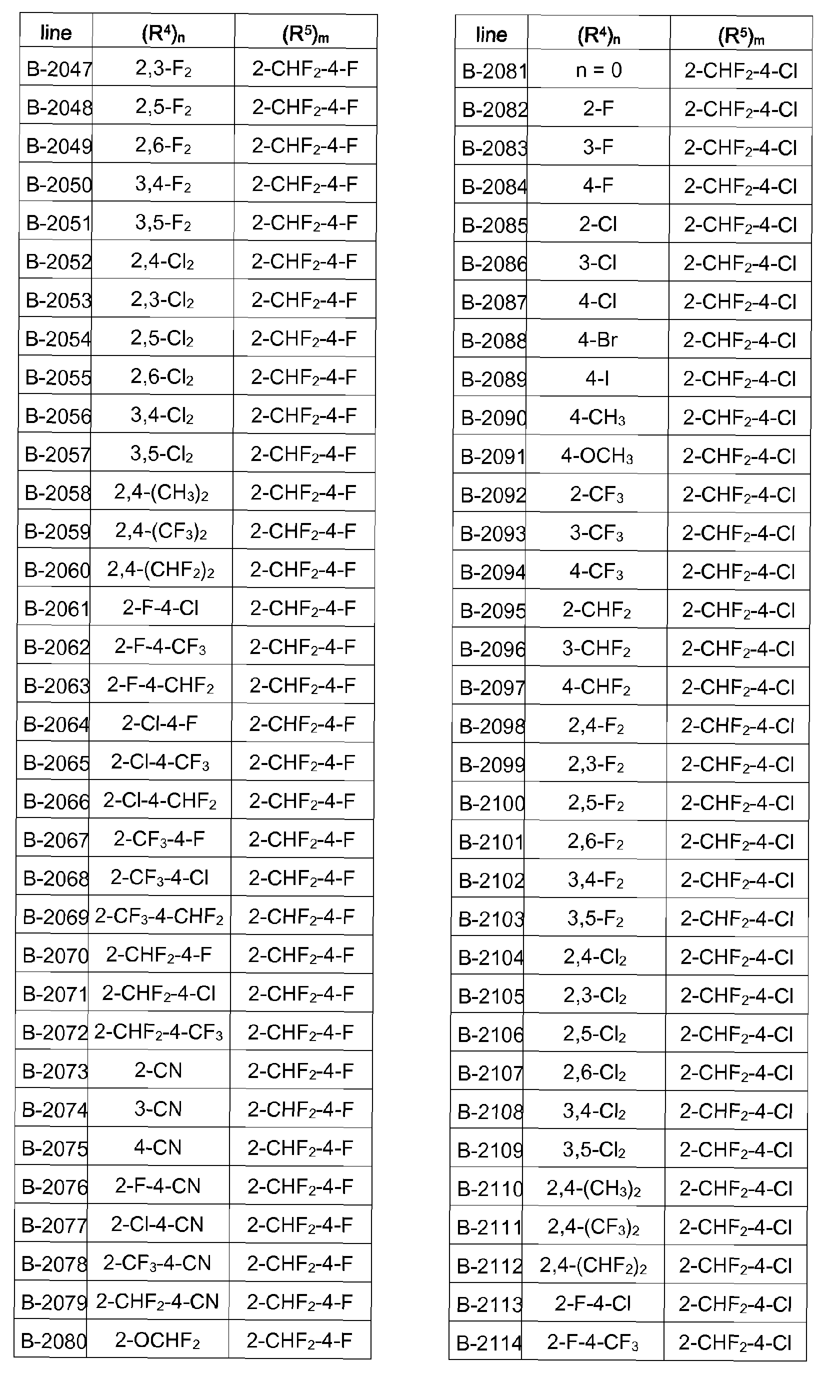

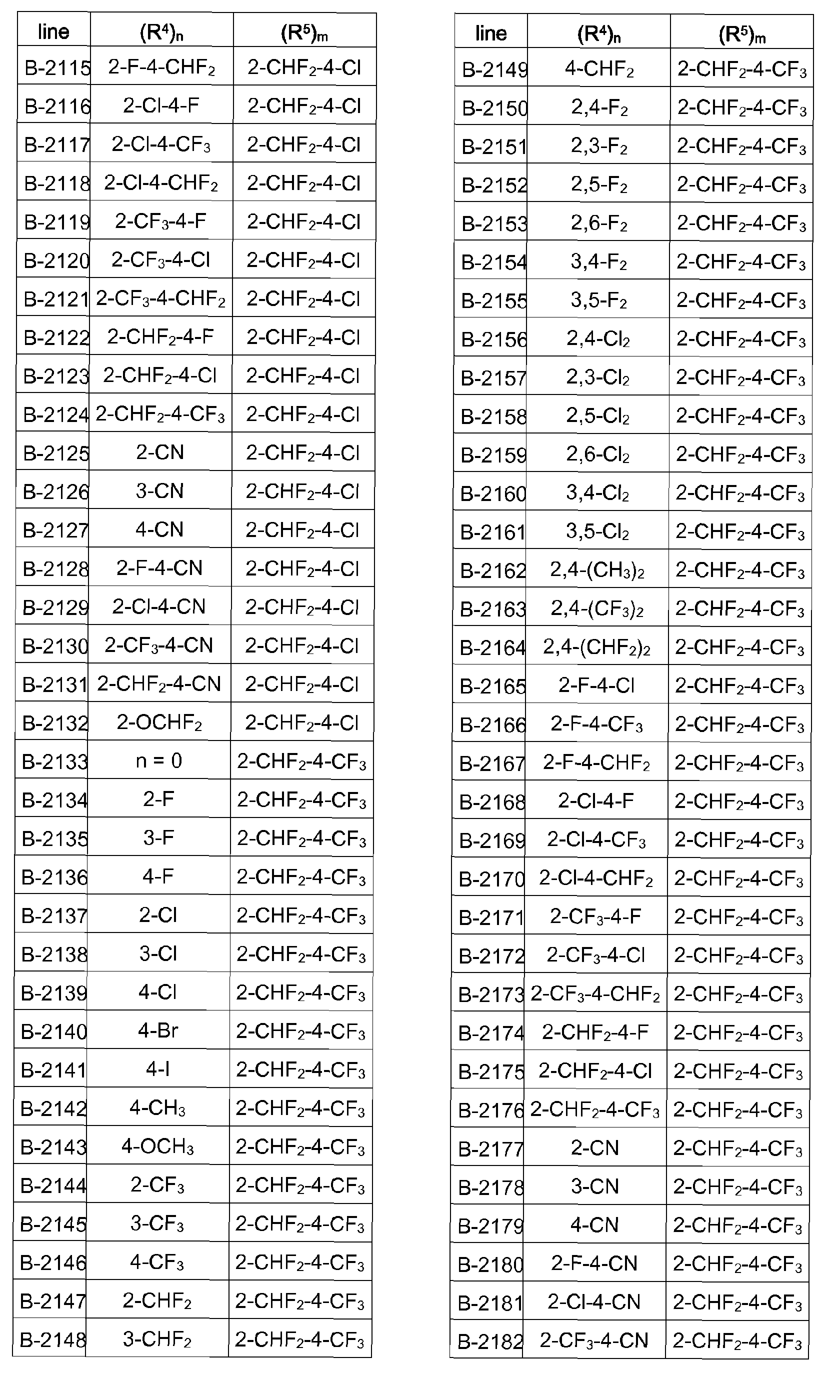

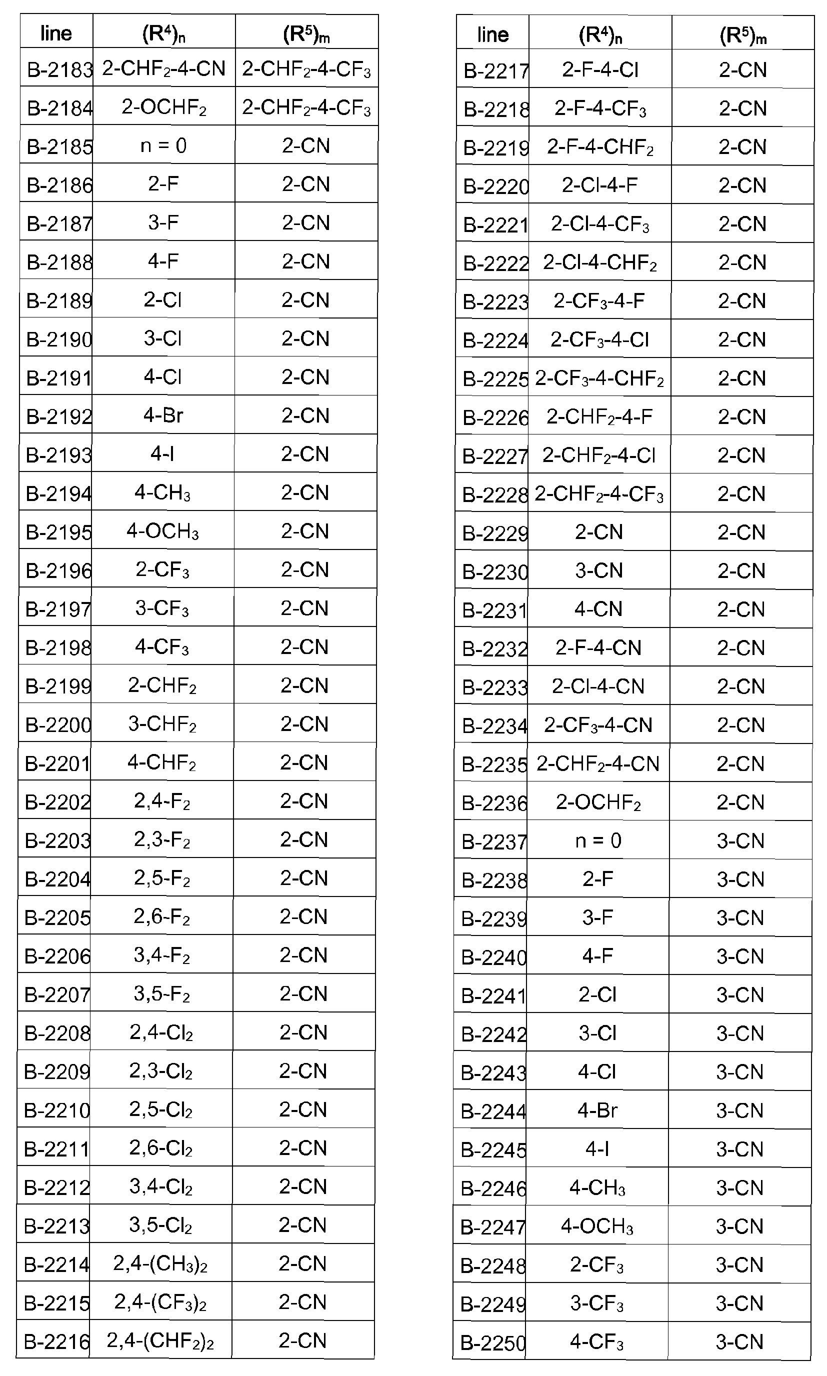

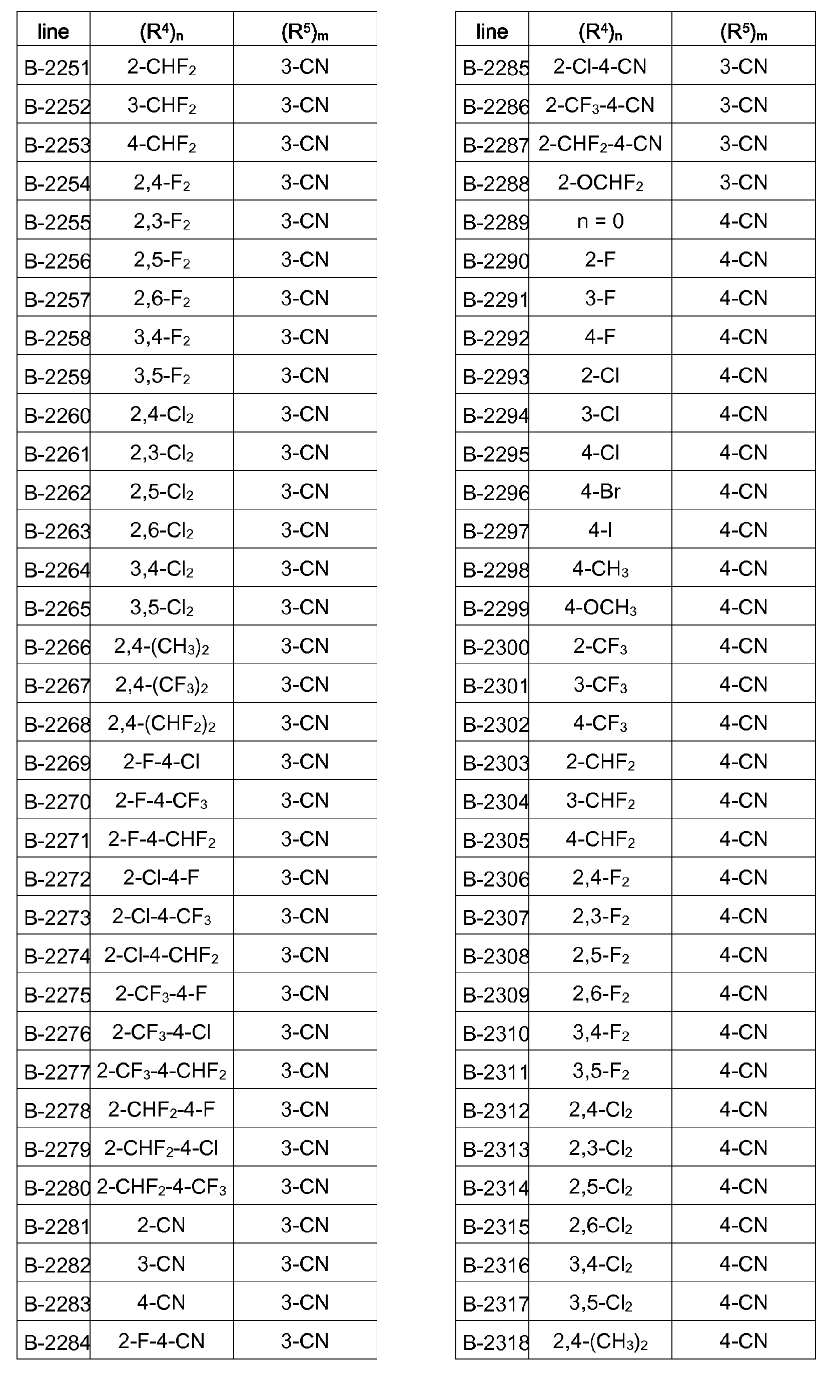

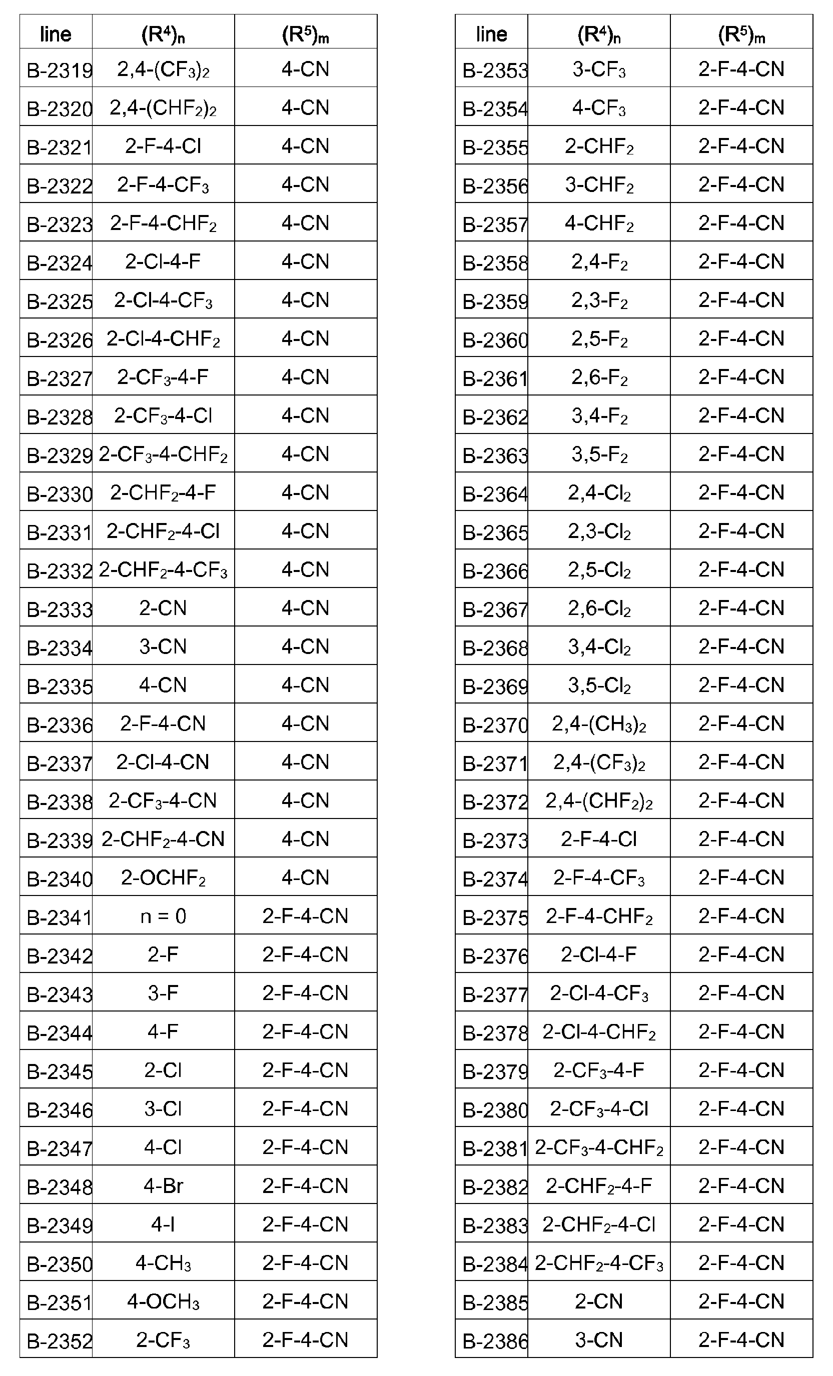

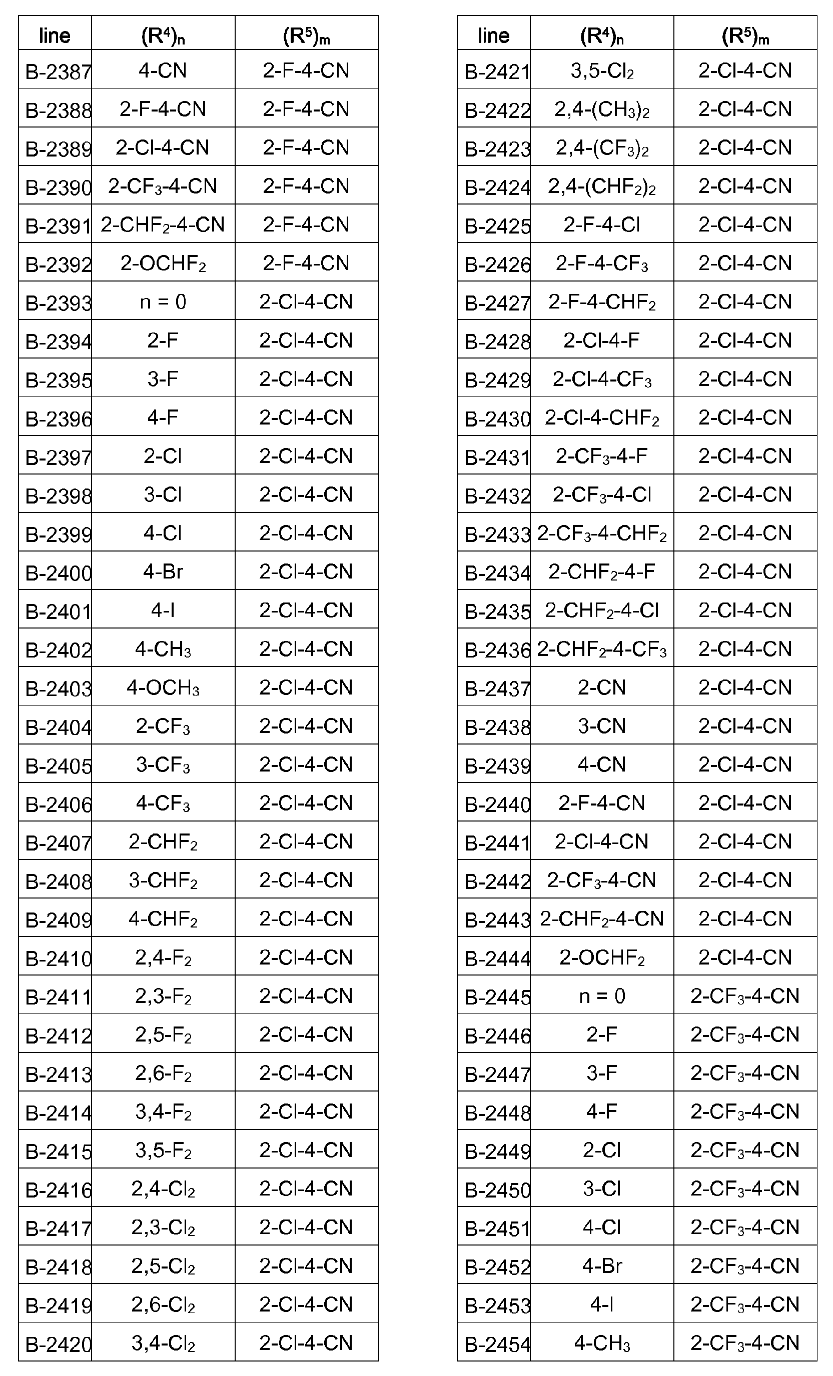

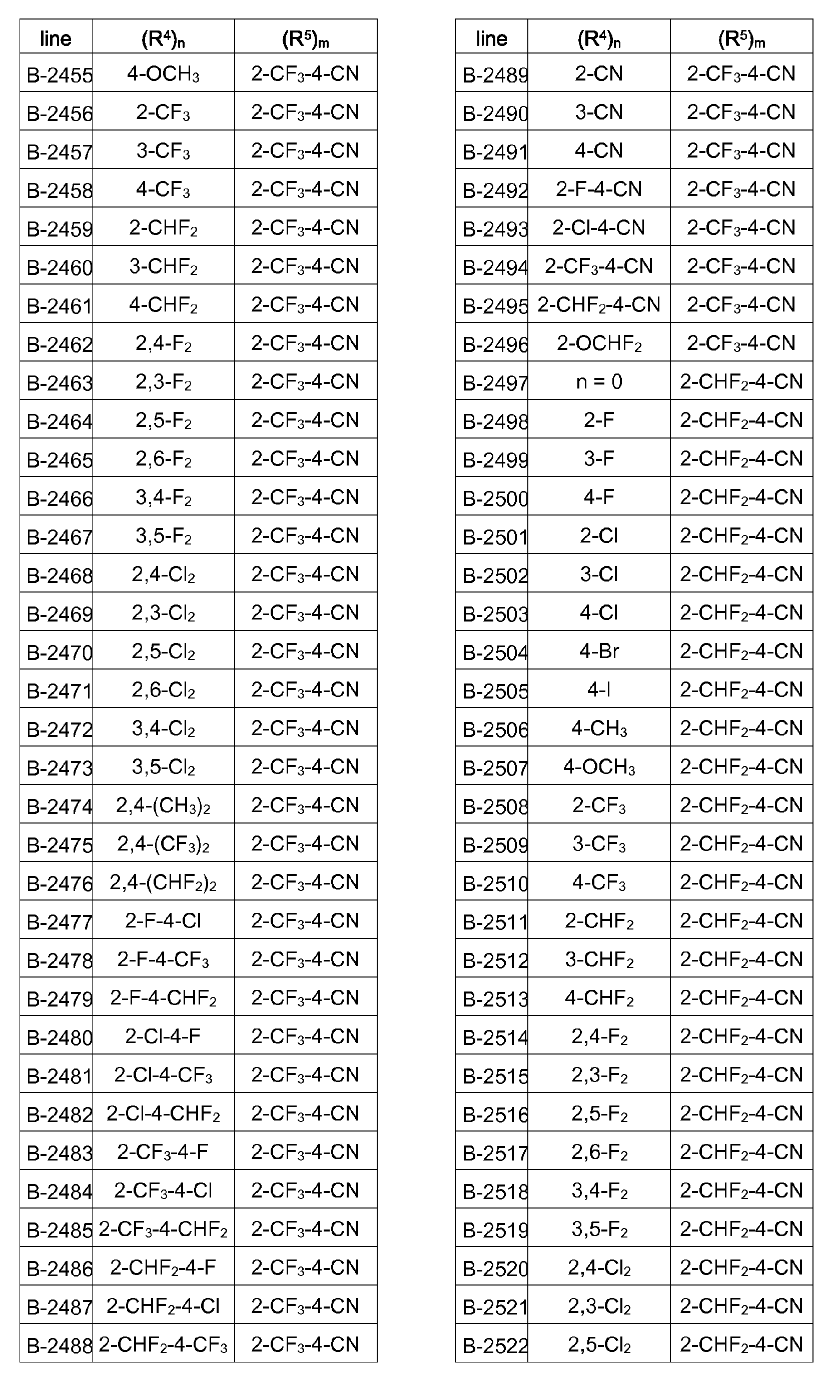

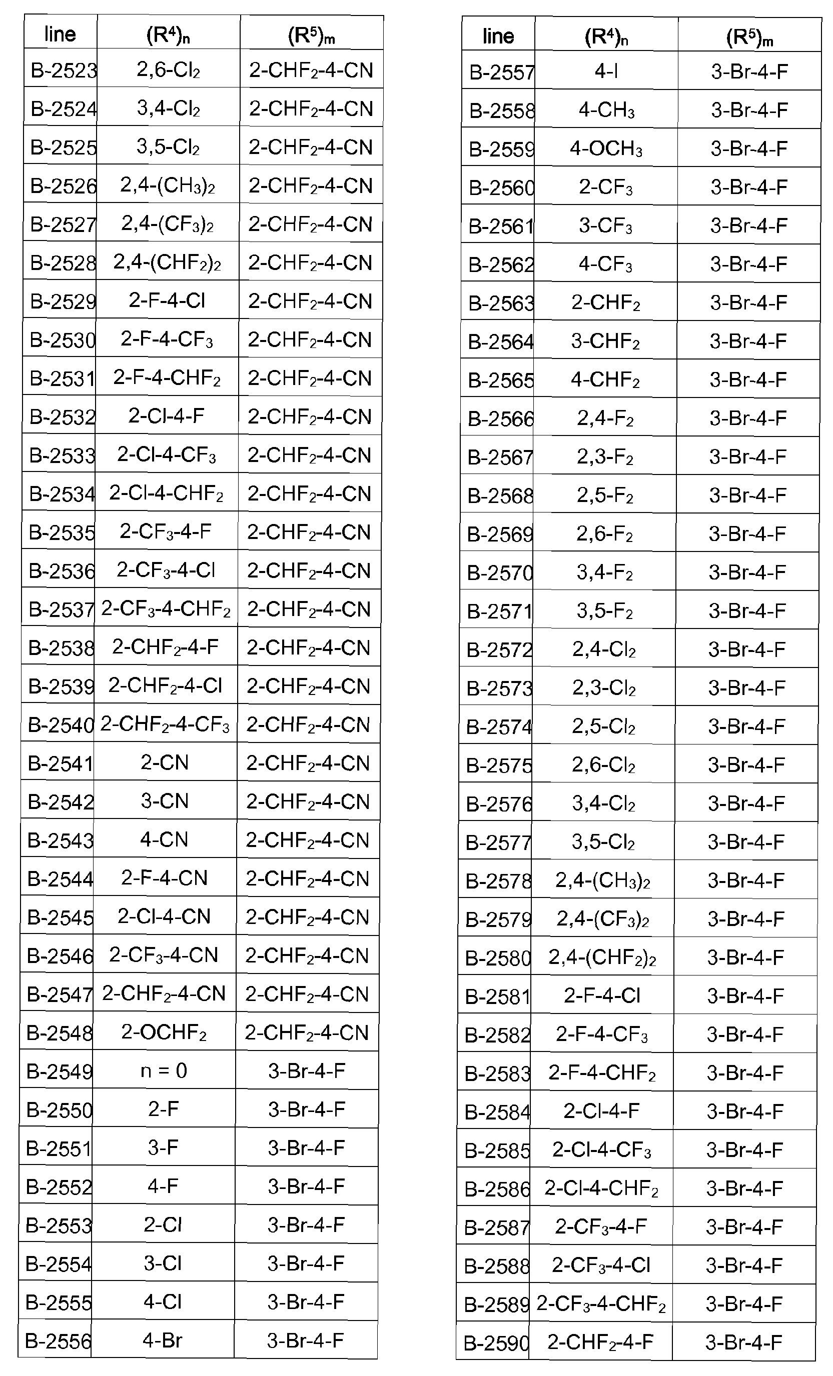

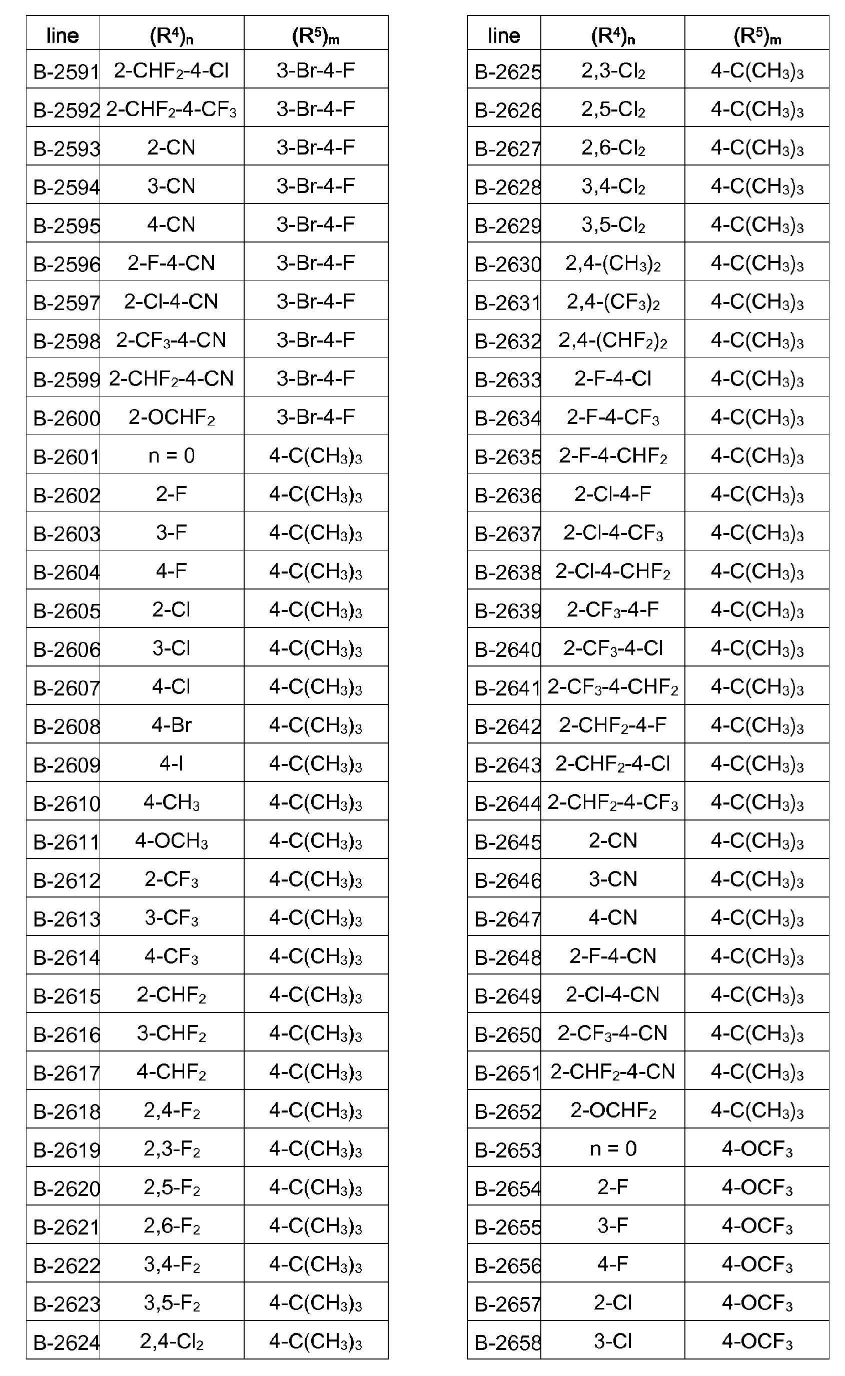

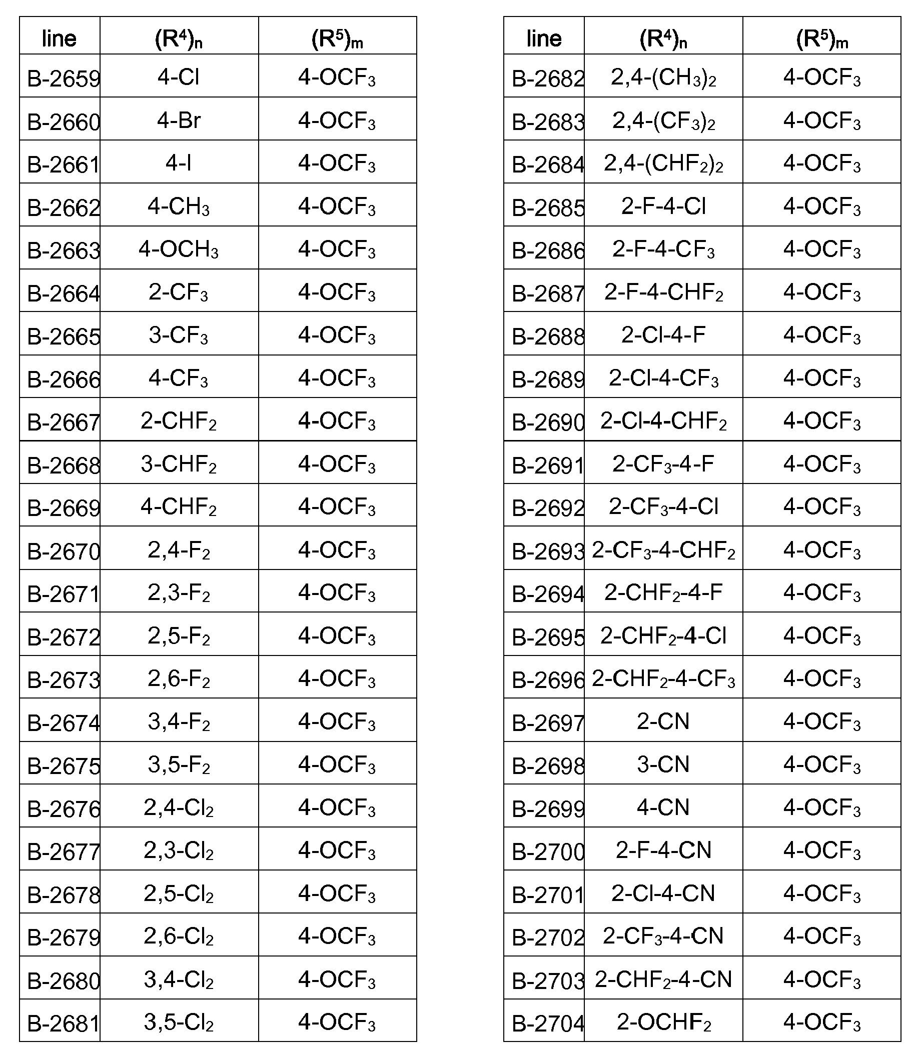

With respect to their use, particular preference is given to the compounds compiled in the Tables 1 to 96 below, wherein the meaning of A is N and the meaning of the combination of substituents R¹, R² and D are in each case selected from one line A-1 to A-96 in Table A and wherein the meaning of the combination of substituents R⁴, R⁵, n and m are selected from line B-1 to B-2704 as described in Table B.
Table 1: Compounds I, wherein A is N and wherein the combination of substituents R¹, R² and D corresponds to line A-1 in table A and wherein the combination of substituents (R⁴)ₙ and (R⁵)ₘ corresponds to one line B-1 to B-2704 in Table B (compounds I.a1.b1 to I.a1.b2704).
Table 2: Compounds I, wherein A is N and the combination of substituents R¹, R² and D corresponds to line A-2 in table A and wherein the combination of substituents (R⁴)ₙ and (R⁵)ₘ corresponds to one line B-1 to B-2704 in Table B (compounds I.a2.b1 to I.a2.b2704).
Table 3: Compounds I, wherein A is N and the combination of substituents R¹, R² and D corresponds to line A-3 in table A and wherein the combination of substituents (R⁴)ₙ and (R⁵)ₘ corresponds to one line B-1 to B-2704 in Table B (compounds I.a3.b1 to I.a3.b2704).
Table 4: Compounds I, wherein A is N and the combination of substituents R¹, R² and D corresponds to line A-4 in table A and wherein the combination of substituents (R⁴)ₙ and (R⁵)ₘ corresponds to one line B-1 to B-2704 in Table B (compounds I.a4.b1 to I.a4.b2704).
Table 5: Compounds I, wherein A is N and the combination of substituents R¹, R² and D corresponds to line A-5 in table A and wherein the combination of substituents (R⁴)ₙ and (R⁵)ₘ corresponds to one line B-1 to B-2704 in Table B (compounds I.a5.b1 to I.a5.b2704).
Table 6: Compounds I, wherein A is N and the combination of substituents R¹, R² and D corresponds to line A-6 in table A and wherein the combination of substituents (R⁴)ₙ and (R⁵)ₘ corresponds to one line B-1 to B-2704 in Table B (compounds I.a6.b1 to I.a6.b2704).
Table 7: Compounds I, wherein A is N and the combination of substituents R¹, R² and D corresponds to line A-7 in table A and wherein the combination of substituents (R⁴)ₙ and (R⁵)ₘ corresponds to one line B-1 to B-2704 in Table B (compounds I.a7.b1 to I.a7.b2704).
Table 8: Compounds I, wherein A is N and the combination of substituents R¹, R² and D corresponds to line A-8 in table A and wherein the combination of substituents (R⁴)ₙ and (R⁵)ₘ corresponds to one line B-1 to B-2704 in Table B (compounds I.a8.b1 to I.a8.b2704).
Table 9: Compounds I, wherein A is N and the combination of substituents R¹, R² and D corresponds to line A-9 in table A and wherein the combination of substituents (R⁴)ₙ and (R⁵)ₘ corresponds to one line B-1 to B-2704 in Table B (compounds I.a9.b1 to I.a9.b2704).
Table 10: Compounds I, wherein A is N and the combination of substituents R¹, R² and D corresponds to line A-1 0 in table A and wherein the combination of substituents (R⁴)ₙ and (R⁵)ₘ corresponds to one line B-1 to B-2704 in Table B (compounds I.a10.b1 to I.a10.b2704).
Table 11: Compounds I, wherein A is N and the combination of substituents R¹, R² and D corresponds to line A-11 in table A and wherein the combination of substituents (R⁴)ₙ and (R⁵)ₘ corresponds to one line B-1 to B-2704 in Table B (compounds I.a11.b1 to I.a11.b2704).
Table 12: Compounds I, wherein A is N and the combination of substituents R¹, R² and D corresponds to line A-12 in table A and wherein the combination of substituents (R⁴)ₙ and (R⁵)ₘ corresponds to one line B-1 to B-2704 in Table B (compounds I.a12.b1 to I.a12.b2704).
Table 13: Compounds I, wherein A is N and the combination of substituents R¹, R² and D corresponds to line A-13 in table A and wherein the combination of substituents (R⁴)ₙ and (R⁵)ₘ corresponds to one line B-1 to B-2704 in Table B (compounds I.a13.b1 to I.a13.b2704).
Table 14: Compounds I, wherein A is N and the combination of substituents R¹, R² and D corresponds to line A-14 in table A and wherein the combination of substituents (R⁴)ₙ and (R⁵)ₘ corresponds to one line B-1 to B-2704 in Table B (compounds I.a14.b1 to I.a14.b2704).
Table 15: Compounds I, wherein A is N and the combination of substituents R¹, R² and D corresponds to line A-15 in table A and wherein the combination of substituents (R⁴)ₙ and (R⁵)ₘ corresponds to one line B-1 to B-2704 in Table B (compounds I.a15.b1 to I.a15.b2704).
Table 16: Compounds I, wherein A is N and the combination of substituents R¹, R² and D corresponds to line A-16 in table A and wherein the combination of substituents (R⁴)ₙ and (R⁵)ₘ corresponds to one line B-1 to B-2704 in Table B (compounds I.a16.b1 to I.a16.b2704).
Table 17: Compounds I, wherein A is N and the combination of substituents R¹, R² and D corresponds to line A-17 in table A and wherein the combination of substituents (R⁴)ₙ and (R⁵)ₘ corresponds to one line B-1 to B-2704 in Table B (compounds I.a17.b1 to I.a17.b2704).
Table 18: Compounds I, wherein A is N and the combination of substituents R¹, R² and D corresponds to line A-18 in table A and wherein the combination of substituents (R⁴)ₙ and (R⁵)ₘ corresponds to one line B-1 to B-2704 in Table B (compounds I.a18.b1 to I.a18.b2704).
Table 19: Compounds I, wherein A is N and the combination of substituents R¹, R² and D corresponds to line A-19 in table A and wherein the combination of substituents (R⁴)ₙ and (R⁵)ₘ corresponds to one line B-1 to B-2704 in Table B (compounds I.a19.b1 to I.a19.b2704).
Table 20: Compounds I, wherein A is N and the combination of substituents R¹, R² and D corresponds to line A-20 in table A and wherein the combination of substituents (R⁴)ₙ and (R⁵)ₘ corresponds to one line B-1 to B-2704 in Table B (compounds I.a20.b1 to I.a20.b2704).
Table 21: Compounds I, wherein A is N and the combination of substituents R¹, R² and D corresponds to line A-21 in table A and wherein the combination of substituents (R⁴)ₙ and (R⁵)ₘ corresponds to one line B-1 to B-2704 in Table B (compounds I.a21.b1 to I.a21.b2704).
Table 22: Compounds I, wherein A is N and the combination of substituents R¹, R² and D corresponds to line A-22 in table A and wherein the combination of substituents (R⁴)ₙ and (R⁵)ₘ corresponds to one line B-1 to B-2704 in Table B (compounds I.a22.b1 to I.a22.b2704).
Table 23: Compounds I, wherein A is N and the combination of substituents R¹, R² and D corresponds to line A-23 in table A and wherein the combination of substituents (R⁴)ₙ and (R⁵)ₘ corresponds to one line B-1 to B-2704 in Table B (compounds I.a23.b1 to I.a23.b2704).
Table 24: Compounds I, wherein A is N and the combination of substituents R¹, R² and D corresponds to line A-24 in table A and wherein the combination of substituents (R⁴)ₙ and (R⁵)ₘ corresponds to one line B-1 to B-2704 in Table B (compounds I.a24.b1 to I.a24.b2704).
Table 25: Compounds I, wherein A is N and the combination of substituents R¹, R² and D corresponds to line A-25 in table A and wherein the combination of substituents (R⁴)ₙ and (R⁵)ₘ corresponds to one line B-1 to B-2704 in Table B (compounds I.a25.b1 to I.a25.b2704).
Table 26: Compounds I, wherein A is N and the combination of substituents R¹, R² and D corresponds to line A-26 in table A and wherein the combination of substituents (R⁴)ₙ and (R⁵)ₘ corresponds to one line B-1 to B-2704 in Table B (compounds I.a26.b1 to I.a26.b2704).
Table 27: Compounds I, wherein A is N and the combination of substituents R¹, R² and D corresponds to line A-27 in table A and wherein the combination of substituents (R⁴)ₙ and (R⁵)ₘ corresponds to one line B-1 to B-2704 in Table B (compounds I.a27.b1 to I.a27.b2704).
Table 28: Compounds I, wherein A is N and the combination of substituents R¹, R² and D corresponds to line A-28 in table A and wherein the combination of substituents (R⁴)ₙ and (R⁵)ₘ corresponds to one line B-1 to B-2704 in Table B (compounds I.a28.b1 to I.a28.b2704).
Table 29: Compounds I, wherein A is N and the combination of substituents R¹, R² and D corresponds to line A-29 in table A and wherein the combination of substituents (R⁴)ₙ and (R⁵)ₘ corresponds to one line B-1 to B-2704 in Table B (compounds I.a29.b1 to I.a29.b2704).
Table 30: Compounds I, wherein A is N and the combination of substituents R¹, R² and D corresponds to line A-30 in table A and wherein the combination of substituents (R⁴)ₙ and (R⁵)ₘ corresponds to one line B-1 to B-2704 in Table B (compounds I.a30.b1 to I.a30.b2704).
Table 31: Compounds I, wherein A is N and the combination of substituents R¹, R² and D corresponds to line A-31 in table A and wherein the combination of substituents (R⁴)ₙ and (R⁵)ₘ corresponds to one line B-1 to B-2704 in Table B (compounds I.a31.b1 to I.a31.b2704).
Table 32: Compounds I, wherein A is N and the combination of substituents R¹, R² and D corresponds to line A-32 in table A and wherein the combination of substituents (R⁴)ₙ and (R⁵)ₘ corresponds to one line B-1 to B-2704 in Table B (compounds I.a32.b1 to I.a32.b2704).
Table 33: Compounds I, wherein A is N and the combination of substituents R¹, R² and D corresponds to line A-33 in table A and wherein the combination of substituents (R⁴)ₙ and (R⁵)ₘ corresponds to one line B-1 to B-2704 in Table B (compounds I.a33.b1 to I.a33.b2704).
Table 34: Compounds I, wherein A is N and the combination of substituents R¹, R² and D corresponds to line A-34 in table A and wherein the combination of substituents (R⁴)ₙ and (R⁵)ₘ corresponds to one line B-1 to B-2704 in Table B (compounds I.a34.b1 to I.a34.b2704).
Table 35: Compounds I, wherein A is N and the combination of substituents R¹, R² and D corresponds to line A-35 in table A and wherein the combination of substituents (R⁴)ₙ and (R⁵)ₘ corresponds to one line B-1 to B-2704 in Table B (compounds I.a35.b1 to I.a35.b2704).
Table 36: Compounds I, wherein A is N and the combination of substituents R¹, R² and D corresponds to line A-36 in table A and wherein the combination of substituents (R⁴)ₙ and (R⁵)ₘ corresponds to one line B-1 to B-2704 in Table B (compounds I.a36.b1 to I.a36.b2704).
Table 37: Compounds I, wherein A is N and the combination of substituents R¹, R² and D corresponds to line A-37 in table A and wherein the combination of substituents (R⁴)ₙ and (R⁵)ₘ corresponds to one line B-1 to B-2704 in Table B (compounds I.a37.b1 to I.a37.b2704).
Table 38: Compounds I, wherein A is N and the combination of substituents R¹, R² and D corresponds to line A-38 in table A and wherein the combination of substituents (R⁴)ₙ and (R⁵)ₘ corresponds to one line B-1 to B-2704 in Table B (compounds I.a38.b1 to I.a38.b2704).
Table 39: Compounds I, wherein A is N and the combination of substituents R¹, R² and D corresponds to line A-39 in table A and wherein the combination of substituents (R⁴)ₙ and (R⁵)ₘ corresponds to one line B-1 to B-2704 in Table B (compounds I.a39.b1 to I.a39.b2704).
Table 40: Compounds I, wherein A is N and the combination of substituents R¹, R² and D corresponds to line A-40 in table A and wherein the combination of substituents (R⁴)ₙ and (R⁵)ₘ corresponds to one line B-1 to B-2704 in Table B (compounds I.a40.b1 to I.a40.b2704).
Table 41: Compounds I, wherein A is N and the combination of substituents R¹, R² and D corresponds to line A-41 in table A and wherein the combination of substituents (R⁴)ₙ and (R⁵)ₘ corresponds to one line B-1 to B-2704 in Table B (compounds I.a41.b1 to I.a41.b2704).
Table 42: Compounds I, wherein A is N and the combination of substituents R¹, R² and D corresponds to line A-42 in table A and wherein the combination of substituents (R⁴)ₙ and (R⁵)ₘ corresponds to one line B-1 to B-2704 in Table B (compounds I.a42.b1 to I.a42.b2704).
Table 43: Compounds I, wherein A is N and the combination of substituents R¹, R² and D corresponds to line A-43 in table A and wherein the combination of substituents (R⁴)ₙ and (R⁵)ₘ corresponds to one line B-1 to B-2704 in Table B (compounds I.a43.b1 to I.a43.b2704).
Table 44: Compounds I, wherein A is N and the combination of substituents R¹, R² and D corresponds to line A-44 in table A and wherein the combination of substituents (R⁴)ₙ and (R⁵)ₘ corresponds to one line B-1 to B-2704 in Table B (compounds I.a44.b1 to I.a44.b2704).
Table 45: Compounds I, wherein A is N and the combination of substituents R¹, R² and D corresponds to line A-45 in table A and wherein the combination of substituents (R⁴)ₙ and (R⁵)ₘ corresponds to one line B-1 to B-2704 in Table B (compounds I.a45.b1 to I.a45.b2704).
Table 46: Compounds I, wherein A is N and the combination of substituents R¹, R² and D corresponds to line A-46 in table A and wherein the combination of substituents (R⁴)ₙ and (R⁵)ₘ corresponds to one line B-1 to B-2704 in Table B (compounds I.a46.b1 to I.a46.b2704).
Table 47: Compounds I, wherein A is N and the combination of substituents R¹, R² and D corresponds to line A-47 in table A and wherein the combination of substituents (R⁴)ₙ and (R⁵)ₘ corresponds to one line B-1 to B-2704 in Table B (compounds I.a47.b1 to I.a47.b2704).
Table 48: Compounds I, wherein A is N and the combination of substituents R¹, R² and D corresponds to line A-48 in table A and wherein the combination of substituents (R⁴)ₙ and (R⁵)ₘ corresponds to one line B-1 to B-2704 in Table B (compounds I.a48.b1 to I.a48.b2704).
Table 49: Compounds I, wherein A is N and the combination of substituents R¹, R² and D corresponds to line A-49 in table A and wherein the combination of substituents (R⁴)ₙ and (R⁵)ₘ corresponds to one line B-1 to B-2704 in Table B (compounds I.a49.b1 to I.a49.b2704).
Table 50: Compounds I, wherein A is N and the combination of substituents R¹, R² and D corresponds to line A-50 in table A and wherein the combination of substituents (R⁴)ₙ and (R⁵)ₘ corresponds to one line B-1 to B-2704 in Table B (compounds I.a50.b1 to I.a50.b2704).
Table 51: Compounds I, wherein A is N and the combination of substituents R¹, R² and D corresponds to line A-51 in table A and wherein the combination of substituents (R⁴)ₙ and (R⁵)ₘ corresponds to one line B-1 to B-2704 in Table B (compounds I.a51.b1 to I.a51.b2704).
Table 52: Compounds I, wherein A is N and the combination of substituents R¹, R² and D corresponds to line A-52 in table A and wherein the combination of substituents (R⁴)ₙ and (R⁵)ₘ corresponds to one line B-1 to B-2704 in Table B (compounds I.a52.b1 to I.a52.b2704).
Table 53: Compounds I, wherein A is N and the combination of substituents R¹, R² and D corresponds to line A-53 in table A and wherein the combination of substituents (R⁴)ₙ and (R⁵)ₘ corresponds to one line B-1 to B-2704 in Table B (compounds I.a53.b1 to I.a53.b2704).
Table 54: Compounds I, wherein A is N and the combination of substituents R¹, R² and D corresponds to line A-54 in table A and wherein the combination of substituents (R⁴)ₙ and (R⁵)ₘ corresponds to one line B-1 to B-2704 in Table B (compounds I.a54.b1 to I.a54.b2704).
Table 55: Compounds I, wherein A is N and the combination of substituents R¹, R² and D corresponds to line A-55 in table A and wherein the combination of substituents (R⁴)ₙ and (R⁵)ₘ corresponds to one line B-1 to B-2704 in Table B (compounds I.a55.b1 to I.a55.b2704).
Table 56: Compounds I, wherein A is N and the combination of substituents R¹, R² and D corresponds to line A-56 in table A and wherein the combination of substituents (R⁴)ₙ and (R⁵)ₘ corresponds to one line B-1 to B-2704 in Table B (compounds I.a56.b1 to I.a56.b2704).
Table 57: Compounds I, wherein A is N and the combination of substituents R¹, R² and D corresponds to line A-57 in table A and wherein the combination of substituents (R⁴)ₙ and (R⁵)ₘ corresponds to one line B-1 to B-2704 in Table B (compounds I.a57.b1 to I.a57.b2704).
Table 58: Compounds I, wherein A is N and the combination of substituents R¹, R² and D corresponds to line A-58 in table A and wherein the combination of substituents (R⁴)ₙ and (R⁵)ₘ corresponds to one line B-1 to B-2704 in Table B (compounds I.a58.b1 to I.a58.b2704).
Table 59: Compounds I, wherein A is N and the combination of substituents R¹, R² and D corresponds to line A-59 in table A and wherein the combination of substituents (R⁴)ₙ and (R⁵)ₘ corresponds to one line B-1 to B-2704 in Table B (compounds I.a59.b1 to I.a59.b2704).
Table 60: Compounds I, wherein A is N and the combination of substituents R¹, R² and D corresponds to line A-60 in table A and wherein the combination of substituents (R⁴)ₙ and (R⁵)ₘ corresponds to one line B-1 to B-2704 in Table B (compounds I.a60.b1 to I.a60.b2704).
Table 61: Compounds I, wherein A is N and the combination of substituents R¹, R² and D corresponds to line A-61 in table A and wherein the combination of substituents (R⁴)ₙ and (R⁵)ₘ corresponds to one line B-1 to B-2704 in Table B (compounds I.a61.b1 to I.a61.b2704).
Table 62: Compounds I, wherein A is N and the combination of substituents R¹, R² and D corresponds to line A-62 in table A and wherein the combination of substituents (R⁴)ₙ and (R⁵)ₘ corresponds to one line B-1 to B-2704 in Table B (compounds I.a62.b1 to I.a62.b2704).
Table 63: Compounds I, wherein A is N and the combination of substituents R¹, R² and D corresponds to line A-63 in table A and wherein the combination of substituents (R⁴)ₙ and (R⁵)ₘ corresponds to one line B-1 to B-2704 in Table B (compounds I.a63.b1 to I.a63.b2704).
Table 64: Compounds I, wherein A is N and the combination of substituents R¹, R² and D corresponds to line A-64 in table A and wherein the combination of substituents (R⁴)ₙ and (R⁵)ₘ corresponds to one line B-1 to B-2704 in Table B (compounds I.a64.b1 to I.a64.b2704).
Table 65: Compounds I, wherein A is N and the combination of substituents R¹, R² and D corresponds to line A-65 in table A and wherein the combination of substituents (R⁴)ₙ and (R⁵)ₘ corresponds to one line B-1 to B-2704 in Table B (compounds I.a65.b1 to I.a65.b2704).
Table 66: Compounds I, wherein A is N and the combination of substituents R¹, R² and D corresponds to line A-66 in table A and wherein the combination of substituents (R⁴)ₙ and (R⁵)ₘ corresponds to one line B-1 to B-2704 in Table B (compounds I.a66.b1 to I.a66.b2704).
Table 67: Compounds I, wherein A is N and the combination of substituents R¹, R² and D corresponds to line A-67 in table A and wherein the combination of substituents (R⁴)ₙ and (R⁵)ₘ corresponds to one line B-1 to B-2704 in Table B (compounds I.a67.b1 to I.a67.b2704).
Table 68: Compounds I, wherein A is N and the combination of substituents R¹, R² and D corresponds to line A-68 in table A and wherein the combination of substituents (R⁴)ₙ and (R⁵)ₘ corresponds to one line B-1 to B-2704 in Table B (compounds I.a68.b1 to I.a68.b2704).
Table 69: Compounds I, wherein A is N and the combination of substituents R¹, R² and D corresponds to line A-69 in table A and wherein the combination of substituents (R⁴)ₙ and (R⁵)ₘ corresponds to one line B-1 to B-2704 in Table B (compounds I.a69.b1 to I.a69.b2704).
Table 70: Compounds I, wherein A is N and the combination of substituents R¹, R² and D corresponds to line A-70 in table A and wherein the combination of substituents (R⁴)ₙ and (R⁵)ₘ corresponds to one line B-1 to B-2704 in Table B (compounds I.a70.b1 to I.a70.b2704).
Table 71: Compounds I, wherein A is N and the combination of substituents R¹, R² and D corresponds to line A-71 in table A and wherein the combination of substituents (R⁴)ₙ and (R⁵)ₘ corresponds to one line B-1 to B-2704 in Table B (compounds I.a71.b1 to I.a71.b2704).
Table 72: Compounds I, wherein A is N and the combination of substituents R¹, R² and D corresponds to line A-72 in table A and wherein the combination of substituents (R⁴)ₙ and (R⁵)ₘ corresponds to one line B-1 to B-2704 in Table B (compounds I.a72.b1 to I.a72.b2704).
Table 73: Compounds I, wherein A is N and the combination of substituents R¹, R² and D corresponds to line A-73 in table A and wherein the combination of substituents (R⁴)ₙ and (R⁵)ₘ corresponds to one line B-1 to B-2704 in Table B (compounds I.a73.b1 to I.a73.b2704).
Table 74: Compounds I, wherein A is N and the combination of substituents R¹, R² and D corresponds to line A-74 in table A and wherein the combination of substituents (R⁴)ₙ and (R⁵)ₘ corresponds to one line B-1 to B-2704 in Table B (compounds I.a74.b1 to I.a74.b2704).
Table 75: Compounds I, wherein A is N and the combination of substituents R¹, R² and D corresponds to line A-75 in table A and wherein the combination of substituents (R⁴)ₙ and (R⁵)ₘ corresponds to one line B-1 to B-2704 in Table B (compounds I.a75.b1 to I.a75.b2704).
Table 76: Compounds I, wherein A is N and the combination of substituents R¹, R² and D corresponds to line A-76 in table A and wherein the combination of substituents (R⁴)ₙ and (R⁵)ₘ corresponds to one line B-1 to B-2704 in Table B (compounds I.a76.b1 to I.a76.b2704).
Table 77: Compounds I, wherein A is N and the combination of substituents R¹, R² and D corresponds to line A-77 in table A and wherein the combination of substituents (R⁴)ₙ and (R⁵)ₘ corresponds to one line B-1 to B-2704 in Table B (compounds I.a77.b1 to I.a77.b2704).
Table 78: Compounds I, wherein A is N and the combination of substituents R¹, R² and D corresponds to line A-78 in table A and wherein the combination of substituents (R⁴)ₙ and (R⁵)ₘ corresponds to one line B-1 to B-2704 in Table B (compounds I.a78.b1 to I.a78.b2704).
Table 79: Compounds I, wherein A is N and the combination of substituents R¹, R² and D corresponds to line A-79 in table A and wherein the combination of substituents (R⁴)ₙ and (R⁵)ₘ corresponds to one line B-1 to B-2704 in Table B (compounds I.a79.b1 to I.a79.b2704).
Table 80: Compounds I, wherein A is N and the combination of substituents R¹, R² and D corresponds to line A-80 in table A and wherein the combination of substituents (R⁴)ₙ and (R⁵)ₘ corresponds to one line B-1 to B-2704 in Table B (compounds I.a80.b1 to I.a80.b2704).
Table 81: Compounds I, wherein A is N and the combination of substituents R¹, R² and D corresponds to line A-81 in table A and wherein the combination of substituents (R⁴)ₙ and (R⁵)ₘ corresponds to one line B-1 to B-2704 in Table B (compounds I.a81.b1 to I.a81.b2704).
Table 82: Compounds I, wherein A is N and the combination of substituents R¹, R² and D corresponds to line A-82 in table A and wherein the combination of substituents (R⁴)ₙ and (R⁵)ₘ corresponds to one line B-1 to B-2704 in Table B (compounds I.a82.b1 to I.a82.b2704).
Table 83: Compounds I, wherein A is N and the combination of substituents R¹, R² and D corresponds to line A-83 in table A and wherein the combination of substituents (R⁴)ₙ and (R⁵)ₘ corresponds to one line B-1 to B-2704 in Table B (compounds I.a83.b1 to I.a83.b2704).
Table 84: Compounds I, wherein A is N and the combination of substituents R¹, R² and D corresponds to line A-84 in table A and wherein the combination of substituents (R⁴)ₙ and (R⁵)ₘ corresponds to one line B-1 to B-2704 in Table B (compounds I.a84.b1 to I.a84.b2704).
Table 85: Compounds I, wherein A is N and the combination of substituents R¹, R² and D corresponds to line A-85 in table A and wherein the combination of substituents (R⁴)ₙ and (R⁵)ₘ corresponds to one line B-1 to B-2704 in Table B (compounds I.a85.b1 to I.a85.b2704).
Table 86: Compounds I, wherein A is N and the combination of substituents R¹, R² and D corresponds to line A-86 in table A and wherein the combination of substituents (R⁴)ₙ and (R⁵)ₘ corresponds to one line B-1 to B-2704 in Table B (compounds I.a86.b1 to I.a86.b2704).
Table 87: Compounds I, wherein A is N and the combination of substituents R¹, R² and D corresponds to line A-87 in table A and wherein the combination of substituents (R⁴)ₙ and (R⁵)ₘ corresponds to one line B-1 to B-2704 in Table B (compounds I.a87.b1 to I.a87.b2704).
Table 88: Compounds I, wherein A is N and the combination of substituents R¹, R² and D corresponds to line A-88 in table A and wherein the combination of substituents (R⁴)ₙ and (R⁵)ₘ corresponds to one line B-1 to B-2704 in Table B (compounds I.a88.b1 to I.a88.b2704).
Table 89: Compounds I, wherein A is N and the combination of substituents R¹, R² and D corresponds to line A-89 in table A and wherein the combination of substituents (R⁴)ₙ and (R⁵)ₘ corresponds to one line B-1 to B-2704 in Table B (compounds I.a89.b1 to I.a89.b2704).
Table 90: Compounds I, wherein A is N and the combination of substituents R¹, R² and D corresponds to line A-90 in table A and wherein the combination of substituents (R⁴)ₙ and (R⁵)ₘ corresponds to one line B-1 to B-2704 in Table B (compounds I.a90.b1 to I.a90.b2704).
Table 91: Compounds I, wherein A is N and the combination of substituents R¹, R² and D corresponds to line A-91 in table A and wherein the combination of substituents (R⁴)ₙ and (R⁵)ₘ corresponds to one line B-1 to B-2704 in Table B (compounds I.a91.b1 to I.a91.b2704).
Table 92: Compounds I, wherein A is N and the combination of substituents R¹, R² and D corresponds to line A-92 in table A and wherein the combination of substituents (R⁴)ₙ and (R⁵)ₘ corresponds to one line B-1 to B-2704 in Table B (compounds I.a92.b1 to I.a92.b2704).
Table 93: Compounds I, wherein A is N and the combination of substituents R¹, R² and D corresponds to line A-93 in table A and wherein the combination of substituents (R⁴)ₙ and (R⁵)ₘ corresponds to one line B-1 to B-2704 in Table B (compounds I.a93.b1 to I.a93.b2704).
Table 94: Compounds I, wherein A is N and the combination of substituents R¹, R² and D corresponds to line A-94 in table A and wherein the combination of substituents (R⁴)ₙ and (R⁵)ₘ corresponds to one line B-1 to B-2704 in Table B (compounds I.a94.b1 to I.a94.b2704).
Table 95: Compounds I, wherein A is N and the combination of substituents R¹, R² and D corresponds to line A-95 in table A and wherein the combination of substituents (R⁴)ₙ and (R⁵)ₘ corresponds to one line B-1 to B-2704 in Table B (compounds I.a95.b1 to I.a95.b2704).
Table 96: Compounds I, wherein A is N and the combination of substituents R¹, R² and D corresponds to line A-96 in table A and wherein the combination of substituents (R⁴)ₙ and (R⁵)ₘ corresponds to one line B-1 to B-2704 in Table B (compounds I.a96.b1 to I.a96.b2704).

Accordingly a compound I having the following formula 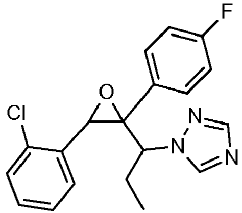 wherein A is N, D is hydrogen, R¹ is ethyl, R² is hydrogen, (R⁴)ₙ is 2-Cl and (R⁵)ₘ is 4-F, is denominated as compound I.a2.b90 and is individualized in table 2.

The compounds I and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.
Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxyl-phenylpyruvate dioxygenase (HPPD) inhibitors or phytoene desaturase (PDS) inhibit-tors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield® summer rape (Canola, BASF SE, Germany) being tolerant to imidazolinones, e. g. imazamox, or ExpressSun® sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady® (glyphosate-tolerant, Monsanto, U.S.A.), Cultivance® (imidazolinone tolerant, BASF SE, Germany) and LibertyLink® (glufosinate-tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. Photorhabdus spp. or Xenorhabdus spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g. Agrisure® CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme). Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against Phytophthora infestans derived from the mexican wild potato Solanum bulbocastanum) or T4-lysozym (e. g. potato cultivars capable of synthesizing these proteins with increased resistance against bacteria such as Erwinia amylvora). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.
Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera® rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora® potato, BASF SE, Germany).

The compounds I and compositions thereof, respectively, are particularly suitable for controlling the following plant diseases:
Albugo spp. (white rust) on ornamentals, vegetables (e. g. A. candida) and sunflowers (e. g. A. tragopogonis); Alternaria spp. (Alternaria leaf spot) on vegetables, rape (A. brassicola or brassicae), sugar beets (A. tenuis), fruits, rice, soybeans, potatoes (e. g. A. solani or A. alternata), tomatoes (e. g. A. solani or A. alternata) and wheat; Aphano-myces spp. on sugar beets and vegetables; Ascochyta spp. on cereals and vegetables, e. g. A. tritici (anthracnose) on wheat and A. hordei on barley; Bipolaris and Drechslera spp. (teleomorph: Cochliobolus spp.), e. g. Southern leaf blight (D. maydis) or Northern leaf blight (B. zeicola) on corn, e. g. spot blotch (B. sorokiniana) on cereals and e.g. B. oryzae on rice and turfs; Blumeria (formerly Erysiphe) graminis (powdery mildew) on cereals (e. g. on wheat or barley); Botrytis cinerea (teleomorph: Botryotinia fuckeliana: grey mold) on fruits and berries (e. g. strawberries), vegetables (e. g. lettuce, carrots, celery and cabbages), rape, flowers, vines, forestry plants and wheat; Bremia lactucae (downy mildew) on lettuce; Ceratocystis (syn. Ophiostoma) spp. (rot or wilt) on broadleaved trees and evergreens, e. g. C. ulmi (Dutch elm disease) on elms; Cercospora spp. (Cercospora leaf spots) on corn (e.g. Gray leaf spot: C. zeae-maydis), rice, sugar beets (e. g. C. beticola), sugar cane, vegetables, coffee, soybeans (e. g. C. sojina or C. kikuchii) and rice; Cladosporium spp. on tomatoes (e. g. C. fulvum: leaf mold) and cereals, e. g. C. herbarum (black ear) on wheat; Claviceps purpurea (ergot) on cereals; Cochliobolus (anamorph: Helminthosporium of Bipolaris) spp. (leaf spots) on corn (C. carbonum), cereals (e. g. C. sativus, anamorph: B. sorokiniana) and rice (e. g. C. miyabeanus, anamorph: H. oryzae); Colletotrichum (teleomorph: Glomerella) spp. (anthracnose) on cotton (e. g. C. gossypii), corn (e. g. C. graminicola: Anthracnose stalk rot), soft fruits, potatoes (e. g. C. coccodes: black dot), beans (e. g. C. lindemuthianum) and soybeans (e. g. C. truncatum or C. gloeosporioides); Corticium spp., e. g. C. sasakii (sheath blight) on rice; Corynespora cassiicola (leaf spots) on soybeans and ornamentals; Cycloconium spp., e. g. C. oleaginum on olive trees; Cylindrocarpon spp. (e. g. fruit tree canker or young vine decline, teleomorph: Nectria or Neonectria spp.) on fruit trees, vines (e. g. C. liriodendri, teleomorph: Neonectria liriodendri: Black Foot Disease) and ornamentals; Dematophora (teleomorph: Rosellinia) necatrix (root and stem rot) on soybeans; Diaporthe spp., e. g. D. phaseolorum (damping off) on soybeans; Drechslera (syn. Helminthosporium, teleomorph: Pyrenophora) spp. on corn, cereals, such as barley (e. g. D. teres, net blotch) and wheat (e. g. D. tritici-repentis: tan spot), rice and turf; Esca (dieback, apoplexy) on vines, caused by Formitiporia (syn. Phellinus) punctata, F. mediterranea, Phaeomoniella chlamydospora (earlier Phaeoacremonium chlamydosporum), Phaeoacremonium aleophilum and/or Botryosphaeria obtusa; Elsinoe spp. on pome fruits (E. pyri), soft fruits (E. veneta: anthracnose) and vines (E. ampelina: anthracnose); Entyloma oryzae (leaf smut) on rice; Epicoccum spp. (black mold) on wheat; Erysiphe spp. (powdery mildew) on sugar beets (E. betae), vegetables (e. g. E. pisi), such as cucurbits (e. g. E. cichoracearum), cabbages, rape (e. g. E. cruciferarum); Eutypa lata (Eutypa canker or dieback, anamorph: Cytosporina lata, syn. Libertella blepharis) on fruit trees, vines and ornamental woods; Exserohilum (syn. Helminthosporium) spp. on corn (e. g. E. turcicum); Fusarium (teleomorph: Gibberella) spp. (wilt, root or stem rot) on various plants, such as F. graminearum or F. culmorum (root rot, scab or head blight) on cereals (e. g. wheat or barley), F. oxysporum on tomatoes, F. solani on soybeans and F. verticillioides on corn; Gaeumannomyces graminis (take-all) on cereals (e. g. wheat or barley) and corn; Gibberella spp. on cereals (e. g. G. zeae) and rice (e. g. G. fujikuroi: Bakanae disease); Glomerella cingulata on vines, pome fruits and other plants and G. gossypii on cotton; Grain-staining complex on rice; Guignardia bidwellii (black rot) on vines; Gymnosporangium spp. on rosaceous plants and junipers, e. g. G. sabinae (rust) on pears; Helminthosporium spp. (syn. Drechslera, teleomorph: Cochliobolus) on corn, cereals and rice; Hemileia spp., e. g. H. vastatrix (coffee leaf rust) on coffee; Isariopsis clavispora (syn. Cladosporium vitis) on vines; Macrophomina phaseolina (syn. phaseoli) (root and stem rot) on soybeans and cotton; Microdochium (syn. Fusarium) nivale (pink snow mold) on cereals (e. g. wheat or barley); Microsphaera diffusa (powdery mildew) on soybeans; Monilinia spp., e. g. M. laxa, M. fructicola and M. fructigena (bloom and twig blight, brown rot) on stone fruits and other rosaceous plants; Mycosphaerella spp. on cereals, bananas, soft fruits and ground nuts, such as e. g. M. graminicola (anamorph: Septoria tritici, Septoria blotch) on wheat or M. fijiensis (black Sigatoka disease) on bananas; Peronospora spp. (downy mildew) on cabbage (e. g. P. brassicae), rape (e. g. P. parasitica), onions (e. g. P. destructor), tobacco (P. tabacina) and soybeans (e. g. P. manshurica); Phakopsora pachyrhizi and P. meibomiae (soybean rust) on soybeans; Phialophora spp. e. g. on vines (e. g. P. tracheiphila and P. tetraspora) and soybeans (e. g. P. gregata: stem rot); Phoma lingam (root and stem rot) on rape and cabbage and P. betae (root rot, leaf spot and damping-off) on sugar beets; Phomopsis spp. on sunflowers, vines (e. g. P. viticola: can and leaf spot) and soybeans (e. g. stem rot: P. phaseoli, teleomorph: Diaporthe phaseolorum); Physoderma maydis (brown spots) on corn; Phytophthora spp. (wilt, root, leaf, fruit and stem root) on various plants, such as paprika and cucurbits (e. g. P. capsici), soybeans (e. g. P. megasperma, syn. P. sojae), potatoes and tomatoes (e. g. P. infestans: late blight) and broad-leaved trees (e. g. P. ramorum: sudden oak death); Plasmodiophora brassicae (club root) on cabbage, rape, radish and other plants; Plasmopara spp., e. g. P. viticola (grapevine downy mildew) on vines and P. halstedii on sunflowers; Podosphaera spp. (powdery mildew) on rosaceous plants, hop, pome and soft fruits, e. g. P. leucotricha on apples; Polymyxa spp., e. g. on cereals, such as barley and wheat (P. graminis) and sugar beets (P. betae) and thereby transmitted viral diseases; Pseudocercosporella herpotrichoides (eyespot, teleomorph: Tapesia yallundae) on cereals, e. g. wheat or barley; Pseudoperonospora (downy mildew) on various plants, e. g. P. cubensis on cucurbits or P. humili on hop; Pseudopezicula tracheiphila (red fire disease or ,rotbrenner', anamorph: Phialophora) on vines; Puccinia spp. (rusts) on various plants, e. g. P. triticina (brown or leaf rust), P. striiformis (stripe or yellow rust), P. hordei (dwarf rust), P. graminis (stem or black rust) or P. recondita (brown or leaf rust) on cereals, such as e. g. wheat, barley or rye, P. kuehnii (orange rust) on sugar cane and P. asparagi on asparagus; Pyrenophora (anamorph: Drechslera) tritici-repentis (tan spot) on wheat or P. teres (net blotch) on barley; Pyricularia spp., e. g. P. oryzae (teleomorph: Magnaporthe grisea, rice blast) on rice and P. grisea on turf and cereals; Pythium spp. (damping-off) on turf, rice, corn, wheat, cotton, rape, sunflowers, soybeans, sugar beets, vegetables and various other plants (e. g. P. ultimum or P. aphanidermatum); Ramularia spp., e. g. R. collo-cygni (Ramularia leaf spots, Physiological leaf spots) on barley and R. beticola on sugar beets; Rhizoctonia spp. on cotton, rice, potatoes, turf, corn, rape, potatoes, sugar beets, vegetables and various other plants, e. g. R. solani (root and stem rot) on soybeans, R. solani (sheath blight) on rice or R. cerealis (Rhizoctonia spring blight) on wheat or barley; Rhizopus stolonifer (black mold, soft rot) on strawberries, carrots, cabbage, vines and tomatoes; Rhynchosporium secalis (scald) on barley, rye and triticale; Sarocladium oryzae and S. attenuatum (sheath rot) on rice; Sclerotinia spp. (stem rot or white mold) on vegetables and field crops, such as rape, sunflowers (e. g. S. sclerotiorum) and soybeans (e. g. S. rolfsii or S. sclerotiorum); Septoria spp. on various plants, e. g. S. glycines (brown spot) on soybeans, S. tritici (Septoria blotch) on wheat and S. (syn. Stagonospora) nodorum (Stagonospora blotch) on cereals; Uncinula (syn. Erysiphe) necator (powdery mildew, anamorph: Oidium tuckeri) on vines; Setospaeria spp. (leaf blight) on corn (e. g. S. turcicum, syn. Helminthosporium turcicum) and turf; Sphacelotheca spp. (smut) on corn, (e. g. S. reiliana: head smut), sorghum und sugar cane; Sphaerotheca fuliginea (powdery mildew) on cucurbits; Spongospora subterranea (powdery scab) on potatoes and thereby transmitted viral diseases; Stagonospora spp. on cereals, e. g. S. nodorum (Stagonospora blotch, teleomorph: Leptosphaeria [syn. Phaeosphaeria] nodorum) on wheat; Synchytrium endobioticum on potatoes (potato wart disease); Taphrina spp., e. g. T. deformans (leaf curl disease) on peaches and T. pruni (plum pocket) on plums; Thielaviopsis spp. (black root rot) on tobacco, pome fruits, vegetables, soybeans and cotton, e. g. T. basicola (syn. Chalara elegans); Tilletia spp. (common bunt or stinking smut) on cereals, such as e. g. T. tritici (syn. T. caries, wheat bunt) and T. controversa (dwarf bunt) on wheat; Typhula incarnata (grey snow mold) on barley or wheat; Urocystis spp., e. g. U. occulta (stem smut) on rye; Uromyces spp. (rust) on vegetables, such as beans (e. g. U. appendiculatus, syn. U. phaseoli) and sugar beets (e. g. U. betae); Ustilago spp. (loose smut) on cereals (e. g. U. nuda and U. avaenae), corn (e. g. U. maydis: corn smut) and sugar cane; Venturia spp. (scab) on apples (e. g. V. inaequalis) and pears; and Verticillium spp. (wilt) on various plants, such as fruits and ornamentals, vines, soft fruits, vegetables and field crops, e. g. V. dahliae on strawberries, rape, potatoes and tomatoes.

The compounds I and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as Ophiostoma spp., Ceratocystis spp., Aureobasidium pullulans, Sclerophoma spp., Chaetomium spp., Humicola spp., Petriella spp., Trichurus spp.; Basidiomycetes such as Coniophora spp., Coriolus spp., Gloeophyllum spp., Lentinus spp., Pleurotus spp., Poria spp., Serpula spp. and Tyromyces spp., Deuteromycetes such as Aspergillus spp., Cladosporium spp., Penicillium spp., Trichorma spp., Alternaria spp., Paecilomyces spp. and Zygomycetes such as Mucor spp., and in addition in the protection of stored products and harvest the following yeast fungi are worthy of note: Candida spp. and Saccharomyces cerevisae.

The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds I and compositions thereof, resepectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.
Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.
Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).
Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl-and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.
Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.
Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of poly-ethylene oxide and polypropylene oxide, or of the A-B-C type comprising alkanol, poly-ethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines. Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.
Suitable thickeners are polysaccharides (e.g. xanthan gum, carboxymethylcellulose), anorganic clays (organically modified or unmodified), polycarboxylates, and silicates. Suitable bactericides are bronopol and isothiazolinone derivatives such as alkylisothiazolinones and benzisothiazolinones.
Suitable anti-freezing agents are ethylene glycol, propylene glycol, urea and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).
Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound I and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of a compound I and 5-10 wt% emulsifiers (e.g. calcium dodecylben-zenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of a compound I and 1-10 wt% emulsifiers (e.g. calcium dodecylbenzene-sulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e.g. sodium lignosulfonate and alcohol ethoxylate), 0.1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e.g. sodium lignosulfonate), 1-3 wt% wetting agents (e.g. alcohol ethoxy-late) and solid carrier (e.g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e.g. sodium lignosulfonate), 1-5 wt% thickener (e.g. carboxy-methylcellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e.g. finely divided kaolin) ad 100 wt%.
xii) Granules (GR, FG)
   0.5-30 wt% of a compound I is ground finely and associated with solid carrier (e.g. silicate) ad 100 wt%. Granulation is achieved by extrusion, spray-drying or fluidized bed.
xiii) Ultra-low volume liquids (UL)
   1-50 wt% of a compound I are dissolved in organic solvent (e.g. aromatic hydrocarbon) ad 100 wt%.
   The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).
Solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.
In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required. When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material. Various types of oils, wetters, adjuvants, fertilizer, or micronutrients, and further pesticides (e.g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as a virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term pesticides includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology to increase plant growth, biomass, yield or any other quality parameter of the harvestable goods of acrop plant.
Biopesticides are typically created by growing and concentrating naturally occurring organisms and/or their metabolites including bacteria and other microbes, fungi, viruses, nematodes, proteins, etc. They are often considered to be important components of integrated pest management (IPM) programmes.
Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classed as microbial pesticides, even though they are multi-cellular.
(2) Biochemical pesticides are naturally occurring substances that control pests or provide other crop protection uses as defined below, but are relatively non-toxic to mammals.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.
According to one embodiment, individual components of the composition according to the invention such as parts of a kit or parts of a binary or ternary mixture may be mixed by the user himself in a spray tank or any other kind of vessel used for applications (e.g seed treater drums, seed pelleting machinery, knapsack sprayer) and further auxiliaries may be added, if appropriate.

When living microorganisms, such as pesticides from groups L1), L3) and L5), form part of such kit, it must be taken care that choice and amounts of the components (e.g. chemcial pesticidal agents) and of the further auxiliaries should not influence the viability of the microbial pesticides in the composition mixed by the user. Especially for bactericides and solvents, compatibility with the respective microbial pesticide has to be taken into account.
Consequently, one embodiment of the invention is a kit for preparing a usable pesticidal composition, the kit compring a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.
The following list of pesticides II (e.g. pesticidally-active substances and biopesticides), in conjunction with which the compounds I can be used, is intended to illustrate the possible combinations but does not limit them:
A) Respiration inhibitors
   - Inhibitors of complex III at Qₒ site (e.g. strobilurins): azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17) and 2-(2-(3-(2,6-dichlorophenyl)-1-methyl-allylidene-aminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21);
   - inhibitors of complex III at Qi site: cyazofamid (A.2.1), amisulbrom (A.2.2), [(3S,6S,7R,8R)-8-benzyl-3-[(3-acetoxy-4-methoxy-pyridine-2-carbonyl)amino]-6-methyl-4,9-dioxo-1,5-dioxonan-7-yl]2-methylpropanoate (A.2.3), [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate (A.2.4), [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate (A.2.5), [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methyl-propanoate (A.2.6); (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate (A.2.7);
   - inhibitors of complex II (e. g. carboxamides): benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.14), penthiopyrad (A.3.15), sedaxane (A.3.16), tecloftalam (A.3.17), thifluzamide (A.3.18), N-(4'-trifluoromethylthiobiphenyl-2-yl)-3-difluoromethyl-1-methyl-1 H-pyrazole-4-carboxamide (A.3.19), N-(2-(1,3,3-trimethyl-butyl)-phenyl)-1,3-dimethyl-5-fluoro-1 H-pyrazole-4-carboxamide (A.3.20), 3-(difluoromethyl)-1-methyl-N-(1,1,3-trimethylindan-4-yl)pyrazole-4-carboxamide (A.3.21), 3-(trifluoromethyl)-1-methyl-N-(1,1,3-trimethylindan-4-yl)pyrazole-4-carboxamide (A.3.22), 1,3-dimethyl-N-(1,1,3-trimethylindan-4-yl)pyrazole-4-carboxamide (A.3.23), 3-(trifluoromethyl)-1,5-dimethyl-N-(1,1,3-trimethylindan-4-yl)pyrazole-4-carboxamide (A.3.24), 1,3,5-trimethyl-N-(1,1,3-trimethylindan-4-yl)pyrazole-4-carboxamide (A.3.25), N-(7-fluoro-1,1,3-trimethyl-indan-4-yl)-1,3-dimethyl-pyrazole-4-carboxamide (A.3.26), N-[2-(2,4-d ichlorophenyl)-2-methoxy-1-methyl-ethyl]-3-(d ifl uoromethyl)-1-methyl-pyrazole-4-carboxamide (A.3.27);
   - other respiration inhibitors (e.g. complex I, uncouplers): diflumetorim (A.4.1), (5,8-difluoroquinazolin-4-yl)-{2-[2-fluoro-4-(4-trifluoromethylpyridin-2-yloxy)-phenyl]-ethyl}-amine (A.4.2); nitrophenyl derivates: binapacryl (A.4.3), dinobuton (A.4.4), dinocap (A.4.5), fluazinam (A.4.6); ferimzone (A.4.7); organometal compounds: fentin salts, such as fentin-acetate (A.4.8), fentin chloride (A.4.9) or fentin hydroxide (A.4.10); ametoctradin (A.4.11); and silthiofam (A.4.12);
B) Sterol biosynthesis inhibitors (SBI fungicides)
   - C14 demethylase inhibitors (DMI fungicides): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[rel-(2S;3R)-3-(2-chlorophenyl)-2-(2,4-difluorophenyl)-oxiranylmethyl]-5-thiocyanato-1H-[1,2,4]triazolo (B.1.31), 2-[rel-(2S;3R)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chlorophenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), 2-[4-(4-chlorophenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1-(1,2,4-triazol-1-yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41); imidazoles: imazalil (B.1.42), pefurazoate (B.1.43), prochloraz (B.1.44), triflumizol (B.1.45); pyrimidines, pyridines and piperazines: fenarimol (B.1.46), nuarimol (B.1.47), pyrifenox (B.1.48), triforine (B.1.49), [3-(4-chloro-2-fluoro-phenyl)-5-(2,4-difluoro-phenyl)isoxazol-4-yl]-(3-pyridyl)methanol (B.1.50);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (mefenoxam, C.1.5), ofurace (C.1.6), oxadixyl (C.1.7);
   - others: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7);
D) Inhibitors of cell division and cytoskeleton
   - tubulin inhibitors, such as benzimidazoles, thiophanates: benomyl (D1.1), carbendazim (D1.2), fuberidazole (D1.3), thiabendazole (D1.4), thiophanate-methyl (D1.5); triazolopyrimidines: 5-chloro-7-(4-methylpiperidin-1-yl)-6-(2,4,6-trifluorophenyl)-[1,2,4]triazolo[1,5-a]pyrimidine (D1.6);
   - other cell division inhibitors: diethofencarb (D2.1), ethaboxam (D2.2), pencycuron (D2.3), fluopicolide (D2.4), zoxamide (D2.5), metrafenone (D2.6), pyriofenone (D2.7);
E) Inhibitors of amino acid and protein synthesis
   - methionine synthesis inhibitors (anilino-pyrimidines): cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6), polyoxine (E.2.7), validamycin A (E.2.8);
F) Signal transduction inhibitors
   - MAP / histidine kinase inhibitors: fluoroimid (F.1.1), iprodione (F.1.2), procymidone (F.1.3), vinclozolin (F.1.4), fenpiclonil (F.1.5), fludioxonil (F.1.6);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7) and N-(1-(1-(4-cyano-phenyl)-ethanesulfonyl)-but-2-yl) carbamic acid-(4-fluorophenyl) ester (G.3.8);
   - compounds affecting cell membrane permeability and fatty acides: propamocarb (G.4.1);
   - fatty acid amide hydrolase inhibitors: oxathiapiprolin (G.5.1), 2-{3-[2-(1-{[3,5-bis(difluoromethyl-1H-pyrazol-1-yl]acetyl}piperidin-4-yl)-1,3-thiazol-4-yl]-4,5-dihydro-1,2-oxazol-5-yl}phenyl methanesulfonate (G.5.2), 2-{3-[2-(1-{[3,5-bis(difluoromethyl)-1H-pyrazol-1-yl]acetyl}piperidin-4-yl)1,3-thiazol-4-yl]-4,5-dihydro-1,2-oxazol-5-yl}-3-chlorophenyl methanesulfonate (G.5.3);
H) Inhibitors with Multi Site Action
   - inorganic active substances: Bordeaux mixture (H.1.1), copper acetate (H.1.2), copper hydroxide (H.1.3), copper oxychloride (H.1.4), basic copper sulfate (H.1.5), sulfur (H.1.6);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds (e.g. phthalimides, sulfamides, chloronitriles): anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.1 0), tolylfluanid (H.3.11), N-(4-chloro-2-nitro-phenyl)-N-ethyl-4-methyl-benzenesulfonamide (H.3.12);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1H,5H-[1,4]dithiino[2,3-c:5,6-c']dipyrrole-1,3,5,7(2H,6H)-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (I.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclomezine (K.1.7), difenzoquat (K.1.8), difenzoquat-methylsulfate (K.1.9), diphenylamin (K.1.10), fenpyrazamine (K.1.11), flumetover (K.1.12), flusulfamide (K.1.13), flutianil (K.1.14), methasulfocarb (K.1.15), nitrapyrin (K.1.16), nitrothal-isopropyl (K.1.18), oxathiapiprolin (K.1.19), tolprocarb (K.1.20), oxin-copper (K.1.21), proquinazid (K.1.22), tebufloquin (K.1.23), tecloftalam (K.1.24), triazoxide (K.1.25), 2-butoxy-6-iodo-3-propylchromen-4-one (K.1.26), 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone (K.1.27), 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone (K.1.28), 2-[3,5-bis(difluoromethyl)-1H-pyrazol-1-yl]-1-[4-(4-{5-[2-chloro-6-(prop-2-yn-1-yl-oxy)phenyl]-4,5-dihydro-1,2-oxazol-3-yl}-1,3-thiazol-2-yl)piperidin-1-yl]ethanone (K.1.29), N-(cyclopropylmethoxyimino-(6-difluoro-methoxy-2,3-difluoro-phenyl)-methyl)-2-phenyl acetamide (K.1.30), N'-(4-(4-chloro-3-trifluoromethyl-phenoxy)-2,5-dimethyl-phenyl)-N-ethyl-N-methyl formamidine (K.1.31), N'-(4-(4-fluoro-3-trifluoromethyl-phenoxy)-2,5-dimethyl-phenyl)-N-ethyl-N-methyl formamidine (K.1.32), N'-(2-methyl-5-trifluoromethyl-4-(3-trimethylsilanyl-propoxy)-phenyl)-N-ethyl-N-methyl formamidine (K.1.33), N'-(5-difluoromethyl-2-methyl-4-(3-trimethylsilanyl-propoxy)-phenyl)-N-ethyl-N-methyl formamidine (K.1.34), methoxyacetic acid 6-tert-butyl-8-fluoro-2,3-dimethyl-quinolin-4-yl ester (K.1.35), 3-[5-(4-methylphenyl)-2,3-dimethyl-isoxazolidin-3-yl]-pyridine (K.1.36), 3-[5-(4-chlorophenyl)-2,3-dimethyl-isoxazolidin-3-yl]-pyridine (pyrisoxazole) (K.1.37), N-(6-methoxy-pyridin-3-yl) cyclopropanecarboxylic acid amide (K.1.38), 5-chloro-1-(4,6-dimethoxy-pyrimidin-2-yl)-2-methyl-1H-benzoimidazole (K.1.39), 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), 2-[2-[(7,8-difluoro-2-methyl-3-quinolyl)oxy]-6-fluoro-phenyl]propan-2-ol (K.1.43), 2-[2-fluoro-6-[(8-fluoro-2-methyl-3-quinolyl)oxy]phen-yl]propan-2-ol (K.1.44), 3-(5-fluoro-3,3,4,4-tetramethyl-3,4-dihydroisoquinolin-1-yl)quinoline (K.1.45), 3-(4,4-difluoro-3,3-dimethyl-3,4-dihydroisoquinolin-1-yl)quinoline (K.1.46), 3-(4,4,5-trifluoro-3,3-dimethyl-3,4-dihydroisoquinolin-1-yl)quinoline (K.1.47);
L) Biopesticides
   L1) Microbial pesticides with fungicidal, bactericidal, viricidal and/or plant defense activator activity: Ampelomyces quisqualis, Aspergillus flavus, Aureobasidium pullulans, Bacillus amyloliquefaciens, B. mojavensis, B. pumilus, B. simplex, B. solisalsi, B. subtilis, B. subtilis var. amyloliquefaciens, Candida oleophila, C. saitoana, Clavibacter michiganensis (bacteriophages), Coniothyrium minitans, Cryphonectria parasitica, Cryptococcus albidus, Dilophosphora alopecuri, Fusarium oxysporum, Clonostachys rosea f. catenulate (also named Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus polymyxa, Pantoea vagans, Phlebiopsis gigantea, Pseudomonas sp., Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperellum, T. atroviride, T. fertile, T. gamsii, T. harmatum, T. harzianum; mixture of T. harzianum and T. viride; mixture of T. polysporum and T. harzianum; T. stromaticum, T. virens (also named Gliocladium virens), T. viride, Typhula phacorrhiza, Ulocladium oudemansii, Verticillium dahlia, zucchini yellow mosaic virus (avirulent strain);
   L2) Biochemical pesticides with fungicidal, bactericidal, viricidal and/or plant defense activator activity: chitosan (hydrolysate), harpin protein, laminarin, Menhaden fish oil, natamycin, Plum pox virus coat protein, potassium or sodium bicarbonate, Reynoutria sachlinensis extract, salicylic acid, tea tree oil;
   L3) Microbial pesticides with insecticidal, acaricidal, molluscidal and/or nematicidal activity: Agrobacterium radiobacter, Bacillus cereus, B. firmus, B. thuringiensis, B. thuringiensis ssp. aizawai, B. t. ssp. israelensis, B. t. ssp. galleriae, B. t. ssp. kurstaki, B. t. ssp. tenebrionis, Beauveria bassiana, B. brongniartii , Burkholderia sp., Chromobacterium subtsugae, Cydia pomonella granulosis virus, Cryptophlebia leucotreta granulovirus (CrIeGV), Isaria fumosorosea, Heterorhabditis bacteriophora, Lecanicillium longisporum, L. muscarium (formerly Verticillium lecanii), Metarhizium anisopliae, M. anisopliae var. acridum, Nomuraea rileyi, Paecilomyces fumosoroseus, P. lilacinus, Paenibacillus popilliae, Pasteuria spp., P. nishizawae, P. penetrans, P. ramose, P. reneformis, P. thornea, P. usgae, Pseudomonas fluorescens, Steinernema carpocapsae, S. feltiae, S. kraussei;
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadecadien-1-ol acetate, (E,Z)-3,13-octadecadien-1-ol, R-1-octen-3-ol, pentatermanone, potassium silicate, sorbitol actanoate, (E,Z,Z)-3,8,11-tetradecatrienyl acetate, (Z,E)-9,12-tetradecadien-1-yl acetate, Z-7-tetradecen-2-one, Z-9-tetradecen-1-yl acetate, Z-11-tetradecenal, Z-11-tetradecen-1-ol, Acacia negra extract, extract of grapefruit seeds and pulp, extract of Chenopodium ambrosiodae, Catnip oil, Neem oil, Quillay extract, Tagetes oil;
   L5) Microbial pesticides with plant stress reducing, plant growth regulator, plant growth promoting and/or yield enhancing activity: Azospirillum amazonense, A. brasilense, A. lipoferum, A. irakense, A. halopraeferens, Bradyrhizobium sp., B. elkanii, B. japonicum, B. liaoningense, B. lupini, Delftia acidovorans, Glomus intraradices, Mesorhizobium sp., Paenibacillus alvei, Penicillium bilaiae, Rhizobium leguminosarum bv. phaseoli, R. I. trifolii, R. I. bv. viciae, R. tropici, Sinorhizobium meliloti;
   L6) Biochemical pesticides with plant stress reducing, plant growth regulator and/or plant yield enhancing activity: abscisic acid, aluminium silicate (kaolin), 3-decen-2-one, formononetin, genistein, hesperetin, homobrassinlide, humates, jasmonic acid or salts or derivatives thereof, lysophosphatidyl ethanolamine, naringenin, polymeric polyhydroxy acid, Ascophyllum nodosum (Norwegian kelp, Brown kelp) extract and Ecklonia maxima (kelp) extract;
M) Growth regulators
   abscisic acid (M.1.1), amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor (N.1.1), alachlor, butachlor, dimethachlor, dimethenamid (N.1.2), flufenacet (N.1.3), mefenacet (N.1.4), metolachlor (N.1.5), metazachlor (N.1.6), napropamide, naproanilide, pethoxamid, pretilachlor, propachlor, thenylchlor;
   - amino acid derivatives: bilanafos, glyphosate (N.2.1), glufosinate (N.2.2), sulfosate (N.2.3);
   - aryloxyphenoxypropionates: clodinafop (N.3.1), cyhalofop-butyl, fenoxaprop (N.3.2), fluazifop (N.3.3), haloxyfop (N.3.4), metamifop, propaquizafop, quizalofop, quizalofop-P-tefuryl;
   - Bipyridyls: diquat, paraquat (N.4.1);
   - (thio)carbamates: asulam, butylate, carbetamide, desmedipham, dimepiperate, eptam (EPTC), esprocarb, molinate, orbencarb, phenmedipham (N.5.1), prosulfocarb, pyributicarb, thiobencarb, triallate;
   - cyclohexanediones: butroxydim, clethodim (N.6.1), cycloxydim (N.6.2), profoxydim (N.6.3), sethoxydim (N.6.4), tepraloxydim (N.6.5), tralkoxydim;
   - dinitroanilines: benfluralin, ethalfluralin, oryzalin, pendimethalin (N.7.1), prodiamine (N.7.2), trifluralin (N.7.3);
   - diphenyl ethers: acifluorfen (N.8.1), aclonifen, bifenox, diclofop, ethoxyfen, fomesafen, lactofen, oxyfluorfen;
   - hydroxybenzonitriles: bomoxynil (N.9.1), dichlobenil, ioxynil;
   - imidazolinones: imazamethabenz, imazamox (N.10.1), imazapic (N.10.2), imazapyr (N.10.3), imazaquin (N.10.4), imazethapyr (N.10.5);
   - phenoxy acetic acids: clomeprop, 2,4-dichlorophenoxyacetic acid (2,4-D) (N.11.1), 2,4-DB, dichlorprop, MCPA, MCPA-thioethyl, MCPB, Mecoprop;
   - pyrazines: chloridazon (N.11.1), flufenpyr-ethyl, fluthiacet, norflurazon, pyridate;
   - pyridines: aminopyralid, clopyralid (N.12.1), diflufenican, dithiopyr, fluridone, fluroxypyr (N.12.2), picloram (N.12.3), picolinafen (N.12.4), thiazopyr;
   - sulfonyl ureas: amidosulfuron, azimsulfuron, bensulfuron (N.13.1), chlorimuron-ethyl (N.13.2), chlorsulfuron, cinosulfuron, cyclosulfamuron (N.13.3), ethoxysulfuron, flazasulfuron, flucetosulfuron, flupyrsulfuron, foramsulfuron, halosulfuron, imazosulfuron, iodosulfuron (N.13.4), mesosulfuron (N.13.5), metazosulfuron, metsulfuron-methyl (N.13.6), nicosulfuron (N.13.7), oxasulfuron, primisulfuron, prosulfuron, pyrazosulfuron, rimsulfuron (N.13.8), sulfometuron, sulfosulfuron, thifensulfuron, triasulfuron, tribenuron, trifloxysulfuron, triflusulfuron (N.13.9), tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine (N.14.1), cyanazine, dimethametryn, ethiozin, hexazinone (N.14.2), metamitron, metribuzin, prometryn, simazine, terbuthylazine, terbutryn, triaziflam;
   - ureas: chlorotoluron, daimuron, diuron (N.15.1), fluometuron, isoproturon, linuron, methabenzthiazuron, tebuthiuron;
   - other acetolactate synthase inhibitors: bispyribac-sodium, cloransulam-methyl, diclosulam, florasulam (N.16.1), flucarbazone, flumetsulam, metosulam, orthosulfamuron, penoxsulam, propoxycarbazone, pyribambenz-propyl, pyribenzoxim, pyriftalid, pyriminobac-methyl, pyrimisulfan, pyrithiobac, pyroxasulfone (N.16.2), pyroxsulam;
   - others: amicarbazone, aminotriazole, anilofos, beflubutamid, benazolin, bencarbazone,benfluresate, benzofenap, bentazone (N.17.1), benzobicyclon, bicyclopyrone, bromacil, bromobutide, butafenacil, butamifos, cafenstrole, carfentrazone, cinidon-ethyl (N.17.2), chlorthal, cinmethylin (N.17.3), clomazone (N.17.4), cumyluron, cyprosulfamide, dicamba (N.17.5), difenzoquat, diflufenzopyr (N.17.6), Drechslera monoceras, endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac (N.17.7), quinmerac (N.17.8), mesotrione (N.17.9), methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil (N.17.10), sulcotrione (N.17.11), sulfentrazone, terbacil, tefuryltrione, tembotrione, thiencarbazone, topramezone (N.17.12), (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chlorophenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester;
O) Insecticides
   - organo(thio)phosphates: acephate (O.1.1), azamethiphos (0.1.2), azinphosmethyl (0.1.3), chlorpyrifos (O.1.4), chlorpyrifos-methyl (0.1.5), chlorfenvinphos (0.1.6), diazinon (0.1.7), dichlorvos (0.1.8), dicrotophos (0.1.9), dimethoate (O.1.10), disulfoton (O.1.11), ethion (0.1.12), fenitrothion (0.1.13), fenthion (0.1.14), isoxathion (0.1.15), malathion (0.1.16), methamidophos (0.1.17), methidathion (0.1.18), methyl-parathion (0.1.19), mevinphos (0.1.20), monocrotophos (O.1.21), oxydemeton-methyl (0.1.22), paraoxon (0.1.23), parathion (0.1.24), phenthoate (0.1.25), phosalone (0.1.26), phosmet (0.1.27), phosphamidon (0.1.28), phorate (0.1.29), phoxim (0.1.30), pirimiphos-methyl (0.1.31), profenofos (0.1.32), prothiofos (0.1.33), sulprophos (0.1.34), tetrachlorvinphos (0.1.35), terbufos (0.1.36), triazophos (0.1.37), trichlorfon (0.1.38);
   - carbamates: alanycarb (O.2.1), aldicarb (O.2.2), bendiocarb (O.2.3), benfuracarb (O.2.4), carbaryl (O.2.5), carbofuran (O.2.6), carbosulfan (O.2.7), fenoxycarb (O.2.8), furathiocarb (O.2.9), methiocarb (0.2.10), methomyl (O.2.11), oxamyl (0.2.12), pirimicarb (0.2.13), propoxur (0.2.14), thiodicarb (0.2.15), triazamate (0.2.16);
   - pyrethroids: allethrin (O.3.1), bifenthrin (O.3.2), cyfluthrin (O.3.3), cyhalothrin (O.3.4), cyphenothrin (O.3.5), cypermethrin (O.3.6), alpha-cypermethrin (O.3.7), beta-cypermethrin (O.3.8), zeta-cypermethrin (O.3.9), deltamethrin (0.3.10), esfenvalerate (O.3.11), etofenprox (O.3.11), fenpropathrin (0.3.12), fenvalerate (0.3.13), imiprothrin (0.3.14), lambda-cyhalothrin (0.3.15), permethrin (0.3.16), prallethrin (0.3.17), pyrethrin I and II (O.3.18), resmethrin (0.3.19), silafluofen (O.3.20), tau-fluvalinate (O.3.21), tefluthrin (O.3.22), tetramethrin (O.3.23), tralomethrin (O.3.24), transfluthrin (O.3.25), profluthrin (O.3.26), dimefluthrin (O.3.27);
   - insect growth regulators: a) chitin synthesis inhibitors: benzoylureas: chlorfluazuron (O.4.1), cyramazin (O.4.2), diflubenzuron (O.4.3), flucycloxuron (O.4.4), flufenoxuron (O.4.5), hexaflumuron (O.4.6), lufenuron (O.4.7), novaluron (O.4.8), teflubenzuron (O.4.9), triflumuron (O.4.10); buprofezin (O.4.11), diofenolan (O.4.12), hexythiazox (O.4.13), etoxazole (O.4.14), clofentazine (O.4.15); b) ecdysone antagonists: halofenozide (O.4.16), methoxyfenozide (O.4.17), tebufenozide (O.4.18), azadirachtin (O.4.19); c) juvenoids: pyriproxyfen (O.4.20), methoprene (O.4.21), fenoxycarb (O.4.22); d) lipid biosynthesis inhibitors: spirodiclofen (O.4.23), spiromesifen (O.4.24), spirotetramat (O.4.24);
   - nicotinic receptor agonists/antagonists compounds: clothianidin (O.5.1), dinotefuran (O.5.2), flupyradifurone (O.5.3), imidacloprid (O.5.4), thiamethoxam (O.5.5), nitenpyram (O.5.6), acetamiprid (O.5.7), thiacloprid (O.5.8), 1-2-chloro-thiazol-5-ylmethyl)-2-nitrimino-3,5-dimethyl-[1,3,5]triazinane (O.5.9);
   - GABA antagonist compounds: endosulfan (0.6.19, ethiprole (O.6.2), fipronil (O.6.3), vaniliprole (O.6.4), pyrafluprole (O.6.5), pyriprole (O.6.6), 5-amino-1-(2,6-dichloro-4-methyl-phenyl)-4-sulfinamoyl-1 H-pyrazole-3-carbothioic acid amide (O.6.7);
   - macrocyclic lactone insecticides: abamectin (O.7.1), emamectin (O.7.2), milbemectin (O.7.3), lepimectin (O.7.4), spinosad (O.7.5), spinetoram (O.7.6);
   - mitochondrial electron transport inhibitor (METI) I acaricides: fenazaquin (0.8.1), pyridaben (O.8.2), tebufenpyrad (O.8.3), tolfenpyrad (O.8.4), flufenerim (O.8.5);
   - METI II and III compounds: acequinocyl (O.9.1), fluacyprim (O.9.2), hydramethylnon (O.9.3);
   - Uncouplers: chlorfenapyr (O.10.1);
   - oxidative phosphorylation inhibitors: cyhexatin (O.11.1), diafenthiuron (0.11.2), fenbutatin oxide (0.11.3), propargite (0.11.4);
   - moulting disruptor compounds: cryomazine (O.12.1);
   - mixed function oxidase inhibitors: piperonyl butoxide (O.13.1);
   - sodium channel blockers: indoxacarb (0.14.1), metaflumizone (0.14.2);
   - ryanodine receptor inhibitors: chlorantraniliprole (O.15.1), cyantraniliprole (O.15.2), flubendiamide (O.15.3), N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide (O.15.4); N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide (O.15.5); N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide (O.15.6); N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide (O.15.7); N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide (O.15.8); N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide (O.15.9); N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide (O.15.10); N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide (O.15.11);
   - others: benclothiaz (O.16.1), bifenazate (0.16.2), artap (0.16.3), flonicamid (0.16.4), pyridalyl (O.16.5), pymetrozine (0.16.6), sulfur (0.16.7), thiocyclam (0.16.8), cyenopyrafen (0.16.9), flupyrazofos (0.16.10), cyflumetofen (O.16.11), amidoflumet (0.16.12), imicyafos (O.16.13), bistrifluron (0.16.14), pyrifluquinazon (O.16.15) and 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a, 12b-trimethyl-11-oxo-9-(3-pyridinyl)-2H,11H-naphtho[2,11-b]pyrano[3,4-e]pyran-3,6-diyl] cyclopropaneacetic acid ester (0.16.16).

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to L), as described above, is more efficient than combating those fungi with individual compounds I or individual fungicides from groups A) to L). By applying compounds I together with at least one active substance from groups A) to O) a synergistic effect can be obtained, i.e. more then simple addition of the individual effects is obtained (synergistic mixtures).
This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compounds I and a pesticide II sequentially the time between both applications may vary e.g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day. In case of a mixture comprising a pesticide II selected from group L), it is preferred that the pesticide II is applied as last treatment.
According to the invention, the solid material (dry matter) of the biopesticides (with the exception of oils such as Neem oil, Tagetes oil, etc.) are considered as active components (e.g. to be obtained after drying or evaporation of the extraction medium or the suspension medium in case of liquid formulations of the microbial pesticides).
In accordance with the present invention, the weight ratios and percentages used herein for a biological extract such as Quillay extract are based on the total weight of the dry content (solid material) of the respective extract(s).
The total weight ratios of compositions comprising at least one microbial pesticide in the form of viable microbial cells including dormant forms, can be determined using the amount of CFU of the respective microorganism to calclulate the total weight of the respective active component with the following equation that 1 x 10⁹ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as Steinernema feltiae.

In binary mixtures, i.e. compositions according to the invention comprising one compound I (component 1) and one further active substance (component 2), e. g. one active substance from groups A) to O), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and even more preferably in the range of from 1:3 to 3:1 and in particular in the range of from 1:2 to 2:1.

According to a further embodiments of the binary mixtures and compositions, the weight ratio of the component 1) and the component 2) usually is in the range of from 1000:1 to 1:1, often in the range of from 100:1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.
According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.
In ternary mixtures, i.e. compositions according to the invention comprising one compound I (component 1) and a first further active substance (component 2) and a second further active substance (component 3), e. g. two active substances from groups A) to O), the weight ratio of component 1 and component 2 depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.
Any further active components are, if desired, added in a ratio of from 20:1 to 1:20 to the component 1).
These ratios are also suitable for inventive mixtures applied by seed treatment.

Preference is also given to mixtures comprising a compound I (component 1) and at least one active substance selected from group A) (component 2) and particularly selected from azoxystrobin (A.1.1), dimoxystrobin (A.1.4), fluoxastrobin (A.1.8), kresoxim-methyl (A.1.9), orysastrobin (A.1.12), picoxystrobin (A.1.13), pyraclostrobin (A.1.14), trifloxystrobin (A.1.17), pyribencarb (A.1.19), famoxadone (A.1.21), cyazofamid (A.2.1), amisulbrom (A.2.2), benzovindiflupyr (A.3.2), bixafen (A.3.3), boscalid (A.3.4), fluopyram (A.3.7), flutolanil (A.3.8), fluxapyroxad (A.3.9), isopyrazam (A.3.12), oxycarboxin (A.3.14), penflufen (A.3.14), penthiopyrad (A.3.15), sedaxane (A.3.16), N-(4'-trifluoromethylthiobiphenyl-2-yl)-3-difluoromethyl-1-methyl-1H-pyrazole-4-carboxamide (A.3.19), N-(2-(1,3,3-trimethyl-butyl)-phenyl)-1,3-dimethyl-5-fluoro-1H-pyrazole-4-carboxamide (A.3.20), 3-(difluoromethyl)-1-methyl-N-(1,1,3-trimethylindan-4-yl)pyrazole-4-carboxamide (A.3.21), 3-(trifluoromethyl)-1-methyl-N-(1,1,3-trimethyl-indan-4-yl)pyrazole-4-carboxamide (A.3.22), 1,3-dimethyl-N-(1,1,3-trimethylindan-4-yl)pyrazole-4-carboxamide (A.3.23), 3-(trifluoromethyl)-1,5-dimethyl-N-(1,1,3-trimethylindan-4-yl)pyrazole-4-carboxamide (A.3.24), 1,3,5-trimethyl-N-(1,1,3-trimethylindan-4-yl)pyrazole-4-carboxamide (A.3.25), N-(7-fluoro-1,1,3-trimethyl-indan-4-yl)-1,3-dimethyl-pyrazole-4-carboxamide (A.3.26), N-[2-(2,4-dichlorophenyl)-2-methoxy-1-methyl-ethyl]-3-(difluoromethyl)-1-methyl-pyrazole-4-carboxamide (A.3.27), dinocap (A.4.5), fluazinam (A.4.6); fentin-acetate (A.4.8), fentin chloride (A.4.9) and ametoctradin (A.4.11).
Preference is given to mixtures comprising a compound of formula I (component 1) and at least one active substance selected from group B) (component 2) and particularly selected from cyproconazole (B.1.4), difenoconazole (B.1.5), diniconazole (B.1.6), epoxiconazole (B.1.8), fluquinconazole (B.1.10), flusilazole (B.1.11), flutriafol (B.1.12), metconazole (B.1.17), myclobutanil (B.1.18), penconazole (B.1.21), propiconazole (B.1.22), prothioconazole (B.1.23), tebuconazole (B.1.25), tetraconazole (B.1.26), triadimefon (B.1.27), triadimenol (B.1.28), triticonazole (B.1.29), uniconazole (B.1.30),
1-[*rel*-(2*S*;3*R*)-3-(2-chlorophenyl)-2-(2,4-difluorophenyl)-oxiranylmethyl]-5-thiocyanato-1H-[1,2,4]triazolo (B.1.31), 2-[*rel*-(2*S*;3R)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chloro-phenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), 2-[4-(4-chloro-phenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1-(1,2,4-triazol-1-yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), imazalil (B.1.42), prochloraz (B.1.44), fenarimol (B.1.46), triforine (B.1.49), [3-(4-chloro-2-fluoro-phenyl)-5-(2,4-difluorophenyl)isoxazol4-yl]-(3-pyridyl)methanol (B.1.50), dodemorph (B.2.2), fenpropimorph (B.2.4), tridemorph (B.2.5), fenpropidin (B.2.6), piperalin (B.2.7), spiroxamine (B.2.8) and fenhexamid (B.3.1).
Preference is given to mixtures comprising a compound of formula I (component 1) and at least one active substance selected from group C) (component 2) and particularly selected from metalaxyl (C.1.4), metalaxyl-M (mefenoxam, C.1.5), ofurace (C.1.6) and bupirimate (C.2.4).
Preference is given to mixtures comprising a compound of formula I (component 1) and at least one active substance selected from group D) (component 2) and particularly selected from benomyl (D1.1), carbendazim (D1.2), thiabendazole (D1.4), thiophanate-methyl (D1.5), ethaboxam (D2.2), fluopicolide (D2.4), zoxamide (D2.5), metrafenone (D2.6), pyriofenone (D2.7).
Preference is also given to mixtures comprising a compound I (component 1) and at least one active substance selected from group E) (component 2) and particularly selected from cyprodinil (E.1.1), mepanipyrim (E.1.2), pyrimethanil (E.1.3). Preference is also given to mixtures comprising a compound I (component 1) and at least one active substance selected from group F) (component 2) and particularly selected from iprodione (F.1.2), vinclozolin (F.1.4), fenpiclonil (F.1.5), fludioxonil (F.1.6) and quinoxyfen (F.2.1).
Preference is also given to mixtures comprising a compound I (component 1) and at least one active substance selected from group G) (component 2) and particularly selected from dimethomorph (G.3.1), flumorph (G.3.2), mandipropamid (G.3.3), pyrimorph (G.3.4), benthiavalicarb (G.3.5), iprovalicarb (G.3.6), propamocarb (G.4.1), oxathiapiprolin (G.5.1).
Preference is also given to mixtures comprising a compound I (component 1) and at least one active substance selected from group H) (component 2) and particularly selected from copper acetate (H.1.2), copper hydroxide (H.1.3), copper oxychloride (H.1.4), basic copper sulfate (H.1.5), sulfur (H.1.6), mancozeb (H.2.2), metiram (H.2.5), thiram (H.2.7), chlorothalonil (H.3.2), captafol (H.3.3), captan (H.3.4), folpet (H.3.5), dichlofluanid (H.3.6), N-(4-chloro-2-nitro-phenyl)-N-ethyl-4-methyl-benzenesulfonamide (H.3.12), dodine (H.4.2), iminoctadine (H.4.6), dithianon (H.4.9) and 2,6-dimethyl-1 H,5H-[1,4]dithiino[2,3-c:5,6-c']dipyrrole-1,3,5,7(2H,6H)-tetraone (H.4.10).
Preference is also given to mixtures comprising a compound I (component 1) and at least one active substance selected from group I) (component 2) and particularly selected from carpropamid (I.2.3), and fenoxanil (I.2.5).
Preference is also given to mixtures comprising a compound I (component 1) and at least one active substance selected from group J) (component 2) and particularly selected from acibenzolar-S-methyl (J.1.1), probenazole (J.1.2), isotianil (J.1.3), tiadinil (J.1.4), prohexadione-calcium (J.1.5), fosetyl (J.1.6), fosetyl-aluminum (J.1.7), phosphorous acid and its salts (J.1.8), potassium or sodium bicarbonate (J.1.9). Preference is also given to mixtures comprising a compound I (component 1) and at least one active substance selected from group K) (component 2) and particularly selected from cymoxanil (K.1.4), dazomet (K.1.5), difenzoquat (K.1.8), flumetover (K.1.12), flusulfamide (K.1.13), flutianil (K.1.14), methasulfocarb (K.1.15), oxathiapiprolin (K.1.19) and proquinazid (K.1.22).

The biopesticides from group L) of pesticides II, their preparation and their pesticidal activity e.g. against harmful fungi or insects are known (e-Pesticide Manual V 5.2 (ISBN 978 1 901396 85 0) (2008-2011); http://www.epa.gov/opp00001/biopesticides/, see product lists therein; http://www.omri.org/omri-lists, see lists therein; Bio-Pesticides Database BPDB http://sitem.herts.ac.uk/aeru/bpdb/, see A to Z link therein).
The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) and/or L6) may also have fungicidal, bactericidal, viricidal, plant defense activator, insecticidal, acaricidal, molluscidal, pheromone and/or nematicidal activity.

Many of these biopesticides are registered and/or are commercially available: aluminium silicate (Screen™ Duo from Certis LLC, USA), Agrobacterium radiobacter K1026 (e.g. NoGall® from Becker Underwood Pty Ltd., Australia), A. radiobacter K84 (Nature 280, 697-699, 1979; e.g. GallTrol® from AG Biochem, Inc., C, USA), Ampelomyces quisqualis M-10 (e.g. AQ 10® from Intrachem Bio GmbH & Co. KG, Germany), Ascophyllum nodosum (Norwegian kelp, Brown kelp) extract or filtrate (e.g. ORKA GOLD from Becker Underwood, South Africa; or Goemar® from Laboratoires Goemar, France), Aspergillus flavus NRRL 21882 isolated from a peanut in Georgia in 1991 by the USDA, National Peanut Research Laboratory (e.g. in Afla-Guard® from Syngenta, CH), mixtures of Aureobasidium pullulans DSM14940 and DSM 14941 (e.g. blastospores in BlossomProtect® from bio-ferm GmbH, Germany), Azospirillum amazonense BR 11140 (SpY2^{T}) (Proc. 9th Int. and 1st Latin American PGPR meeting, Quimara, Medellín, Colombia 2012, p. 60, ISBN 978-958-46-0908-3), A. brasilense AZ39 (Eur. J. Soil Biol 45(1), 28-35, 2009), A. brasilense XOH (e.g. AZOS from Xtreme Gardening, USA or RTI Reforestation Technologies International; USA), A. brasilense BR 11002 (Proc. 9th Int. and 1st Latin American PGPR meeting, Quimara, Medellín, Colombia 2012, p. 60, ISBN 978-958-46-0908-3), A. brasilense BR 11005 (SP245; e.g. in GELFIX Gramíneas from BASF Agricultural Specialties Ltd., Brazil), A. lipoferum BR 11646 (Sp31) (Proc. 9th Int. and 1st Latin American PGPR meeting, Quimara, Medellín, Colombia 2012, p. 60), Bacillus amyloliquefaciens FZB42 (e.g. in RhizoVital® 42 from AbiTEP GmbH, Berlin, Germany), B. amyloliquefaciens IN937a (J. Microbiol. Biotechnol. 17(2), 280-286, 2007; e.g. in BioYield® from Gustafson LLC, TX, USA), B. amyloliquefaciens IT-45 (CNCM I-3800) (e.g. Rhizocell C from ITHEC, France), B. amyloliquefaciens subsp. plantarum MBI600 (NRRL B-50595, deposited at United States Department of Agriculture) (e.g. Integral®, Subtilex® NG from Becker Underwood, USA), B. cereus CNCM I-1562 (US 6,406,690), B. firmus CNCM I-1582 (WO 2009/126473, WO 2009/124707, US 6,406,690; Votivo® from Bayer Crop Science LP, USA), B. pumilus GB34 (ATCC 700814; e.g. in YieldShield® from Gustafson LLC, TX, USA), and Bacillus pumilus KFP9F (NRRL B-50754) (e.g. in BAC-UP or FUSION-P from Becker Underwood South Africa), B. pumilus QST 2808 (NRRL B-30087) (e.g. Sonata® and Ballad® Plus from AgraQuest Inc., USA), B. subtilis GB03 (e.g. Kodiak® or BioYield® from Gustafson, Inc., USA; or Companion® from Growth Products, Ltd., White Plains, NY 10603, USA), B. subtilis GB07 (Epic® from Gustafson, Inc., USA), B. subtilis QST-713 (NRRL B-21661 in Rhapsody®, Serenade® MAX and Serenade® ASO from AgraQuest Inc., USA), B. subtilis var. amyloliquefaciens FZB24 (e.g. Taegro® from Novozyme Biologicals, Inc., USA), B. subtilis var. amyloliquefaciens D747 (e.g. Double Nickel 55 from Certis LLC, USA), B. thuringiensis ssp. aizawai ABTS-1857 (e.g. in XenTari® from BioFa AG, Münsingen, Germany), B. t. ssp. aizawai SAN 401 1, ABG-6305 and ABG-6346, Bacillus t. ssp. israelensis AM65-52 (e.g. in VectoBac® from Valent BioSciences, IL, USA), Bacillus thuringiensis ssp. kurstaki SB4 (NRRL B-50753; e.g. Beta Pro® from Becker Underwood, South Africa), B. t. ssp. kurstaki ABTS-351 identical to HD-1 (ATCC SD-1275; e.g. in Dipel® DF from Valent BioSciences, IL, USA), B. t. ssp. kurstaki EG 2348 (e.g. in Lepinox® or Rapax® from CBC (Europe) S.r.l., Italy), B. t. ssp. tenebrionis DSM 2803 (EP 0 585 215 B1; identical to NRRL B-15939; Mycogen Corp.), B. t. ssp. tenebrionis NB-125 (DSM 5526; EP 0 585 215 B1; also referred to as SAN 418 I or ABG-6479; former production strain of Novo-Nordisk), B. t. ssp. tenebrionis NB-176 (or NB-176-1) a gamma-irridated, induced high-yielding mutant of strain NB-125 (DSM 5480; EP 585 215 B1; Novodor® from Valent BioSciences, Switzerland), Beauveria bassiana ATCC 74040 (e.g. in Naturalis® from CBC (Europe) S.r.l., Italy), B. bassiana DSM 12256 (US 200020031495; e.g. BioExpert® SC from Live Sytems Technology S.A., Colombia), B. bassiana GHA (BotaniGard® 22WGP from Laverlam Int. Corp., USA), B. bassiana PPRI 5339 (ARSEF number 5339 in the USDA ARS collection of entomopathogenic fungal cultures; NRRL 50757) (e.g. BroadBand® from Becker Underwood, South Africa), B. brongniartii (e.g. in Melocont® from Agrifutur, Agrianello, Italy, for control of cockchafer; J. Appl. Microbiol. 100(5),1063-72, 2006), Bradyrhizobium sp. (e.g. Vault® from Becker Underwood, USA), B. japonicum (e.g. VAULT® from Becker Underwood, USA), Candida oleophila I-182 (NRRL Y-18846; e.g. Aspire® from Ecogen Inc., USA, Phytoparasitica 23(3), 231-234, 1995), C. oleophila strain O (NRRL Y-2317; Biological Control 51, 403-408, 2009),, Candida saitoana (e.g. Biocure® (in mixture with lysozyme) and BioCoat® from Micro Flo Company, USA (BASF SE) and Arysta), Chitosan (e.g. Armour-Zen® from BotriZen Ltd., NZ), Clonostachys rosea f. catenulata, also named Gliocladium catenulatum (e.g. isolate J 1446: Prestop® from Verdera Oy, Finland), Chromobacterium subtsugae PRAA4-1 isolated from soil under an eastern hemlock (Tsuga canadensis) in the Catoctin Mountain region of central Maryland (e.g. in GRANDEVO from Marrone Bio Innovations, USA), Coniothyrium minitans CON/M/91-08 (e.g. Contans® WG from Prophyta, Germany), Cryphonectria parasitica (e.g. Endothia parasitica from CNICM, France), Cryptococcus albidus (e.g. YIELD PLUS® from Anchor Bio-Technologies, South Africa), Cryptophlebia leucotreta granulovirus (CrIeGV) (e.g. in CRYPTEX from Adermatt Biocontrol, Switzerland), Cydia pomonella granulovirus (CpGV) V03 (DSM GV-0006; e.g. in MADEX Max from Andermatt Biocontrol, Switzerland), CpGV V22 (DSM GV-0014; e.g. in MADEX Twin from Adermatt Biocontrol, Switzerland), Delftia acidovorans RAY209 (ATCC PTA-4249; WO 2003/57861; e.g. in BIOBOOST from Brett Young, Winnipeg, Canada), Dilophosphora alopecuri (Twist Fungus from Becker Underwood, Australia), Ecklonia maxima (kelp) extract (e.g. KELPAK SL from Kelp Products Ltd, South Africa), formononetin (e.g. in MYCONATE from Plant Health Care plc, U.K.), Fusarium oxysporum (e.g. BIOFOX® from S.I.A.P.A., Italy, FUSACLEAN® from Natural Plant Protection, France), Glomus intraradices (e.g. MYC 4000 from ITHEC, France), Glomus intraradices RTI-801 (e.g. MYKOS from Xtreme Gardening, USA or RTI Reforestation Technologies International; USA), grapefruit seeds and pulp extract (e.g. BC-1 000 from Chemie S.A., Chile), harpin (alpha-beta) protein (e.g. MESSENGER or HARP-N-Tek from Plant Health Care plc, U.K.; Science 257, 1-132, 1992), Heterorhabditis bacteriophaga (e.g. Nemasys® G from Becker Underwood Ltd., UK), Isaria fumosorosea Apopka-97 (ATCC 20874) (PFR-97™ from Certis LLC, USA), cis-jasmone (US 8,221,736), laminarin (e.g. in VACCIPLANT from Laboratoires Goemar, St. Malo, France or Stähler SA, Switzerland), Lecanicillium longisporum KV42 and KV71 (e.g. VERTALEC® from Koppert BV, Netherlands), L. muscarium KV01 (formerly Verticillium lecanii) (e.g. MYCOTAL from Koppert BV, Netherlands), Lysobacter antibioticus 13-1 (Biological Control 45, 288-296, 2008), L. antibioticus HS124 (Curr. Microbiol. 59(6), 608-615, 2009), L. enzymogenes 3.1T8 (Microbiol. Res. 158, 107-115; Biological Control 31(2), 145-154, 2004), Metarhizium anisopliae var. acridum IMI 330189 (isolated from Ornithacris cavroisi in Niger; also NRRL 50758) (e.g. GREEN MUSCLE® from Becker Underwood, South Africa), M. a. var. acridum FI-985 (e.g. GREEN GUARD® SC from Becker Underwood Pty Ltd, Australia), M. anisopliae FI-1045 (e.g. BIOCANE® from Becker Underwood Pty Ltd, Australia), M. anisopliae F52 (DSM 3884, ATCC 90448; e.g. MET52® Novozymes Biologicals BioAg Group, Canada), M. anisopliae ICIPE 69 (e.g. METATHRIPOL from ICIPE, Nairobe, Kenya), Metschnikowia fructicola (NRRL Y-30752; e.g. SHEMER® from Agrogreen, Israel, now distributed by Bayer CropSciences, Germany; US 6,994,849), Microdochium dimerum (e.g. ANTIBOT® from Agrauxine, France), Microsphaeropsis ochracea P130A (ATCC 74412 isolated from apple leaves from an abandoned orchard, St-Joseph-du-Lac, Quebec, Canada in 1993; Mycologia 94(2), 297-301, 2002), Muscodor albus QST 20799 originally isolated from the bark of a cinnamon tree in Honduras (e.g. in development products Muscudor™ or QRD300 from AgraQuest, USA), Neem oil (e.g. TRILOGY®, TRIACT® 70 EC from Certis LLC, USA), Nomuraea rileyi strains SA86101, GU87401, SR86151, CG128 and VA9101, Paecilomyces fumosoroseus FE 9901 (e.g. NO FLY™ from Natural Industries, Inc., USA), P. lilacinus 251 (e.g. in BioAct®/MeloCon® from Prophyta, Germany; Crop Protection 27, 352-361, 2008; originally isolated from infected nematode eggs in the Philippines), P. lilacinus DSM 15169 (e.g. NEMATA® SC from Live Systems Technology S.A., Colombia), P. lilacinus BCP2 (NRRL 50756; e.g. PL GOLD from Becker Underwood BioAg SA Ltd, South Africa), mixture of Paenibacillus alvei NAS6G6 (NRRL B-50755), Pantoea vagans (formerly agglomerans) C9-1 (originally isolated in 1994 from apple stem tissue; BlightBan C9-1® from NuFrams America Inc., USA, for control of fire blight in apple; J. Bacteriol. 192(24) 6486-6487, 2010), Pasteuria spp. ATCC PTA-9643 (WO 2010/085795), Pasteuria spp. ATCC SD-5832 (WO 2012/064527), P. nishizawae (WO 2010/80169), P. penetrans (US 5,248,500), P. ramose (WO 2010/80619), P. thornea (WO 2010/80169), P. usgae (WO 2010/80169), Penicillium bilaiae (e.g. Jump Start® from Novozymes Biologicals BioAg Group, Canada, originally isolated from soil in southern Alberta; Fertilizer Res. 39, 97-103, 1994), Phlebiopsis gigantea (e.g. RotStop® from Verdera Oy, Finland), Pichia anomala WRL-076 (NRRL Y-30842; US 8,206,972), potassium bicarbonate (e.g. Amicarb® fromm Stähler SA, Switzerland), potassium silicate (e.g. Sil-MATRIX™ from Certis LLC, USA), Pseudozyma flocculosa PF-A22 UL (e.g. Sporodex® from Plant Products Co. Ltd., Canada), Pseudomonas sp. DSM 13134 (WO 2001/40441, e.g. in PRORADIX from Sourcon Padena GmbH & Co. KG, Hechinger Str. 262, 72072 Tübingen, Germany), P. chloraphis MA 342 (e.g. in CERALL or CEDEMON from BioAgri AB, Uppsala, Sweden), P. fluorescens CL 145A (e.g. in ZEQUANOX from Marrone Biolnnovations, Davis, CA, USA; J. Invertebr. Pathol. 113(1):104-14, 2013), Pythium oligandrum DV 74 (ATCC 38472; e.g. POLYVERSUM® from Remeslo SSRO, Biopreparaty, Czech Rep. and GOWAN, USA; US 2013/0035230), Reynoutria sachlinensis extract (e.g. REGALIA® SC from Marrone Biolnnovations, Davis, CA, USA), Rhizobium leguminosarum bv. phaseoli (e.g. RHIZO-STICK from Becker Underwood, USA), R. I. trifolii RP113-7 (e.g. DORMAL from Becker Underwood, USA; Appl. Environ. Microbiol. 44(5), 1096-1101), R. I. bv. viciae P1NP3Cst (also referred to as 1435; New Phytol 179(1), 224-235, 2008; e.g. in NODULATOR PL Peat Granule from Becker Underwood, USA; or in NODULATOR XL PL bfrom Becker Underwood, Canada), R. I. bv. viciae SU303 (e.g. NODULAID Group E from Becker Underwood, Australia), R. I. bv. viciae WSM1455 (e.g. NODULAID Group F from Becker Underwood, Australia), R. tropici SEMIA 4080 (identical to PRF 81; Soil Biology & Biochemistry 39, 867-876, 2007), Sinorhizobium meliloti MSDJ0848 (INRA, France) also referred to as strain 2011 or RCR2011 (Mol Gen Genomics (2004) 272: 1-17; e.g. DORMAL ALFALFA from Becker Underwood, USA; NITRAGIN® Gold from Novozymes Biologicals BioAg Group, Canada), Sphaerodes mycoparasitica IDAC 301008-01 (WO 2011/022809), Steinernema carpocapsae (e.g. MILLENIUM® from Becker Underwood Ltd., UK), S. feltiae (NEMASHIELD® from BioWorks, Inc., USA; NEMASYS® from Becker Underwood Ltd., UK), S. kraussei L137 (NEMASYS® L from Becker Underwood Ltd., UK), Streptomyces griseoviridis K61 (e.g. MYCOSTOP® from Verdera Oy, Espoo, Finland; Crop Protection 25, 468-475, 2006), S. lydicus WYEC 108 (e.g. Actinovate® from Natural Industries, Inc., USA, US 5,403,584), S. violaceusniger YCED-9 (e.g. DT-9® from Natural Industries, Inc., USA, US 5,968,503), Talaromyces flavus V117b (e.g. PROTUS® from Prophyta, Germany), Trichoderma asperellum SKT-1 (e.g. ECO-HOPE® from Kumiai Chemical Industry Co., Ltd., Japan), T. asperellum ICC 012 (e.g. in TENET WP, REMDIER WP, BIOTEN WP from Isagro NC, USA, BIO-TAM from AgraQuest, USA), T. atroviride LC52 (e.g. SENTINEL® from Agrimm Technologies Ltd, NZ), T. atroviride CNCM I-1237 (e.g. in Esquive WG from Agrauxine S.A., France, e.g. against pruning wound diseases on vine and plant root pathogens), T. fertile JM41 R (NRRL 50759; e.g. RICHPLUS™ from Becker Underwood Bio Ag SA Ltd, South Africa), T. gamsii ICC 080 (e.g. in TENET WP, REMDIER WP, BIOTEN WP from Isagro NC, USA, BIO-TAM from AgraQuest, USA), T. harzianum T-22 (e.g. PLANTSHIELD® der Firma BioWorks Inc., USA), T. harzianum TH 35 (e.g. ROOT PRO® from Mycontrol Ltd., Israel), T. harzianum T-39 (e.g. TRICHODEX® and TRICHODERMA 2000® from Mycontrol Ltd., Israel and Makhteshim Ltd., Israel), T. harzianum and T. viride (e.g. TRICHOPEL from Agrimm Technologies Ltd, NZ), T. harzianum ICC012 and T. viride ICC080 (e.g. REMEDIER® WP from Isagro Ricerca, Italy), T. polysporum and T. harzianum (e.g. BINAB® from BINAB Bio-Innovation AB, Sweden), T. stromaticum (e.g. TRICOVAB® from C.E.P.L.A.C., Brazil), T. virens GL-21 (also named Gliocladium virens) (e.g. SOILGARD® from Certis LLC, USA), T. viride (e.g. TRIECO® from Ecosense Labs. (India) Pvt. Ltd., Indien, BIO-CURE® F from T. Stanes & Co. Ltd., Indien), T. viride TV1 (e.g. T. viride TV1 from Agribiotec srl, Italy) and Ulocladium oudemansii HRU3 (e.g. in BOTRY-ZEN® from Botry-Zen Ltd, NZ). Strains can be sourced from genetic resource and deposition centers: American Type Culture Collection, 10801 University Blvd., Manassas, VA 20110-2209, USA (strains with ATCC prefic); CABI Europe - International Mycological Institute, Bakeham Lane, Egham, Surrey, TW20 9TYNRRL, UK (strains with prefices CABI and IMI); Centraalbureau voor Schimmelcultures, Fungal Biodiversity Centre, Uppsalaan 8, PO Box 85167, 3508 AD Utrecht, Netherlands (strains with prefic CBS); Division of Plant Industry, CSIRO, Canberra, Australia (strains with prefix CC); Collection Nationale de Cultures de Microorganismes, Institut Pasteur, 25 rue du Docteur Roux, F-75724 PARIS Cedex 15 (strains with prefix CNCM); Leibniz-Institut DSMZ-Deutsche Sammlung von Mikroorganismen und Zellkulturen GmbH, Inhoffenstraße 7 B, 38124 Braunschweig, Germany (strains with prefix DSM); International Depositary Authority of Canada Collection, Canada (strains with prefix IDAC); Interntional Collection of Micro-orgniasms from Plants, Landcare Research, Private Bag 92170, Auckland Mail Centre, Auckland 1142, New Zealand (strans with prefix ICMP); IITA, PMB 5320, Ibadan, Nigeria (straisn with prefix IITA); The National Collections of Industrial and Marine Bacteria Ltd., Torry Research Station, P.O. Box 31, 135 Abbey Road, Aberdeen, AB9 8DG, Scotland (strains with prefix NCIMB); ARS Culture Collection of the National Center for Agricultural Utilization Research, Agricultural Research Service, U.S. Department of Agriculture, 1815 North University Street, Peoria, Illinois 61604, USA (strains with prefix NRRL); Department of Scientific and Industrial Research Culture Collection, Applied Biochemistry Division, Palmerston North, New Zealand (strains with prefix NZP); FEPAGRO-Fundação Estadual de Pesquisa Agropecuária, Rua Gonçalves Dias, 570, Bairro Menino Deus, Porto Alegre/RS, Brazil (strains with prefix SEMIA); SARDI, Adelaide, South Australia (strains with prefix SRDI); U.S. Department of Agriculture, Agricultural Research Service, Soybean and Alfalfa Research Laboratory, BARC-West, 10300 Baltimore Boulevard, Building 011, Room 19-9, Beltsville, MD 20705, USA (strains with prefix USDA: Beltsville Rhizobium Culture Collection Catalog March 1987 USDA-ARS ARS-30:
http://pdf.usaid.gov/pdf_docs/PNAAW891.pdf); and Murdoch University, Perth, Western Australia (strains with prefix WSM). Further strains may be found at the Global catalogue of Microorganisms: http://gcm.wfcc.info/ and
http://www.landcareresearch.co.nz/resources/collections/icmp and further references to strain collections and their prefixes at http://refs.wdcm.org/collections.htm.
Bacillus amyloliquefaciens subsp. plantarum MBI600 (NRRL B-50595) is deposited under accession number NRRL B-50595 with the strain designation Bacillus subtilis 1430 (and identical to NCIMB 1237). Recently, MBI 600 has been re-classified as Bacillus amyloliquefaciens subsp. plantarum based on polyphasic testing which combines classical microbiological methods relying on a mixture of traditional tools (such as culture-based methods) and molecular tools (such as genotyping and fatty acids analysis). Thus, Bacillus subtilis MBI600 (or MBI 600 or MBI-600) is identical to Bacillus amyloliquefaciens subsp. plantarum MBI600, formerly Bacillus subtilis MBI600. Bacillus amyloliquefaciens MBI600 is known as plant growth-promoting rice seed treatment from Int. J. Microbiol. Res. 3(2) (2011), 120-130 and further described e.g. in US 2012/0149571 A1. This strain MBI600 is e.g. commercially available as liquid formulation product INTEGRAL® (Becker-Underwood Inc., USA).
Bacillus subtilis strain FB17 was originally isolated from red beet roots in North America (System Appl. Microbiol 27 (2004) 372-379). This B. subtilis strain promotes plant health (US 2010/0260735 A1; WO 2011/109395 A2). B. subtilis FB17 has also been deposited at ATCC under number PTA-11857 on April 26, 2011. Bacillus subtilis strain FB17 may be referred elsewhere to as UD1022 or UD10-22.
Bacillus amyloliquefaciens AP-136 (NRRL B-50614), B. amyloliquefaciens AP-188 (NRRL B-50615), B. amyloliquefaciens AP-218 (NRRL B-50618), B. amyloliquefaciens AP-219 (NRRL B-50619), B. amyloliquefaciens AP-295 (NRRL B-50620), B. japonicum SEMIA 5079 (e.g. Gelfix 5 or Adhere 60 from Nitral Urbana Laoboratories, Brazil, a BASF Company), B. japonicum SEMIA 5080 (e.g. GELFIX 5 or ADHERE 60 from Nitral Urbana Laoboratories, Brazil, a BASF Company), B. mojavensis AP-209 (NRRL B-50616), B. solisalsi AP-217 (NRRL B-50617), B. pumilus strain INR-7 (otherwise referred to as BU-F22 (NRRL B-50153) and BU-F33 (NRRL B-50185)), B. simplex ABU 288 (NRRL B-50340) and B. amyloliquefaciens subsp. plantarum MBI600 (NRRL B-50595) have been mentioned i.a. in US patent appl. 20120149571, US 8,445,255, WO 2012/079073. Bradyrhizobium japonicum USDA 3 is known from US patent 7,262,151. Jasmonic acid or salts (jasmonates) or derivatives include without limitation potassium jasmonate, sodium jasmonate, lithium jasmonate, ammonium jasmonate, dimethyl-ammonium jasmonate, isopropylammonium jasmonate, diolammonium jasmonate, diethtriethanolammonium jasmonate, jasmonic acid methyl ester, jasmonic acid amide, jasmonic acid methylamide, jasmonic acid-L-amino acid (amide-linked) conjugates (e.g., conjugates with L-isoleucine, L-valine, L-leucine, or L-phenylalanine), 12-oxo-phytodienoic acid, coronatine, coronafacoyl-L-serine, coronafacoyl-L-threonine, methyl esters of 1-oxo-indanoyl-isoleucine, methyl esters of 1-oxo-indanoyl-leucine, coronalon (2-[(6-ethyl-I-oxo-indane-4-carbonyl) -amino]-3-methyl -pentanoic acid methyl ester), linoleic acid or derivatives thereof and cis-jasmone, or combinations of any of the above.
Humates are humic and fulvic acids extracted from a form of lignite coal and clay, known as leonardite. Humic acids are organic acids that occur in humus and other organically derived materials such as peat and certain soft coal. They have been shown to increase fertilizer efficiency in phosphate and micro-nutrient uptake by plants as well as aiding in the development of plant root systems.

According to one embodiment of the inventive mixtures, the at least one pesticide II is selected from the groups L1) to L6):
L1) Microbial pesticides with fungicidal, bactericidal, viricidal and/or plant defense activator activity: Ampelomyces quisqualis M-10 (L.1.1), Aspergillus flavus NRRL 21882 (L1.2), Aureobasidium pullulans DSM 14940 (L1.3), A. pullulans DSM 14941 (L.1.4), Bacillus amyloliquefaciens AP-136 (NRRL B-50614) (L.1.5), B. amyloliquefaciens AP-188 (NRRL B-50615) (L.1.6), B. amyloliquefaciens AP-218 (NRRL B-50618) (L.1.7), B. amyloliquefaciens AP-219 (NRRL B-50619) (L.1.8), B. amyloliquefaciens AP-295 (NRRL B-50620) (L.1.9), B. amyloliquefaciens FZB42 (L.1.10), B. amyloliquefaciens IN937a (L.1.11), B. amyloliquefaciens IT-45 (CNCM I-3800) (L.1.12), B. amyloliquefaciens subsp. plantarum MBI600 (NRRL B-50595) (L.1.13), B. mojavensis AP-209 (NRRL B-50616) (L.1.15), B. pumilus INR-7 (otherwise referred to as BU-F22 (NRRL B-50153) and BU-F33 (NRRL B-50185)) (L.1.14), B. pumilus KFP9F (L.1.15), B. pumilus QST 2808 (NRRL B-30087) (L.1.16), B. pumilus GHA 181 (L.1.17), B. simplex ABU 288 (NRRL B-50340) (L.1.18), B. solisalsi AP-217 (NRRL B-50617) (L.1.19), B. subtilis CX-9060 (L.1.20), B. subtilis GB03 (L.1.21), B. subtilis GB07 (L.1.22), B. subtilis QST-713 (NRRL B-21661) (L.1.23), B. subtilis var. amyloliquefaciens FZB24 (L.1.24), B. subtilis var. amyloliquefaciens D747 (L.1.25), Candida oleophila I-82 (L.1.26), C. oleophila O (L.1.27), C. saitoana (L.1.28), Clavibacter michiganensis (bacteriophages) (L.1.29), Coniothyrium minitans CON/M/91-08 (L.1.30), Cryphonectria parasitica (L.1.31), Cryptococcus albidus (L.1.32), Dilophosphora alopecuri (L.1.33), Fusarium oxysporum (L.1.34), Clonostachys rosea f. catenulata J1446 (also named Gliocladium catenulatum) (L.1.35), Gliocladium roseum 321 U (L.1.36), Metschnikowia fructicola NRRL Y-30752 (L.1.37), Microdochium dimerum (L.1.38), Microsphaeropsis ochracea P130A (L.1.39), Muscodor albus QST 20799 (L.1.40), Paenibacillus polymyxa PKB1 (ATCC 202127) (L.1.41), Pantoea vagans C9-1 (L.1.42), Phlebiopsis gigantea (L.1.43), Pichia anomala WRL-76 (L.1.44), Pseudozyma flocculosa PF-A22 UL (L.1.45), Pythium oligandrum DV 74 (L.1.46), Sphaerodes mycoparasitica IDAC 301008-01 (L.1.47), Streptomyces griseoviridis K61 (L.1.48), S. lydicus WYEC 108 (L.1.49), S. violaceusniger XL-2 (L.1.50), S. violaceusniger YCED-9 (L.1.51), Talaromyces flavus V117b (L.1.52), Trichoderma asperellum T34 (L.1.53), T. asperellum SKT-1 (L.1.54), T. asperellum ICC 012 (L.1.55), T. atroviride LC52 (L.1.56), T. atroviride CNCM I-1237 (L.1.57), T. fertile JM41 R (L.1.58), T. gamsii ICC 080 (L.1.59), T. harmatum TH 382 (L.1.60), T. harzianum TH-35 (L.1.61), T. harzianum T-22 (L.1.62), T. harzianum T-39 (L.1.63); mixture of T. harzianum ICC012 and T. viride ICC080 (L.1.64); mixture of T. polysporum and T. harzianum (L.1.65); T. stromaticum (L.1.66), T. virens (also named Gliocladium virens) GL-21 (L.1.67), T. virens G41 (L.1.68), T. viride TV1 (L.1.69), Typhula phacorrhiza 94671 (L.1.70), Ulocladium oudemansii HRU3 (L.1.71), Verticillium dahlia (L.1.72), zucchini yellow mosaic virus (avirulent strain) (L.1.73);
L2) Biochemical pesticides with fungicidal, bactericidal, viricidal and/or plant defense activator activity: chitosan (hydrolysate) (L.2.1), harpin protein (L.2.2), laminarin (L.2.3), Menhaden fish oil (L.2.4), natamycin (L.2.5), Plum pox virus coat protein (L.2.6), potassium bicarbonate (L.2.7), Reynoutria sachlinensis extract (L.2.8), salicylic acid (L.2.9), potassium or sodium bicarbonate (L.2.1 0), tea tree oil (L.2.11);
L3) Microbial pesticides with insecticidal, acaricidal, molluscidal and/or nematicidal activity: Agrobacterium radiobacter K1026 (L.3.1), A radiobacter K84 (L.3.2), Bacillus firmus I-1582 (L.3.3); B. thuringiensis ssp. aizawai strains ABTS-1857 (L.3.4), SAN 401 I (L.3.5), ABG-6305 (L.3.6) and ABG-6346 (L.3.7); B. t. ssp. israelensis AM65-52 (L.3.8), B. t. ssp. israelensis SUM-6218 (L.3.9), B. t. ssp. galleriae SDS-502 (L.3.10), B. t. ssp. kurstaki EG 2348 (L.3.11), B. t. ssp. kurstaki SB4 (L.3.12), B. t. ssp. kurstaki ABTS-351 (HD-1) (L.3.13), Beauveria bassiana ATCC 74040 (L.3.14), B. bassiana GHA (L.3.15), B. bassiana H123 (L.3.16), B. bassiana DSM 12256 (L.3.17), B. bassiana PPRI 5339 (L.3.18), B. brongniartii (L.3.19), Burkholderia sp. A396 (L.3.20), Chromobacterium subtsugae PRAA4-1 (L.3.21), Cydia pomonella granulosis virus V22 (L.3.22), Cydia pomonella granulosis virus V1 (L.3.23), Isaria fumosorosea Apopka-97 (L.3.24), Lecanicillium longisporum KV42 (L.3.25), L. longisporum KV71 (L.3.26), L. muscarium (formerly Verticillium lecanii) KV01 (L.3.27), Metarhizium anisopliae FI-985 (L.3.28), M. anisopliae FI-1045 (L.3.29), M. anisopliae F52 (L.3.30), M. anisopliae ICIPE 69 (L.3.31), M. anisopliae var. acridum IMI 330189 (L.3.32); Nomuraea rileyi strains SA86101 (L.3.33), GU87401 (L.3.34), SR86151 (L.3.35), CG128 (L.3.36) and VA9101 (L.3.37); Paecilomyces fumosoroseus FE 9901 (L.3.38), P. lilacinus 251 (L.3.39), P. lilacinus DSM 15169 (L.3.40), P. lilacinus BCP2 (L.3.41), Paenibacillus popilliae Dutky-1940 (NRRL B-2309 = ATCC 14706) (L.3.42), P. popilliae KLN 3, P. popilliae Dutky 1 (L.3.43), Pasteuria spp. Ph3 (L.3.44), Pasteuria spp. ATCC PTA-9643 (L.3.45), Pasteuria spp. ATCC SD-5832 (L.3.46), P. nishizawae PN-1 (L.3.46), P. penetrans (L.3.47), P. ramose (L.3.48), P. reneformis Pr-3 (L.3.49), P. thornea (L.3.50), P. usgae (L.3.51), Pseudomonas fluorescens CL 145A (L.3.52), Steinernema carpocapsae (L.3.53), S. feltiae (L.3.54), S. kraussei L137 (L.3.55);
L4) Biochemical pesticides with insecticidal, acaricidal, molluscidal, pheromone and/or nematicidal activity: L-carvone (L.4.1), citral (L.4.2), (E,Z)-7,9-dodecadien-1-yl acetate (L.4.3), ethyl formate (L.4.4), (E,Z)-2,4-ethyl decadienoate (pear ester) (L.4.5), (Z,Z,E)-7,11,13-hexadecatrienal (L.4.6), heptyl butyrate (L.4.7), isopropyl myristate (L.4.8), cis-jasmone (L.4.9), lavanulyl senecioate (L.4.10), 2-methyl 1-butanol (L.4.11), methyl eugenol (L.4.12), methyl jasmonate (L.4.13), (E,Z)-2,13-octadecadien-1-ol (L.4.14), (E,Z)-2,13-octadecadien-1-ol acetate (L.4.15), (E,Z)-3,13-octadecadien-1-ol (L.4.16), R-1-octen-3-ol (L.4.17), pentatermanone (L.4.18), potassium silicate (L.4.19), sorbitol actanoate (L.4.20), (E,Z,Z)-3,8,11-tetradecatrienyl acetate (L.4.21), (Z,E)-9,12-tetradecadien-1-yl acetate (L.4.22), Z-7-tetradecen-2-one (L.4.23), Z-9-tetradecen-1-yl acetate (L.4.24), Z-11-tetradecenal (L.4.25), Z-11-tetradecen-1-ol (L.4.26), Acacia negra extract (L.4.27), extract of grapefruit seeds and pulp (L.4.28), extract of Chenopodium ambrosiodae (L.4.29), Catnip oil (L.4.30), Neem oil (L.4.31), Quillay extract (L.4.32), Tagetes oil (L.4.33);
L5) Microbial pesticides with plant stress reducing, plant growth regulator, plant growth promoting and/or yield enhancing activity: Azospirillum amazonense BR 11140 (SpY2^{T}) (L.5.1), A. brasilense AZ39 (L.5.2), A. brasilense XOH (L.5.3), A. brasilense BR 11005 (Sp245) (L.5.4), A. brasilense BR 11002 (L.5.5), A. lipoferum BR 11646 (Sp31) (L.5.6), A. irakense (L.5.7), A. halopraeferens (L.5.8), Bradyrhizobium sp. PNL01 (L.5.9), B. sp. (Arachis) CB1015 (L.5.10), B. sp. (Arachis) USDA 3446 (L.5.11), B. sp. (Arachis) SEMIA 6144 (L.5.12), B. sp. (Arachis) SEMIA 6462 (L.5.13), B. sp. (Arachis) SEMIA 6464 (L.5.14), B. sp. (Vigna) (L.5.15), B. elkanii SEMIA 587 (L.5.16), B. elkanii SEMIA 5019 (L.5.17), B. elkanii U-1301 (L.5.18), B. elkanii U-1302 (L.5.19), B. elkanii USDA 74 (L.5.20), B. elkanii USDA 76 (L.5.21), B. elkanii USDA 94 (L.5.22), B. elkanii USDA 3254 (L.5.23), B. japonicum 532c (L.5.24), B. japonicum CPAC 15 (L.5.25), B. japonicum E-109 (L.5.26), B. japonicum G49 (L.5.27), B. japonicum TA-11 (L.5.28), B. japonicum USDA 3 (L.5.29), B. japonicum USDA 31 (L.5.30), B. japonicum USDA 76 (L.5.31), B. japonicum USDA 110 (L.5.32), B. japonicum USDA 121 (L.5.33), B. japonicum USDA 123 (L.5.34), B. japonicum USDA 136 (L.5.35), B. japonicum SEMIA 566 (L.5.36), B. japonicum SEMIA 5079 (L.5.37), B. japonicum SEMIA 5080 (L.5.38), B. japonicum WB74 (L.5.39), B. liaoningense (L.5.40), B. lupini LL13 (L.5.41), B. lupini WU425 (L.5.42), B. lupini WSM471 (L.5.43), B. lupini WSM4024 (L.5.44), Glomus intraradices RTI-801 (L.5.45), Mesorhizobium sp. WSM1271 (L.5.46), M. sp. WSM1497 (L.5.47), M. ciceri CC1192 (L.5.48), M. huakii (L.5.49), M. loti CC829 (L.5.50), M. loti SU343 (L.5.51), Paenibacillus alvei NAS6G6 (L.5.52), Penicillium bilaiae (L.5.53), Rhizobium leguminosarum bv. phaseoli RG-B10 (L.5.54), R. I. bv. trifolii RP113-7 (L.5.55), R. I. bv. trifolii 095 (L.5.63), R. I. bv. trifolii TA1 (L.5.64), R. I. bv. trifolii CC283b (L.5.65), R. I. bv. trifolii CC275e (L.5.66), R. I. bv. trifolii CB782 (L.5.67), R. I. bv. trifolii CC1099 (L.5.68), R. I. bv. trifolii WSM1325 (L.5.69), R. I. bv. viciae SU303 (L.5.56), R. I. bv. viciae WSM1455 (L.5.57), R. I. bv. viciae P1 NP3Cst (L.5.58), R. I. bv. viciae RG-P2 (L.5.70), R. tropici SEMIA 4080 (L.5.59), R. tropici SEMIA 4077 (L.5.71), R. tropici CC511 (L.5.72), Sinorhizobium meliloti MSDJ0848 (L.5.60), S. meliloti NRG185 (L.5.61), S. meliloti RRI128 (L.5.62);
L6) Biochemical pesticides with plant stress reducing, plant growth regulator and/or plant yield enhancing activity: abscisic acid (L.6.1), aluminium silicate (kaolin) (L.6.2), 3-decen-2-one (L.6.3), formononectin (L.6.4), genistein (L.6.5), hesperetin (L.6.6), homobrassinlide (L.6.7), humates (L.6.8), methyl jasmonate (L.6.9), cis-jasmone (L.6.10), lysophosphatidyl ethanlamine (L.6.11), naringenin (L.6.12), polymeric polyhydroxy acid (L.6.13), salicylic acid (L.6.14), Ascophyllum nodosum (Norwegian kelp, Brown kelp) extract (L.6.15) and Ecklonia maxima (kelp) extract (L.6.16).

The present invention furthermore relates to agrochemical compositions comprising a mixture of XXX (component 1) and at least one biopesticide selected from the group L) (component 2), in particular at least one further fungicidal biopesticide selected from the groups L1) and L2), as described above, and if desired at least one suitable auxiliary.
Preference is also given to mixtures comprising as pesticide II (component 2) a biopesticide from group L1), preferably selected from Bacillus amyloliquefaciens AP-136 (NRRL B-50614 and B-50330), B. amyloliquefaciens AP-188 (NRRL B-50615 and B-50331), B. amyloliquefaciens AP-218 (NRRL B-50618), B. amyloliquefaciens AP-219 (NRRL B-50619 and B-50332), B. amyloliquefaciens AP-295 (NRRL B-50620 and B-50333), B. amyloliquefaciens IT-45 (CNCM I-3800), B. amyloliquefaciens subsp. plantarum MBI600 (NRRL B-50595), B. mojavensis AP-209 (NRRL B-50616), B. pumilus INR-7 (otherwise referred to as BU-F22 (NRRL B-50153) and BU-F33 (NRRL B-50185)), B. pumilus KFP9F, B. pumilus QST 2808 (NRRL B-30087), B. pumilus GHA 181, B. simplex ABU 288 (NRRL B-50340), B. solisalsi AP-217 (NRRL B-50617), B. subtilis CX-9060, B. subtilis GB03, B. subtilis GB07, B. subtilis QST-713 (NRRL B-21661), B. subtilis var. amyloliquefaciens FZB24, B. subtilis var. amyloliquefaciens D747, Paenibacillus alvei NAS6G6, Paenibacillus polymyxa PKB1 (ATCC 202127), Sphaerodes mycoparasitica IDAC 301008-01 and Trichoderma fertile J M41 R, even more preferably from Bacillus amyloliquefaciens AP-136 (NRRL B-50614), B. amyloliquefaciens AP-188 (NRRL B-50615), B. amyloliquefaciens AP-218 (NRRL B-50618), B. amyloliquefaciens AP-219 (NRRL B-50619), B. amyloliquefaciens AP-295 (NRRL B-50620), B. amyloliquefaciens IT-45 (CNCM 1-3800), B. mojavensis AP-209 (NRRL B-50616), B. pumilus INR-7 (otherwise referred to as BU-F22 (NRRL B-50153) and BU-F33 (NRRL B-50185)), B. pumilus QST 2808 (NRRL B-30087), B. simplex ABU 288 (NRRL B-50340), B. subtilis QST-713 (NRRL B-21661), B. subtilis MBI600 (NRRL B-50595), Paenibacillus alvei NAS6G6, Sphaerodes mycoparasitica IDAC 301008-01 and Trichoderma fertile JM41 R.
According to one embodiment of the inventive mixtures, the at least one pesticide II is Bacillus amyloliquefaciens subsp. plantarum MB1600. These mixtures are particularly suitable in soybean.
According to another embodiment of the inventive mixtures, the at least one pesticide II is B. pumilus strain INR-7. These mixtures are particularly suitable in soybean and corn.
According to a further embodiment, the at least one pesticide II is Bacillus simplex, preferably B. simplex strain ABU 288. These mixtures are particularly suitable in soybean and corn.
According to one embodiment of the inventive mixtures, the at least one pesticide II is selected from Bacillus amyloliquefaciens AP-136, B. amyloliquefaciens AP-188, B. amyloliquefaciens AP-218, B. amyloliquefaciens AP-219, B. amyloliquefaciens AP-295, B. amyloliquefaciens FZB42, B. amyloliquefaciens IN937a, B. amyloliquefaciens IT-45, B. amyloliquefaciens subsp. plantarum MB1600, B. mojavensis AP-209, B. pumilus GB34, B. pumilus INR-7, B. pumilus KFP9F, B. pumilus QST 2808, B. pumilus GHA 181, B. simplex ABU 288, B. solisalsi AP-217, B. subtilis CX-9060, B. subtilis GB03, B. subtilis GB07, B. subtilis QST-713, B. subtilis var. amyloliquefaciens FZB24 and B. subtilis var. amyloliquefaciens D747. These mixtures are particularly suitable in soybean and corn, in particular for seed treatment.
According to a further embodiment, the at least one pesticide II is selected from Streptomyces spp. Preferably from S. griseoviridis, S. lydicus and S. violaceusniger, in particular from strains S. griseoviridis K61, S. lydicus WYEC 108, S. violaceusniger XL-2 and S. violaceusniger YCED-9.
According to a further embodiment, the at least one pesticide II is Sphaerodes mycoparasitica, preferably Sphaerodes mycoparasitica strain IDAC 301008-01 (also referred to as strain SMCD2220-01). These mixtures are particularly suitable in soybean, cereals and corn, in particular corn especially to combat Fusarium head blight.
The present invention also relates to mixtures wherein the at least one pesticide II is selected from the following yests and fungi: Ampelomyces quisqualis, in particular strain AQ 10, Aureobasidium pullulans, in particular blastospores of strain DSM14940 or blastospores of strain DSM 14941 or mixtures thereof; Candida oleophila, in particular strains I-182 and O, Coniothyrium minitans, in particular strain CON/M/91-8; Dilophosphora alopecuri which reduces annual ryegrass toxicity (ARGT), a disease of livestock resulting from the ingestion of annual ryegrass seed-heads that have been infected by the toxin producing bacterium Rathayibacter toxicus; Gliocladium catenulatum, in particular strain J 1446; Metschnikovia fructicola, in particular strain NRRL Y-30752, Microsphaeropsis ochracea, in particular strain P130A for control of apple scab; (2.13) Muscodor albus, in particular strain QST 20799, Pichia anomala, in particular strain WRL-076, Pseudozyma flocculosa, in particular strain PF-A22 UL; Pythium oligandrum, in particular strain DV74;
The present invention also relates to mixtures wherein the at least one pesticide II is selected from the fungal genus Trichoderma, preferably from the strains Trichoderma asperellum T34, T. asperellum SKT-1, T. asperellum ICC 012, T. atroviride LC52, T. atroviride CNCM I-1237, T. fertile JM41 R, T. gamsii ICC 080, T. harmatum TH 382, T. harzianum TH-35, T. harzianum T-22, T. harzianum T-39, ; mixture of T. harzianum ICC012 and T. viride ICC080; mixture of T. polysporum and T. harzianum; T. stromaticum, T. virens GL-21, T. virens G41 and T. viride TV1; in particular T. fertile JM41 R.
The present invention also relates to mixtures wherein the at least one pesticide II is selected from the fungal genus Ulocladium, in particular U. oudemansii HRU3.

Preference is also given to mixtures comprising as pesticide II (component 2) a biopesticide from group L2), preferably selected from chitosan (hydrolysate), methyl-jasmonate, cis-jasmone, laminarin, Reynoutria sachlinensis extract and tea tree oil; even more preferable from methyl jasmonate and laminarin.

Preference is also given to mixtures comprising as pesticide II (component 2) a biopesticide from group L3), preferably selected from Agrobacterium radiobacter K1026, Bacillus firmus I-1582, Bacillus thuringiensis ssp. kurstaki SB4, Beauveria bassiana GHA, B. bassiana H123, B. bassiana DSM 12256, B. bassiana PPRI 5339, Metarhizium anisopliae var. acridum IMI 330189, M. anisopliae FI-985, M. anisopliae FI-1045, M. anisopliae F52, M. anisopliae ICIPE 69, Paecilomyces lilacinus DSM 15169, P. lilacinus BCP2, Paenibacillus popilliae Dutky-1940 (NRRL B-2309 = ATCC 14706), P. popilliae KLN 3 and P. popilliae Dutky 1, even more preferably from Bacillus thuringiensis ssp. kurstaki SB4, B. bassiana DSM 12256, B. bassiana PPRI 5339, Metarhizium anisopliae var. acridum IMI 330189, M. anisopliae FI-985, M. anisopliae FI-1045, Paecilomyces lilacinus DSM 15169, P. lilacinus BCP2, Paenibacillus popilliae Dutky-1940 (NRRL B-2309 = ATCC 14706), P. popilliae KLN 3 and P. popilliae Dutky 1.
According to a further embodiment, the at least one pesticide II is Beauveria bassiana, preferably selected from Beauveria bassiana ATCC 74040, B. bassiana GHA, B. bassiana H123, B. bassiana DSM 12256 and B. bassiana PPRI 5339, in particular Beauveria bassiana strain PPRI 5339. These mixtures are particularly suitable for wide range of arthropod pests, such as white flies, thrips, mites, aphids, tingids and all their developmental stages (eggs, immature stages, and adults) infesting numerous crops (vegetables, cucurbits, solanaceous fruits, strawberry, flowers and ornamentals, grapevine, citrus, pome, stone fruits, etc.). Recent studies have shown that these antagonistic fungal strains can effectively control also nut-weevils, wireworms (Agriotes spp.), and Tephritid flies, such as the Mediterranean fruit fly, Ceratitis capitata, the cherry fruit fly, Rhagoletis cerasi, and the olive fly, Bactrocera oleae. They are also useful in soybean and corn.
According to a further embodiment, the at least one pesticide II is Beauveria brongniartii.
According to a further embodiment, the at least one pesticide II is Metarhizium anisopliae or M. anisopliae var. acridium, preferably selected from M. anisopliae FI-1045, M. anisopliae F52, M. anisopliae var. acridum strains FI-985 and IMI 330189, in particular strain IMI 330189. These mixtures are particularly suitable for control of arthropod pests in soybean and corn.
According to a further embodiment, the at least one pesticide II is Lecanicillium sp., preferably selected from Lecanicillium longisporum KV42, L. longisporum KV71 and L. muscarium (formerly Verticillium lecanii) KV01.
According to a further embodiment, the at least one pesticide II is Paecilomyces fumosoroseus, preferably strain FE 9901 especially for white fly control.
According to a further embodiment, the at least one pesticide II is selected from Nomuraea rileyi, preferably strains SA86101, GU87401, SR86151, CG128 and VA9101; and P. lilacinus, preferably strains 251, DSM 15169 or BCP2, in particular BCP2, which strains especially control the growth of plant-pathogenic nematodes. According to a further embodiment, the at least one pesticide II is Bacillus firmus, preferably spores of strain CNCM I-1582, preferable for seed treatment of soybean and corn against nematodes and insects.
According to a further embodiment, the at least one pesticide II is B. cereus preferably spores of CNCM I-1562, preferable for seed treatment of soybean and corn against nematodes and insects.
According to a further embodiment, the at least one pesticide II is a mixture of spores of B. firmus and B. cereus, preferably mixtures spores of strains CNCM I-1582 and CNCM I-1562, preferable for seed treatment of soybean and corn against nematodes and insects.
According to a further embodiment, the at least one pesticide II is selected from Bacillus thuringiensis, preferably B. thuringiensis ssp. aizawai, in particular B. t. ssp. aizawai strains ABTS-18, SAN 401 I, ABG-6305 and ABG-6346, which are effective against different lepidopteran species including also noctuidae.
According to a further embodiment, the at least one pesticide II is selected from Bacillus t. ssp. israelensis, preferably AM65-52, SAN 402 I and ABG-6164, which are applied against larvae of various dipteran pests, e.g. mosquitoes and nematoceres. According to a further embodiment, the at least one pesticide II is selected from Bacillus t. ssp. kurstaki preferably from strains EG 2348, SB4 and ABTS-351 (HD-1), in particular B. thuringiensis ssp. kurstaki SB4. These strains are used for control of lepidopteran larvae, but without noctuidae.
According to a further embodiment, the at least one pesticide II is selected from Bacillus thuringiensis subsp. tenebrionis, preferably the strains DSM 2803, NB-125 and NB-176, in particular NB-176, which all protect plants e.g. against leaf beetle larvae.

Preference is also given to mixtures comprising as pesticide II (component 2) a biopesticide from group L4), preferably selected from methyl jasmonate, Acacia negra extract, extract of grapefruit seeds and pulp, Catnip oil, Neem oil, Quillay extract and Tagetes oil, in particular methyl jasmonate or water-based Quillay extract.

Preference is also given to mixtures comprising as pesticide II (component 2) a biopesticide from group L5), preferably selected from Azospirillum amazonense BR 11140 (SpY2^{T}), A. brasilense XOH, A. brasilense BR 11005 (Sp245), A. brasilense BR 11002, A. lipoferum BR 11646 (Sp31), A. irakense, A. halopraeferens, Bacillus amyloliquefaciens AP-136 (NRRL B-50614), Bradyrhizobium sp. (Vigna), B. japonicum USDA 3, B. japonicum USDA 31, B. japonicum USDA 76, B. japonicum USDA 110, B. japonicum USDA 121, Glomus intraradices RTI-801, Paenibacillus alvei NAS6G6, Penicillium bilaiae, Rhizobium leguminosarum bv. phaseoli, R. I. trifolii, R. I. bv. viciae, and Sinorhizobium meliloti, more preferably selected from Azospirillum brasilense BR 11005 (Sp245), Bradyrhizobium sp. (Vigna), B. japonicum USDA 3, B. japonicum USDA 31, B. japonicum USDA 76, B. japonicum USDA 110, B. japonicum USDA 121, Rhizobium leguminosarum bv. phaseoli, R. I. trifolii RP113-7, R. I. bv. viciae SU303, R. I. bv. viciae WSM1455, R. tropici SEMIA 4077, R. tropici SEMIA 4080 and Sinorhizobium meliloti.
According to another embodiment of the inventive mixtures, Bradyrhizobium sp. (meaning any Bradyrhizobium species and/or strain) as pesticide II is Bradyrhizobium japonicum (B. japonicum). These mixtures are particularly suitable in soybean. B. japonicum strains were cultivated using media and fermentation techniques known in the art, e.g. in yeast extract-mannitol broth (YEM) at 27°C for about 5 days.
The present invention also relates to mixtures, wherein the at least one pesticide II is selected from Bradyrhizobium japonicum (B. japonicum) and further comprisies a compound III, wherein compound III is selected from jasmonic acid or salts or derivatives thereof including cis-jasmone, preferably methyl-jasmonate or cis-jasmone. References for various B. japonicum strains are given e.g. in US 7,262,151 (B. japonicum strains USDA 110 (= IITA 2121, SEMIA 5032, RCR 3427, ARS I-110, Nitragin 61A89; isolated from Glycine max in Florida in 1959, Serogroup 110; Appl Environ Microbiol 60, 940-94, 1994), USDA 31 (= Nitragin 61A164; isolated from Glycine max in Wisoconsin in 1941, USA, Serogroup 31), USDA 76 (plant passage of strain USDA 74 which has been isolated from Glycine max in California, USA, in 1956, Serogroup 76), USDA 121 (isolated from Glycine max in Ohio, USA, in 1965), USDA 3 (isolated from Glycine max in Virginia, USA, in 1914, Serogroup 6), USDA 121 (Crop Science 26(5), 911-916, 1986) and USDA 136 (= CB 1809, SEMIA 586, Nitragin 61A136, RCR 3407; isolated from Glycine max in Beltsville, Maryland in 1961; Appl Environ Microbiol 60, 940-94, 1994). Further suitable B. japonicum strain G49 (INRA, Angers, France) is described in Fernandez-Flouret, D. & Cleyet-Marel, J. C. (1987) C R Acad Agric Fr 73, 163-171), especially for soybean grown in Europe, in particular in France. Further suitable B. japonicum strain TA-11 (TA11 NOD+) (NRRL B-18466) is i.a. described in US 5,021,076; Appl Environ Microbiol (1990) 56, 2399-2403 and commercially available as liquid inoculant for soybean (VAULT® NP, Becker Underwood, USA). Further B. japonicum strains as example for pesticide II are described in US2012/0252672A. Further suitable and especially in Canada commercially available strain 532c (The Nitragin Company, Milwaukee, Wisconsin, USA, field isolate from Wisconsin; Nitragin strain collection No. 61A152; Can J Plant Sci 70 (1990), 661-666) (e.g. in RHIZOFLO, HISTICK, HICOAT Super from Becker Underwood, Canada). Preferably, B. japonicum is selected from strains TA-11 and 532c, more preferably a mixture of B. japonicum strains TA-11 and 532c.
Other suitable and commercially available B. japonicum strains (see e.g. Appl Environ Microbiol 2007, 73(8), 2635) are SEMIA 566 (isolated from North American inoculant in 1966 and used in Brazilian commercial inoculants from 1966 to 1978), SEMIA 586 (= CB 1809; originally isolated in Maryland, USA but received from Austrailia in 1966 and used in Brazilian inoculants in 1977), CPAC 15 (= SEMIA 5079; a natural varaiant of SEMIA 566 used in commercial inoculants since 1992) and CPAC 7 (= SEMIA 5080; a natural variant of SEMIA 586 used in commercial inoculants since 1992). These strains are especially suitable for soybean grown in Australia or South America, in particular in Brazil. In particular, mixtures of B. japonicum SEMIA 5079 and SEMIA 5080 are suitable. Some of the abovementioned strains have been re-classified as a novel species Bradyrhizobium elkanii, e.g. strain USDA 76 (Can. J. Microbiol., 1992, 38, 501-505).
Another suitable and commercially available B. japonicum strain is E-109 (variant of strain USDA 138, see e.g. Eur. J. Soil Biol. 45 (2009) 28-35; Biol Fertil Soils (2011) 47:81-89, deposited at Agriculture Collection Laboratory of the Instituto de Microbiologia y Zoologia Agricola (IMYZA), Instituto Nacional de Tecnologi'a Agropecuaria (INTA), Castelar, Argentina). This strain is especially suitable for soybean grown in South America, in particular in Argentina.
Another suitable and commercially available B. japonicum strain are WB74 or WB74-1 (e.g. from Stimuplant CC, South Africa or from SoyGro Bio-Fertilizer Ltd, South Africa). These strains are especially suitable for soybean grown in South America and Africa, in particular in South Africa.

The present invention also relates to mixtures, wherein the at least one pesticide II is selected from Bradyrhizobium elkanii and Bradyrhizobium liaoningense (B. elkanii and B. liaoningense), more preferably from B. elkanii. These mixtures are particularly suitable in soybean. B. elkanii and liaoningense were cultivated using media and fermentation techniques known in the art, e.g. in yeast extract-mannitol broth (YEM) at 27°C for about 5 days.
The present invention also relates to mixtures wherein the at least one pesticide II is selected from selected from B. elkanii and B. liaoningense and further comprises a compound III, wherein compound III is selected from jasmonic acid or salts or derivatives thereof including cis-jasmone, preferably methyl-jasmonate or cis-jasmone. Suitable and commercially available B. elkanii strains are SEMIA 587 and SEMIA 5019 (=29W) (see e.g. Appl Environ Microbiol 2007, 73(8), 2635) and USDA 3254 and USDA 76 and USDA 94. Preferably, mixtures of B. elkanii strains SEMIA 587 and SEMIA 5019 are useful (e.g. in Gelfix 5 from Nitral Urbana Laboratories, Brazil, a BASF Company). Further commercially available B. elkanii strains are U-1301 and U-1302 (e.g. product Nitroagin® Optimize from Novozymes Bio As S.A., Brazil or NITRASEC for soybean from LAGE y Cia, Brazil). These strains are especially suitable for soybean grown in Australia or South America, in particular in Brazil.
The present invention also relates to mixtures, wherein pesticide II is selected from Bradyrhizobium sp. (Arachis) (B. sp. Arachis) which shall describe the cowpea miscellany cross-inoculation group which includes inter alia indigenous cowpea bradyrhizobia on cowpea (Vigna unguiculata), siratro (Macroptilium atropurpureum), lima bean (Phaseolus lunatus), and peanut (Arachis hypogaea). This mixture comprising as pesticide II B. sp. Arachis is especially suitable for use in peanut, Cowpea, Mung bean, Moth bean, Dune bean, Rice bean, Snake bean and Creeping vigna, in particular peanut.
The present invention also relates to mixtures wherein the at least one pesticide II is selected from B. sp. (Arachis) and further comprises a compound III, wherein compound III is selected from jasmonic acid or salts or derivatives thereof including cis-jasmone, preferably methyl-jasmonate or cis-jasmone.
Suitable and commercially available B. sp. (Arachis) strain is CB1015 (= IITA 1006, USDA 3446 presumably originally collected in India; from Australian Inoculants Research Group; see e.g. http://www.qaseeds.com.au/inoculant_applic.php). These strains are especially suitable for peanut grown in Australia, North America or South America, in particular in Brazil. Further suitable strain is Bradyrhizobium sp. PNL01 (Becker Underwood, ; Bisson and Mason, April 29, 2010, Project report, Worcester Polytechnic Institute, Worcester, MA, USA: http://www.wpi.edu/Pubs/E-project/Available/E-project-042810-163614/).
Suitable and commercially available Bradyrhizobium sp. (Arachis) strains especially for cowpea and peanut but also for soybean are Bradyrhizobium SEMIA 6144, SEMIA 6462 (= BR 3267) and SEMIA 6464 (= BR 3262; see e.g. FEMS Microbiology Letters (2010) 303(2), 123-131; Revista Brasileira de Ciencia do Solo (2011) 35(3);739-742, ISSN 0100-0683).

The present invention also relates to mixtures, wherein the at least one pesticide II is selected from Bradyrhizobium sp. (Lupine) (also called B. lupini, B. lupines or Rhizobium lupini). This mixture is especially suitable for use in dry beans and lupins. The present invention also relates to mixtures wherein the at least one pesticide II is selected from Bradyrhizobium sp. (Lupine) (B. lupini) and further comprises a compound III, wherein compound III is selected from jasmonic acid or salts or derivatives thereof including cis-jasmone, preferably methyl-jasmonate or cis-jasmone. Suitable and commercially available B. lupini strain is LL13 (isolated from Lupinus iuteus nodules from French soils; deposited at INRA, Dijon and Angers, France; http://agriculture.gouv.fr/IMG/pdf/ch20060216.pdf). This strain is especially suitable for lupins grown in Australia, North America or Europe, in particular in Europe. Further suitable and commercially available B. lupini strains WU425 (isolated in Esperance, Western Australia from a non-Australian legume Ornthopus compressus), WSM4024 (isolated from lupins in Australia by CRS during a 2005 survey) and WSM471 (isolated from Ornithopus pinnatus in Oyster Harbour, Western Australia) are described e.g. in Palta J.A. and Berger J.B. (eds), 2008, Proceedings 12th International Lupin Conference, 14-18 Sept. 2008, Fremantle, Western Australia. International Lupin Association, Canterbury, New Zealand, 47-50, ISBN 0-86476-153-8:
http://www.lupins.org/pdf/conference/2008/Agronomy%20and %20Production/John%20 Howieson%20and%20G%20OHara.pdf; Appl. Environ. Microbiol. 71, 7041-7052, 2005; Australian J. Exp. Agricult. 36(1), 63-70, 1996.
The present invention also relates to mixtures, wherein the at least one pesticide II is selected from Mesorhizobium sp. (meaning any Mesorhizobium species and/or strain), more preferably Mesorhizobium ciceri. These mixtures are particularly suitable in cowpea.
The present invention also relates to mixtures wherein the at least one pesticide II is selected from Mesorhizobium sp. and further comprises a compound III, wherein compound III is selected from jasmonic acid or salts or derivatives thereof including cis-jasmone, preferably methyl-jasmonate or cis-jasmone.
Suitable and commercially available Mesorhizobium sp. strains are e.g. M. ciceri CC1192 (=UPM 848, CECT 5549; from Horticultural Research Station, Gosford, Australia; collected in Israel from Cicer arietinum nodules; Can J Microbial (2002) 48, 279-284) and Mesorhizobium sp. strains WSM1271 (collected in Sardinia, Italy, from plant host Biserrula pelecinus), WSM 1497 (collected in Mykonos, Greece, from plant host Biserrula pelecinus), M. loti strains CC829 (commerical inoculant for Lotus pedunculatus and L. ulginosus in Australia, isolated from L. ulginosus nodules in USA; NZP 2012), M. loti SU343 (a commercial inoculant for Lotus corniculatus in Australia; isolated from host nodules in USA). For references see e.g. Soil Biol Biochem (2004) 36(8), 1309-1317; Plant and Soil (2011) 348(1-2), 231-243).
Suitable and commercially available M. loti strains are e.g. M. loti CC829 for Lotus pedunculatus.
The present invention also relates to mixtures wherein the at least one pesticide II is selected from Mesorhizobium huakuii, also referred to as Rhizobium huakuii (see e.g. Appl. Environ. Microbiol. 2011, 77(15), 5513-5516). These mixtures are particularly suitable in Astralagus, e.g. Astalagus sinicus (Chinese milkwetch), Thermopsis, e.g. Thermopsis luinoides (Goldenbanner) and alike.
The present invention also relates to mixtures wherein the at least one pesticide II is selected from Mesorhizobium huakuii and further comprises a compound III, wherein compound III is selected from jasmonic acid or salts or derivatives thereof including cis-jasmone, preferably methyl-jasmonate or cis-jasmone.
Suitable and commercially available M. huakuii strain is HN3015 which was isolated from Astralagus sinicus in a rice-growing field of Southern China (see e.g. World J. Microbiol. Biotechn. (2007) 23(6), 845-851, ISSN 0959-3993).
The present invention also relates to mixtures, wherein the at least one pesticide II is selected from Azospirillum amazonense, A. brasilense, A. lipoferum, A. irakense and A. halopraeferens, more preferably from A. brasilense, in particular selected from A. brasilense strains BR 11005 (Sp245) and AZ39 which are both commercially used in Brazil and are obtainable from EMBRAPA-Agribiologia, Brazil. These mixtures are particularly suitable in soybean.
The present invention also relates to mixtures wherein the at least one pesticide II is selected from A. amazonense, A. brasilense, A. lipoferum, A. irakense and A. halopraeferens, more preferably A. brasilense, and further comprises a compound III, wherein compound III is selected from jasmonic acid or salts or derivatives thereof including cis-jasmone, preferably methyl-jasmonate or cis-jasmone.
The present invention also relates to mixtures wherein the at least one pesticide II is selected from Rhizobium leguminosarum bv. phaseoli, especially strain RG-B10 thereof; R. I. trifolii, especially strain RP113-7 thereof, R. I. bv. viciae, in particular strains SU303, WSM1455 and P1 NP3Cst thereof; R. tropici, especially strains CC511, SEMIA 4077 and SEMIA 4080 thereof; and Sinorhizobium meliloti, especially strain MSDJ0848 thereof.
According to a further embodiment, in the inventive mixtures pesticide II is selected from one compound II selected from *Sinorhizobium meliloti* MSDJ0848, *S*. *meliloti* NRG185, *S*. *meliloti* RRI128, *S*. *meliloti* SU277, *Rhizobium leguminosarum bv. phaseoli* RG-B10, *R. leguminosarum bv. viciae* P1 NP3Cst, *R. leguminosarum bv. viciae* RG-P2, *R. leguminosarum bv. viciae* SU303, *R. leguminosarum bv. viciae* WSM1455, *R. leguminosarum bv. trifolii* RP113-7, *R. leguminosarum bv. trifolii* 095, *R*. *leguminosarum bv. trifolii* TA1, *R. leguminosarum bv. trifolii* CC283b, *R. leguminosarum bv. trifolii* CB782, *R. leguminosarum bv. trifolii* CC1099, *R. leguminosarum bv. trifolii* CC275e*, R. leguminosarum bv. trifolii* WSM1325*, R. tropici* CC511, *R*. *tropici* SEMIA 4077 and *R. tropici* SEMIA 4080.
Sinorhizobium meliloti is commercially available from Becker Underwood as product Dormal® Alfalfa & Luzerne. Rhizobium leguminosarum bv. phaseoli is commercially available from Becker Underwood as product Rhizo Stick. These strains are particulyrly suitable as inoculants for various legumes such as alfalfa, clover, peas, beans, lentils, soybeans, peanuts and others.

*Rhizobium leguminosarum bv. phaseoli,* also called *R*. *phaseoli* and recently the type I isolates being re-classified as *R*. *etli,* is commercially available from Becker Underwood, now BASF Corporation, USA, as product Rhizo-Stick for dry beans.
Particularly suitable strains especially for the legume common bean *(Phaseolus vulgaris),* but also for other crops such as corn and lettuce, are as follows: *R. leguminosarum bv. phaseoli* RG-B10 (identical to strain USDA 9041) is commercially available as NODULATOR Dry Bean in Africa, HiStick NT Dry bean in US, and NOUDLATOR Dry Bean in Canada from Becker Underwood, USA, now BASF Corporation, USA and is known from Int. J. Syst. Bacteriol. 46(1), 240-244, 1996; Int. J. Syst. Evol. Microbiol. 50, 159-170, 2000.
Further *R*. *I. bv. phaseoli* or R. *etli* strains are e.g. known from the abovementioned refences and Appl. Environ. Microbiol. 45(3), 737-742, 1983; ibida 54(5), 1280-1283, 1988.
*R*. *legominosarum bv. viciae* P1 NP3Cst (also referred to as 1435) is known from New Phytol. 179(1), 224-235, 2008; and e.g. in NODULATOR PL Peat Granule from Becker Underwood, USA; or in NODULATOR XL PL from Becker Underwood, Canada). *R*. *leguminosarum bv. viciae* RG-P2 (also called P2) is commercially available as inoculant for pean and lentils as RhizUP peat in Canada from Becker Underwood, Canada now BASF Agricultural Specialties Ltd., Canada. *R*. *leguminosarum bv. viciae* WSM1455 is commercially available NODULAID for faba beans peat from Becker Underwood, Australia now BASF Agricultural Specialties Ltd., Australia. *R. leguminosarum bv. viciae* SU303 is commercially available as NODULAID Group E, NODULAID NT peat or NODULATOR granules for peas from Becker Underwood, Australia now BASF Agricultural Specialties Ltd., Australia. *R*. *leguminosarum bv. viciae* WSM1455 is commercially available as NODULAID Group F peat, NODULAID NT and NODULATOR granules for faba bean from Becker Underwood, Australia, now BASF Agricultural Specialties, Australia, and is also as inoculant for faba beans as NODULATOR SA faba bean in Canada or as Faba Sterile Peat in Europe or as NODULATOR faba bean granules in Canada from Becker Underwood, Canada now BASF Agricultural Specialties Ltd., Canada.
*Rhizobium leguminosarum bv. trifolii* is commercially available from Becker Underwood as product Nodulator or DORMAL true clover. Suitable strains are especially useful for all kind of clovers, are as follows: *R. legominosarum bv. trifolii* strains RP113-7 (also called 113-7) and 095 are commercially available from Becker Underwood, USA, now BASF Corporation, USA; see also Appl. Environ. Microbiol. 44(5), 1096-1101. Suitable strain *R. legominosarum bv. trifolii* TA1 obtained from Australia is known from Appl. Environ. Microbiol. 49(1), 127-131, 1985 and commercially available as NODULAID peat for white clover from Becker Underwood, Australia, now BASF Agricultural Specialties, Australia. *R*. *leguminosarum bv. trifolii* CC283b is commercially available as NODULAID peat for Caucasian clover from Becker Underwood, Australia, now BASF Agricultural Specialties, Australia. *R. leguminosarum bv. trifolii* CC1 099 is commercially available as NODULAID peat for sainfoin from Becker Underwood, Australia, now BASF Agricultural Specialties, Australia. *R*. *leguminosarum bv. trifolii* CC275e is commercially available as NODULAID peat for NZ white clover from Becker Underwood, Australia, now BASF Agricultural Specialties, Australia. *R. leguminosarum bv. trifolii* CB782 is commercially available as NODULAID peat for Kenya white clover from Becker Underwood, Australia, now BASF Agricultural Specialties, Australia. *R. legominosarum bv. trifolii* strain WSM1325 has been collected in 1993 from the Greek Island of Serifos, is commercially available in NODULAID peat for sub clover and NODULATOR granules for sub clover both from Becker Underwood, Australia, now BASF Agricultural Specialties, Australia for a broad range of annual clovers of Mediterranean origin, and is known from Stand. Genomic Sci. 2(3), 347-356, 2010. *R. legominosarum bv. trifolii* strain WSM2304 has been isolated from *Trifolium polymorphum* in Uruguay in 1998 and is known from Stand. Genomic Sci. 2(1), 66-76, 2010, and is particularly suitable to nodulate its clover host in Uruguay.
*R. tropici* is useful for a range of legume crops especially in tropical regions such as Brazil. Suitable strains are especially useful for all kind of clovers, are as follows: *R. tropici* strain SEMIA 4080 (identical to PRF 81; known from Soil Biology & Biochemistry 39, 867-876, 2007; BMC Microbiol. 12:84, 2012) is commercially available in NITRAFIX FEIJÃO peat for beans from BASF Agricultural Specialties, Brazil and has been used as commercial inoculant for applications to common bean crops in Brazil since 1998, and is deposited with FEPAGRO-Fundação Estadual de Pesquisa Agropecuária, Rua Gonçalves Dias, 570, Bairro Menino Deus, Porto Alegre/RS, Brazil. *R. tropici* is useful for a range of legume crops especially in tropical regions such as Brazil. Suitable strains are especially useful for all kind of clovers, are as follows: R. *tropici* strain SEMIA 4077 (identical to CIAT899; Rev. Ciênc. Agron. 44(4) Fortaleza Oct./Dec. 2013) is commercially available in NITRAFIX FEIJÃO peat for beans from BASF Agricultural Specialties, Brazil. *R. tropici* strain CC511 is commercially available as NODULAID peat for common bean from Becker Underwood, Australia, now BASF Agricultural Speciealties, Australia, and is known from Agronomy, N.Z. 36, 4-35, 2006.
The present invention also relates to mixtures wherein the at least one pesticide II is selected from R. leguminosarum bv. phaseoli, R. I. trifolii, R. I. bv. viciae, R. tropici and Sinorhizobium meliloti, and further comprises a compound III, wherein compound III is selected from jasmonic acid or salts or derivatives thereof including cis-jasmone, preferably methyl-jasmonate or cis-jasmone.
According to a further embodiment, the at least one pesticide II is selected from Delftia acidovorans, in particular strain RAY209, especially in soybean and canola.
According to a further embodiment, the at least one pesticide II is selected from Lysobacter spp., preferably selected from L.antibioticus, in particular strains 13-1 and HS124, preferably in rice or pepper for control of Phytophthora or bacterial leaf blight.
According to a further embodiment, the at least one pesticide II is selected from L. enzymogenes, in particular strain 3.1T8.
According to a further embodiment, the at least one pesticide II is selected from Lysobacter spp., preferably selected from Pseudomonas spp., in particular strain MA 342 and Pseudomonas sp. DSM 13134.
According to a further embodiment, the at least one pesticide II is selected from Penicillium bilaiae.

Preference is also given to mixtures comprising as pesticide II (component 2) a biopesticide from group L6), preferably selected from abscisic acid, aluminium silicate (kaolin), humates, Ascophyllum nodosum (Norwegian kelp, Brown kelp) extract and Ecklonia maxima (kelp) extract.
Preference is also given to mixtures comprising as pesticide II a biopesticide selected from the isoflavones formonennitin, hesperetin and naringenin.

Accordingly, the present invention furthermore relates to compositions comprising one compound I (component 1) and one further pesticide II (component 2), which further pesticide II is selected from the column "Co. 2" of the lines C-1 to C-830 of Table C.
A further embodiment relates to the compositions C-1 to C-830 listed in Table C, where a row of Table C corresponds in each case to a fungicidal composition comprising as active component one of the in the present specification individualized compounds of formula I (component 1) and the respective further pesticide II from groups A) to O) (component 2) stated in the row in question. Preferably, the compositions described comprise the active components in synergistically effective amounts.

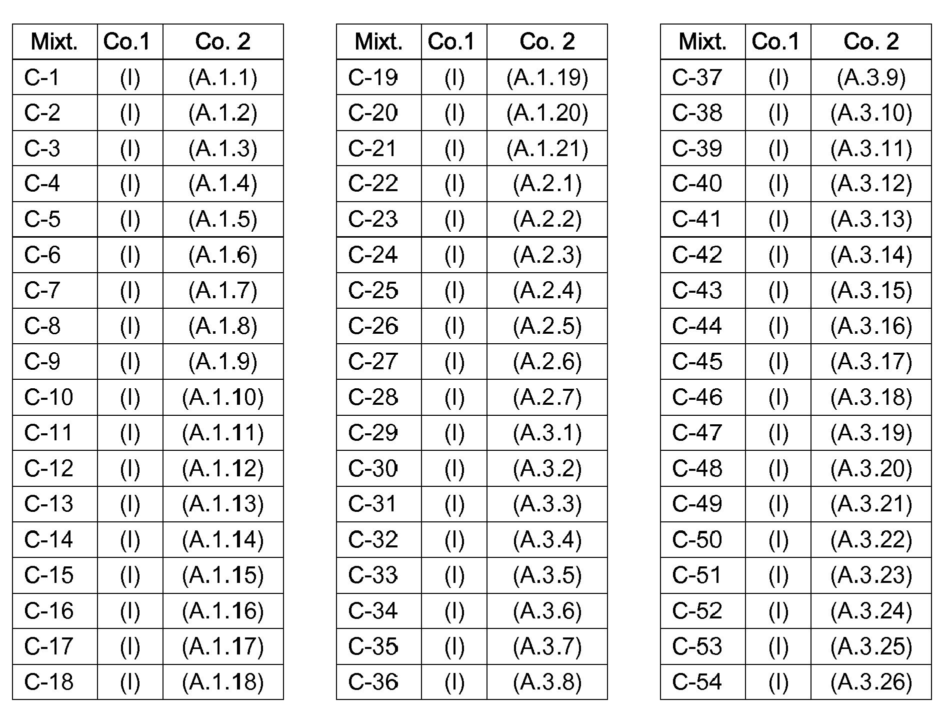

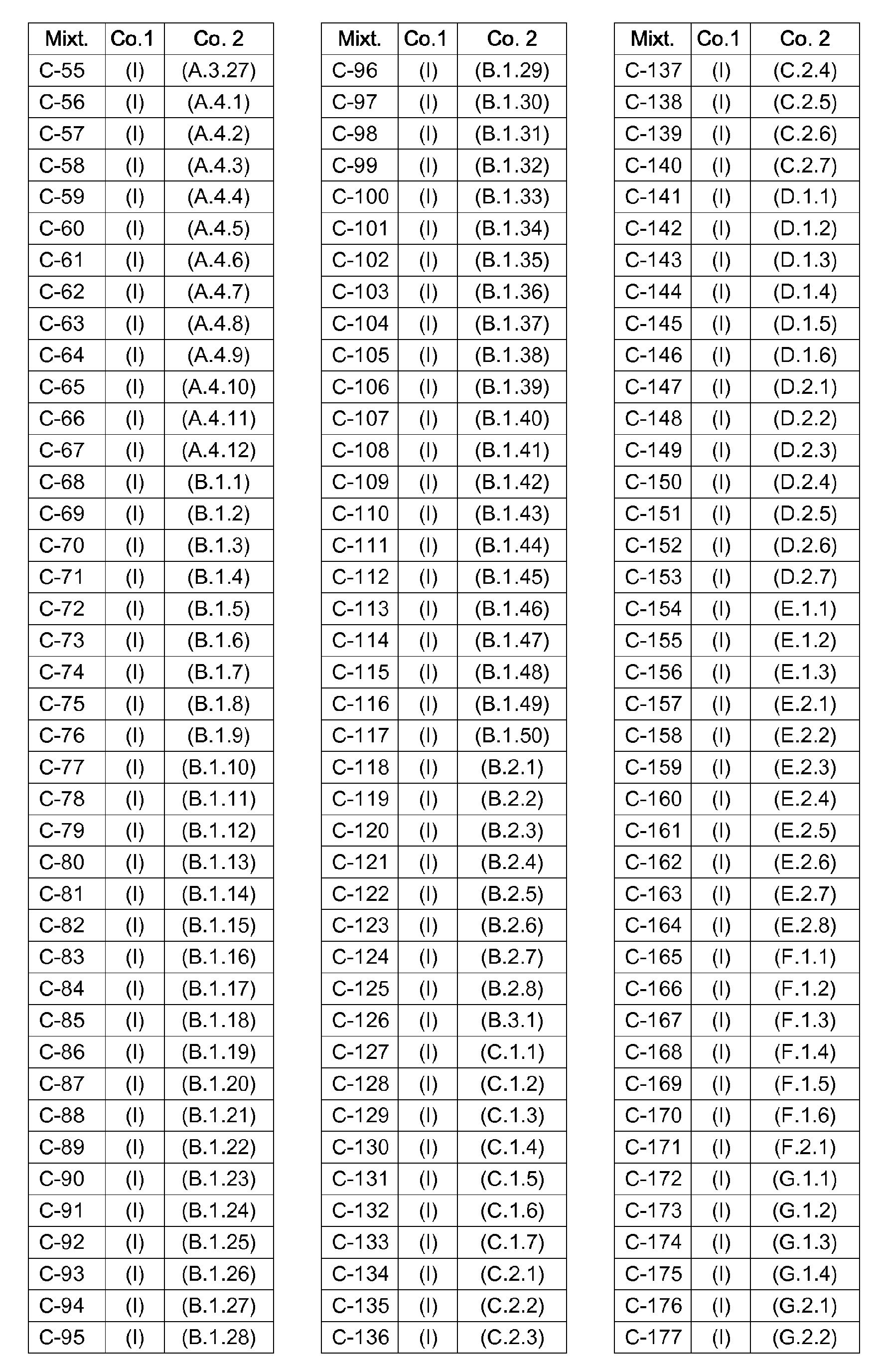

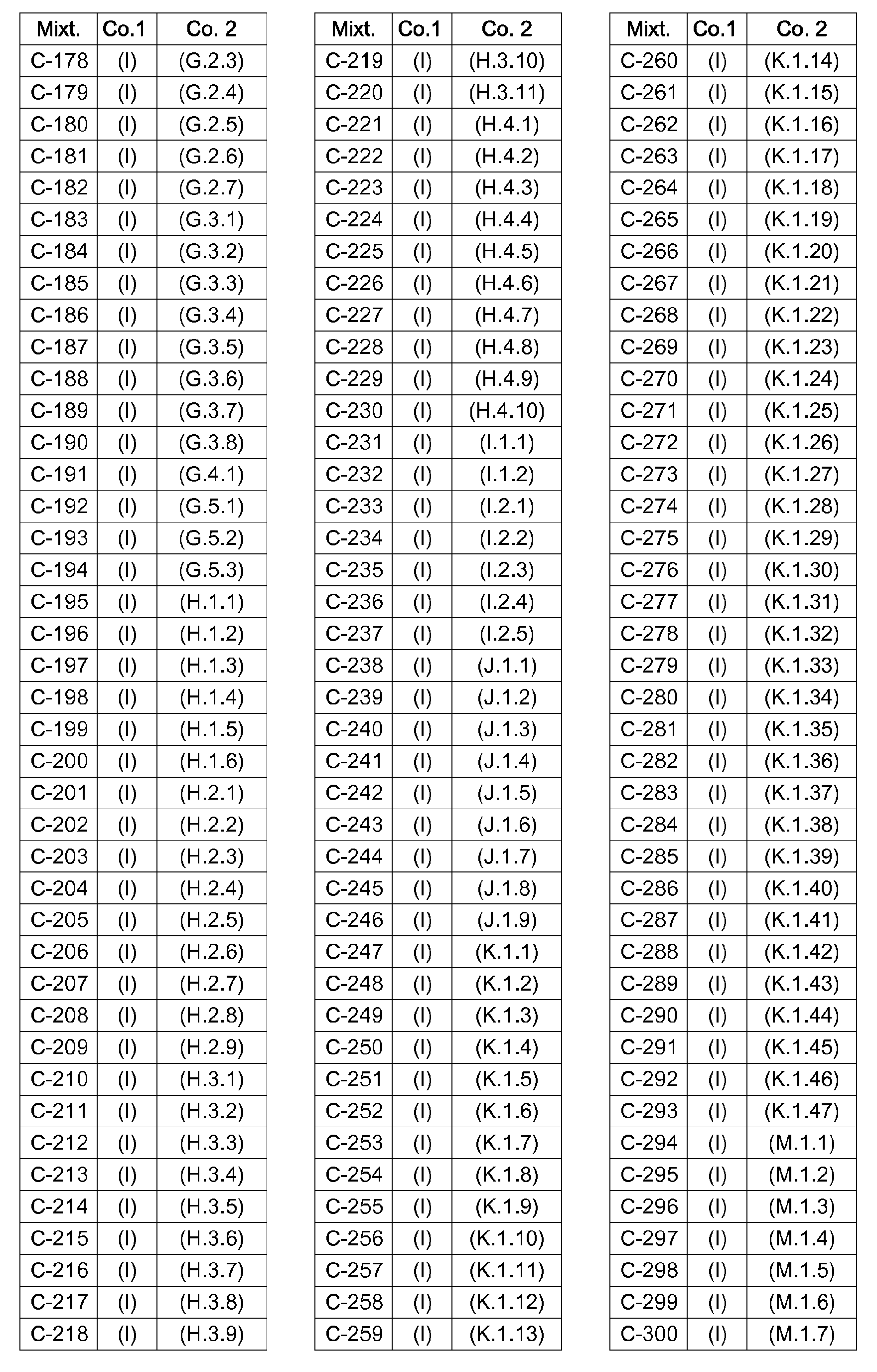

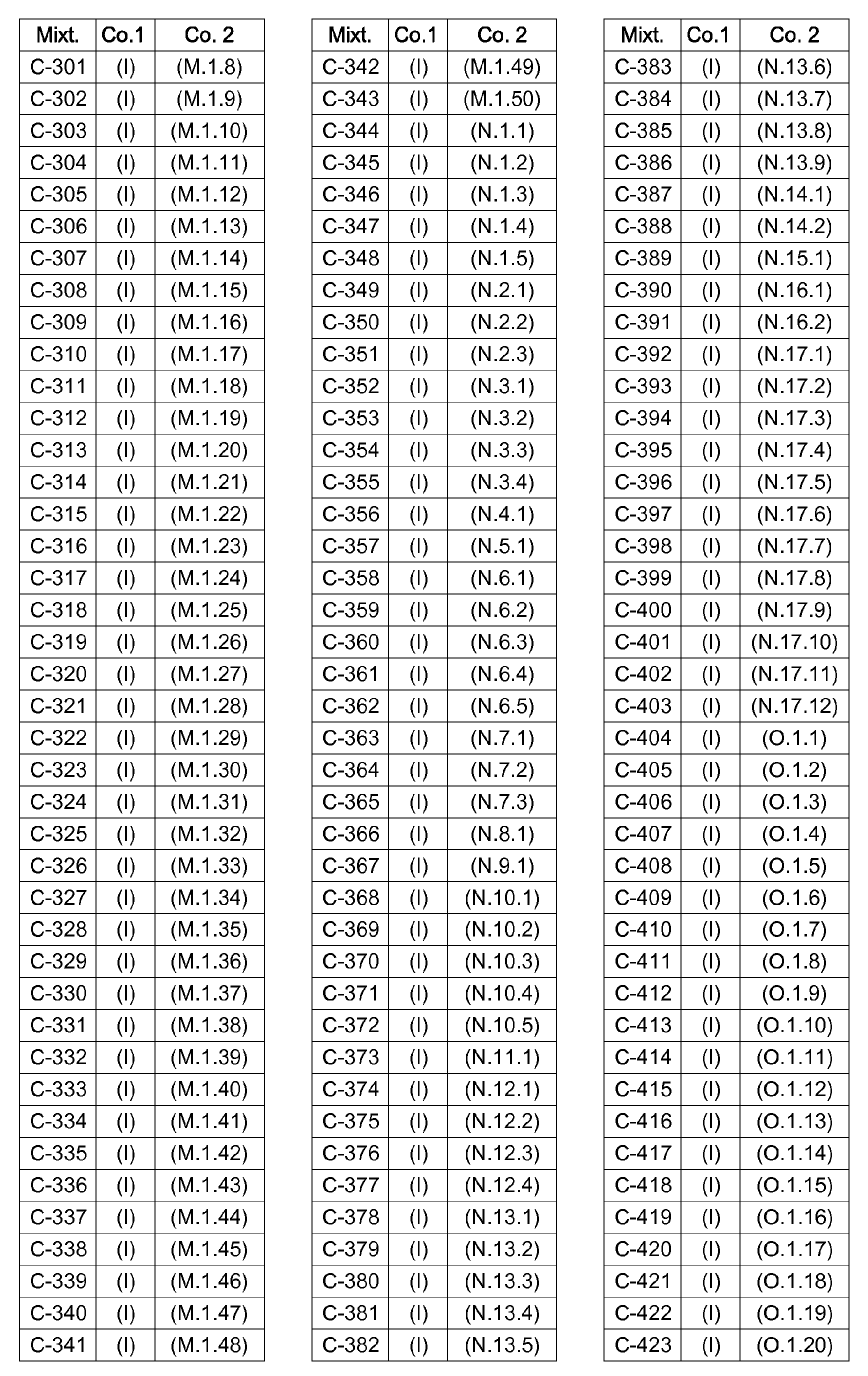

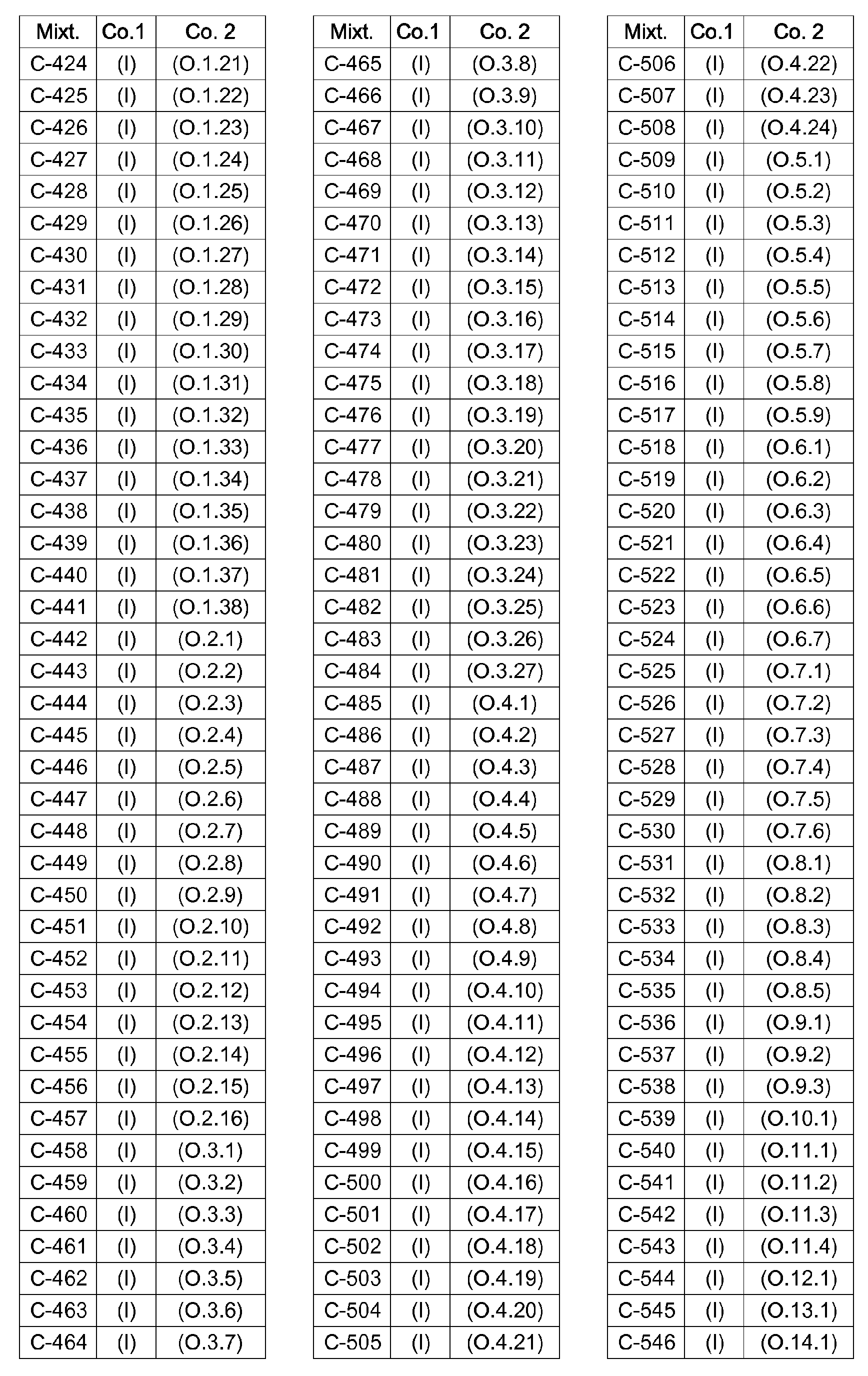

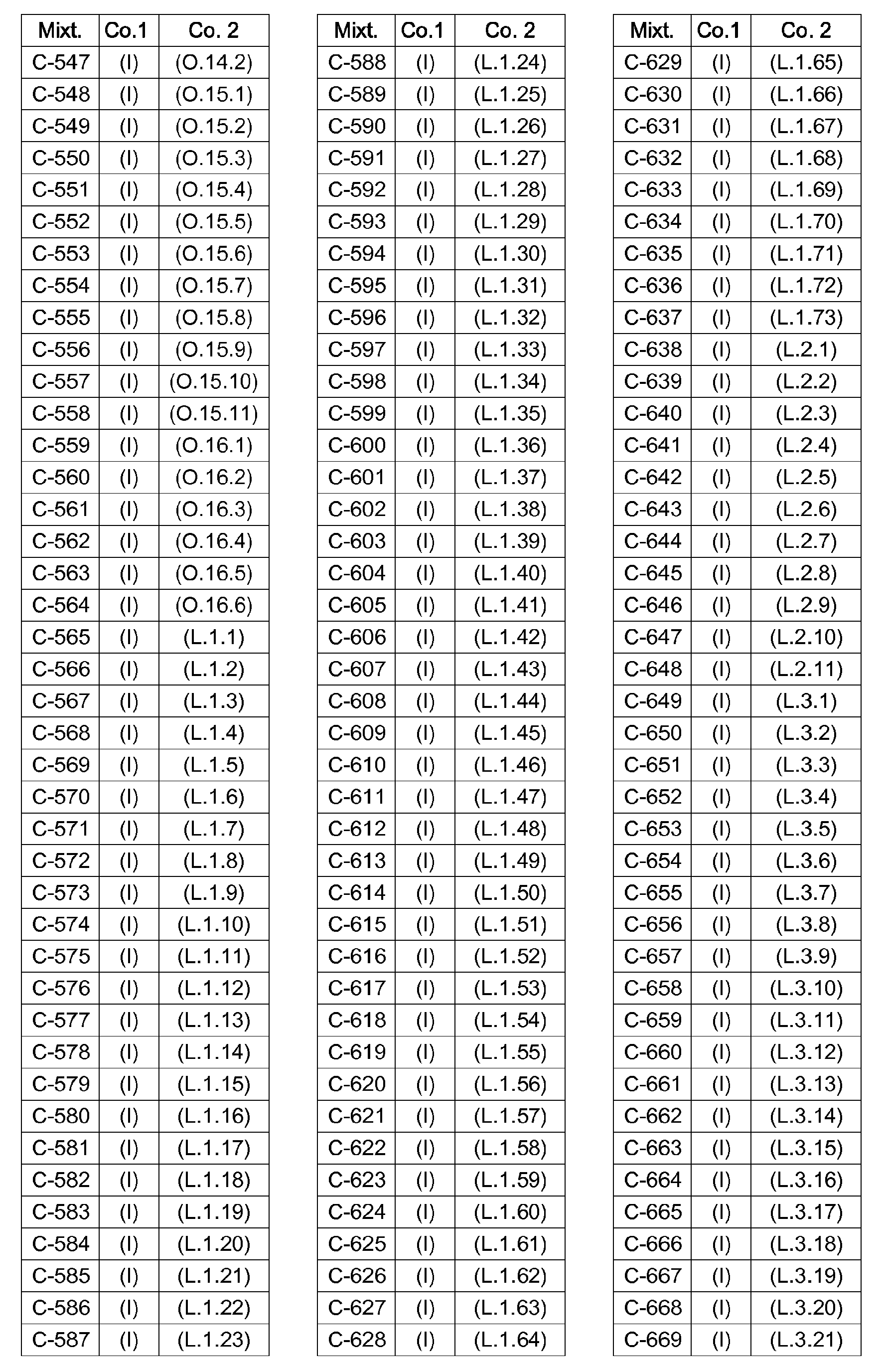

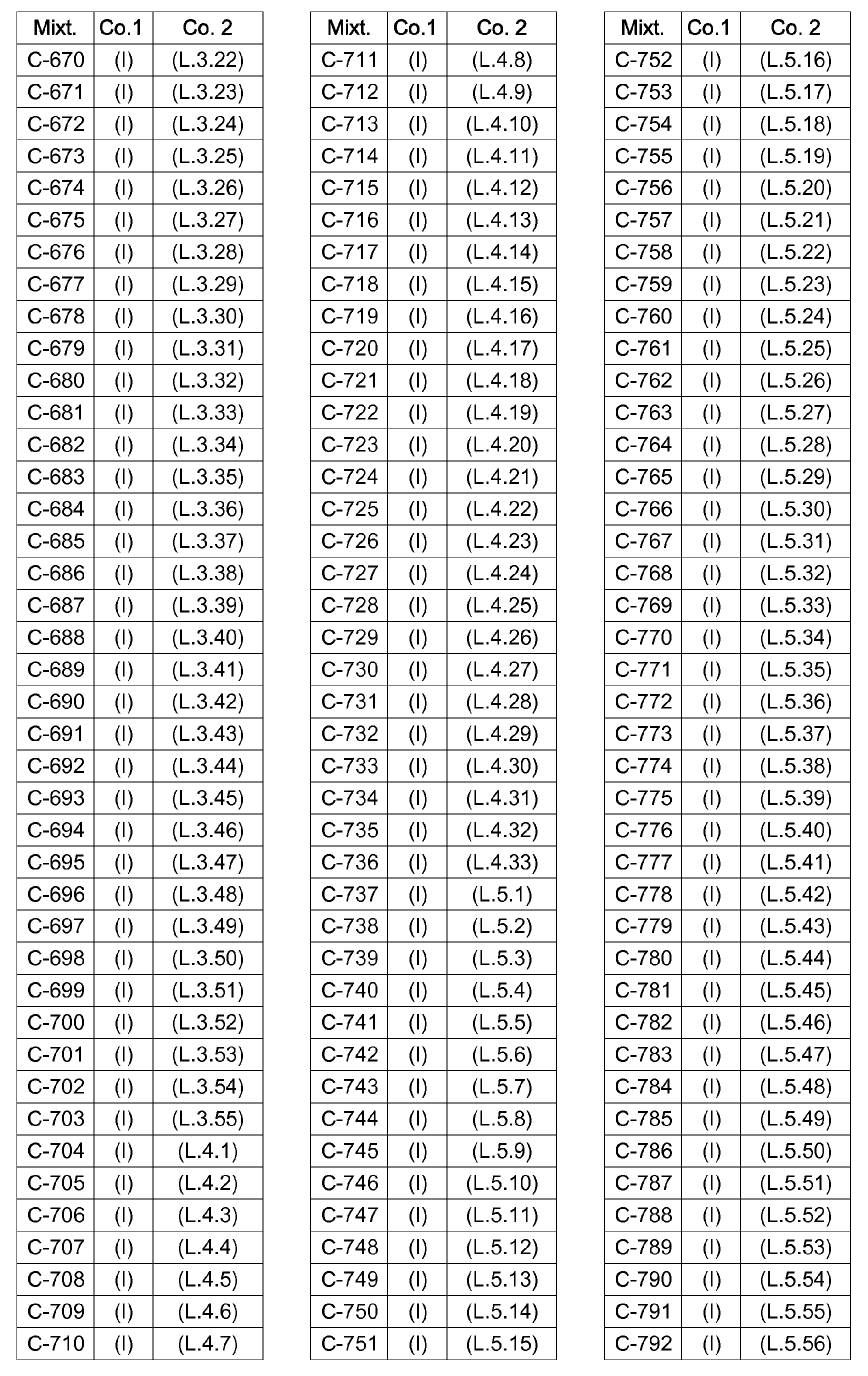

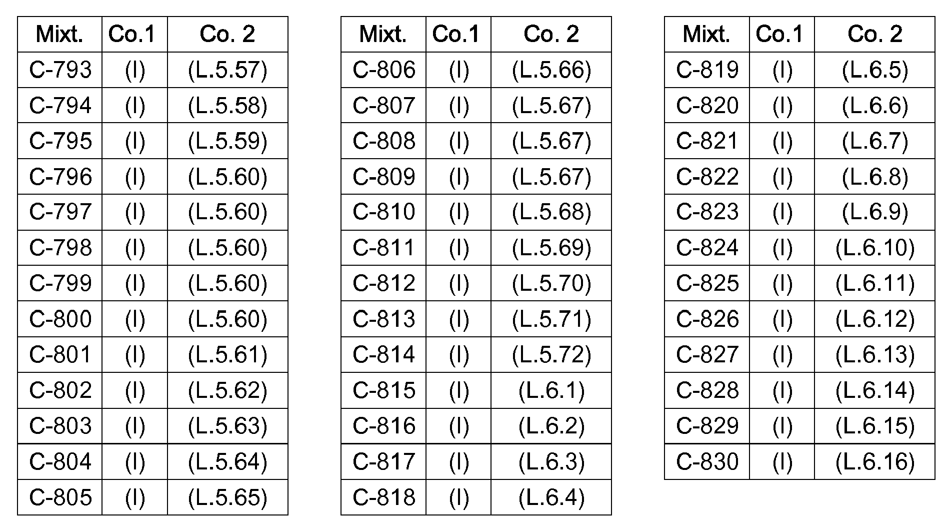

The active substances referred to as component 2, their preparation and their activity e.g. against harmful fungi is known (cf.: hftp://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657, WO2012/168188, WO 2007/006670, WO 2011/77514; WO13/047749, WO 10/069882, WO 13/047441, WO 03/16303, WO 09/90181, WO 13/007767, WO 13/010862, WO 13/127704, WO 13/024009 and WO 13/024010.

The mixtures of active substances can be prepared as compositions comprising besides the active ingredients at least one inert ingredient (auxiliary) by usual means, e. g. by the means given for the compositions of compounds I.
Concerning usual ingredients of such compositions reference is made to the explanations given for the compositions containing compounds I.

The mixtures of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.
According to one embodiment, the microbial pesticides selected from groups L1), L3) and L5) embrace not only the isolated, pure cultures of the respective micro-organism as defined herein, but also its cell-free extract, its suspensions in a whole broth culture or as a metabolite-containing supernatant or a purified metabolite obtained from a whole broth culture of the microorganism or microorganism strain.
According to a further embodiment, the microbial pesticides selected from groups L1), L3 and L5) embraces not only the isolated, pure cultures of the respective micro-organism as defined herein, but also a cell-free extract thereof or at least one metabolite thereof, and/or a mutant of the respective micro-organism having all the identifying characteristics thereof and also a cell-free extract or at least one metabolite of the mutant.
"Whole broth culture" refers to a liquid culture containing both cells and media. "Supernatant" refers to the liquid broth remaining when cells grown in broth are removed by centrifugation, filtration, sedimentation, or other means well known in the art.
The term "cell-free extract" refers to an extract of the vegetative cells, spores and/or the whole culture broth of a microorganism comprising cellular metabolites produced by the respective microorganism obtainable by cell disruption methods known in the art such as solvent-based (e.g. organic solvents such as alcohols sometimesin combination with suitable salts), temperature-based, application of shear forces, cell disrupotion with an ultrasonicator. The desired extract may be concentrated by conventional concentration techniques such as drying, evaporation, centrifugation or alike. Certain washing steps using organic solents and/or water-based media may also be applied to the crude extract preferably prior to use.
The term "metabolite" refers to any compound, substance or byproduct produced by a microorganism (such as fungi and bacteria) that has improves plant growth, water use efficiency of the plant, plant health, plant appearance, or the population of beneficial microorganisms in the soil around the plant activity.
The term "mutant" refers a microorganism obtained by direct mutant selection but also includes microorganisms that have been further mutagenized or otherwise manipulated (e.g., via the introduction of a plasmid). Accordingly, embodiments include mutants, variants, and or derivatives of the respective microorganism, both naturally occurring and artificially induced mutants. For example, mutants may be induced by subjecting the microorganism to known mutagens, such as N-methyl-nitrosoguanidine, using conventional methods.
Suitable bactericides are bronopol and isothiazolinone derivatives such as alkylisothiazolinones and benzisothiazolinones. Suitable anti-freezing agents are ethylene glycol, propylene glycol, urea and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants). Suitable tackifiers or binders are polyvinylpyrrolidons, polyvinylacetates, polyvinyl alcohols, polyacrylates, biological or synthetic waxes, and cellulose ethers.
In the case of mixtures comprising microbial pesticides II selected from groups L1), L3) and L5), the microorganisms as used according to the invention can be cultivated continuously or discontinuously in the batch process or in the fed batch or repeated fed batch process. A review of known methods of cultivation will be found in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).
When living microorganisms, such as pesticides II from groups L1), L3) and L5), form part of the compositions, such compositions can be prepared as compositions comprising besides the active ingredients at least one auxiliary (inert ingredient) by usual means (see e.g. H.D. Burges: Formulation of Micobial Biopestcides, Springer, 1998). Suitable customary types of such compositions are suspensions, dusts, powders, pastes, granules, pressings, capsules, and mixtures thereof. Examples for composition types are suspensions (e.g. SC, OD, FS), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). Herein, it has to be taken into account that each formulation type or choice of auxiliary should not influence the viability of the microorganism during storage of thecomposition and when finally applied to the soil, plant or plant propagation material. Suitable formulations are e.g. mentioned in WO 2008/002371, US 6955,912, US 5,422,107.
Examples for suitable auxiliaries are those mentioned earlier herein, wherein it must be taken care that choice and amounts of such auxiliaries should not influence the viability of the microbial pesticides in the composition. Especially for bactericides and solvents, compatibility with the respective microorganism of the respective microbial pesticide has to be taken into account. In addition, compositions with microbial pesticides may further contain stabilizers or nutrients and UV protectants. Suitable stabilzers or nutrients are e.g. alpha-tocopherol, trehalose, glutamate, potassium sorbate, various sugars like glucose, sucrose, lactose and maltodextrine (H.D. Burges: Formulation of Micobial Biopestcides, Springer, 1998). Suitable UV protectants are e.g. inorganic compouns like titan dioxide, zinc oxide and iron oxide pigments or organic compounds like benzophenones, benzotriazoles and phenyltriazines. The compositions may in addition to auxiliaries mentioned for compositions comprising compounds I herein optionally comprise 0.1 - 80% stabilizers or nutrients and 0.1-10% UV protectants.

When mixtures comprising microbial pesticides are employed in crop protection, the application rates preferably range from about 1 x 10⁶ to 5 x 10¹⁵ (or more) CFU/ha. Preferably, the spore concentration is about 1 x 10⁷ to about 1 x 10¹¹ CFU/ha. In the case of (entomopathogenic) nematodes as microbial pesticides (e.g. Steinernema feltiae), the application rates preferably range inform about 1 x 10⁵ to 1 x 10¹² (or more), more preferably from 1 x 10⁸ to 1 x 10¹¹, even more preferably from 5 x 10⁸ to 1 x 10¹⁰ individuals (e.g. in the form of eggs, juvenile or any other live stages, preferably in an infetive juvenile stage) per ha.
When mixtures comprising microbial pesticides are employed in seed treatment, the application rates with respect to plant propagation material preferably range from about 1 x 10⁶ to 1 x 10¹² (or more) CFU/seed. Preferably, the concentration is about 1 x 10⁶ to about 1 x 10¹¹ CFU/seed. In the case of the microbial pesticides II, the application rates with respect to plant propagation material also preferably range from about 1 x 10⁷ to 1 x 10¹⁴ (or more) CFU per 100 kg of seed, preferably from 1 x 10⁹ to about 1 x 10¹¹ CFU per 100 kg of seed.

## Claims

1. Compounds of formula I 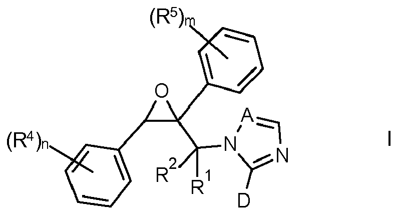 wherein:
A is N or CH;
D is hydrogen, halogen, SR^{D}; wherein
R^{D} is hydrogen, CN, C₁-C₆-alkyl, C₁-C₆-haloalkyl, C₂-C₆-alkenyl, C₂-C₆-haloalkenyl, C₂-C₆-alkynyl or C₂-C₆-haloalkynyl;
R¹ is halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R¹ are unsubstituted or substituted by one, two, three or four or up to the maximum possible number of independently selected substituents R^{1a}; wherein
R^{1a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl;
R² is hydrogen, halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl or heteroaryl-C₁-C₄-alkyl, wherein the heteroaryl group in the last-mentioned groups is 5- or 6-membered; and wherein the aliphatic, alicyclic and aromatic moieties of R² are unsubstituted or substituted by one, two, three or four or up to the maximum possible number of independently selected substituents R^{2a}; wherein
R^{2a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl;
or R¹ and R² together with the carbon atom to which they are bound (denominated as C*) form a vinyl group C*=CR¹¹R²², wherein
R¹¹ and R²² are independently selected from the group consisting of hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
or R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle includes beside carbon atoms one, two, three or four heteroatoms independently selected from the group consisting of N, O and S, and wherein the carbo- and heterocycle are unsubstituted or carry one, two, three or four independently selected substituents R¹², wherein one or two CH₂ groups of the carbo- or heterocycle may be replaced by one or two groups independently selected from the group of C(=O) and C(=S); wherein
R¹² is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl or phenoxy; wherein the phenyl moieties of R¹² are unsubstituted or substituted by one, two, three or four independently selected substituents R^{12a}; wherein
R^{12a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl;
R⁴ and R⁵ are independently selected from the group consisting of halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, phenyl, phenoxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic and alicyclic moieties of R⁴ are unsubstituted or substituted by one, two, three or four or up to the maximum possible number of independently selected substituents R^{4a}; wherein
R^{4a} is halogen, CN, NO₂, OH, C₁-C₄-alkyl, C₁-C₄-haloalkyl, C₃-C₈-cycloalkyl, C₁-C₄-alkoxy or C₁-C₄-halogenalkoxy;
n is 0, 1, 2, 3 or 4;
m is 0, 1, 2, 3 or 4;
and the N-oxides and the agriculturally acceptable salts of the compounds of formula I.

2. Compounds according to claim 1, wherein A is N.

3. Compounds according to claim 1 or 2, wherein D is hydrogen.

4. Compounds according to any of claims 1 to 3, wherein R¹ is halogen, CN, NO₂, OH, NH₂, N(CH₃)₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl or C₂-C₆-alkynyl or C₃-C₈-cycloalkyl, and R² is a radical as defined for R¹ or hydrogen.

5. Compounds according to any of claims 1 to 4, wherein R¹ is CN.

6. Compounds according to any of claims 1 to 5, wherein R¹ is OH, SH or NH₂.

7. Compounds according to any of claims 1 to 6, wherein R¹ is C₃-C₈-cycloalkyl, phenyl or 5- or 6-membered heteroaryl, wherein the alicyclic and aromatic moieties of R¹ are unsubstituted or substituted by one, two, three or four or up to the maximum possible number of independently selected substituents R^{1a}.

8. Compounds according to any of claims 1 to 7, wherein R¹ and R² together with the carbon atom to which they are bound (denominated as C*) form a vinyl group C*=CR¹¹R²², wherein R¹¹ and R²² are as defined or preferably defined below; or R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle includes beside carbon atoms one, two, three or four heteroatoms independently selected from the group consisting of N, O and S, and wherein the carbo- and heterocycle are unsubstituted or carry one, two, three or four independently selected substituents R¹² as defined and preferably defined below, and wherein one or two CH₂ groups of the carbo- or heterocycle may be replaced by one or two groups independently selected from the group of C(=O) and C(=S).

9. Compounds according to any one of claims 1 to 8, wherein n is 1, 2, 3 or 4 and one radical R⁴ is located in ortho-position of the phenyl ring with respect to the oxirane residue and wherein m is 1, 2, 3 or 4 and one radical R⁵ is located in para-position of the phenyl ring with respect to the oxirane residue.

10. Compounds according to any one of claims 1 to 9, wherein R⁴ and R⁵ are independently selected from the group consisting of halogen, CN, NO₂, OH, SH, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl, C₂-C₆-haloalkenyl, C₂-C₆-haloalkynyl, C₃-C₈-cycloalkyl, C₃-C₈-cycloalkyloxy and C₃-C₈-cycloalkyl-C₁-C₄-alkyl.

11. An agrochemical composition which comprises an auxiliary and at least one compound of formula I or an N-oxide or an agriculturally acceptable salt thereof, according to any of claims 1 to 10.

12. An agrochemical composition according to claim 11 comprising at least one further active substance.

13. A method for combating phytopathogenic fungi, comprising treating the fungi or the materials, plants, the soil or seeds to be protected against fungal attack, with an effective amount of at least one compound of formula I or an N-oxide or an agriculturally acceptable salt thereof, according to any of claims 1 to 10.

14. A use of compounds of formula I, their N-oxides or their agriculturally acceptable salts, according to any of claims 1 to 10 for combating phytopathogenic fungi.

15. Seed treated with a compound of formula I, or an N-oxide or an agriculturally acceptable salt thereof, as defined in any of claims 1 to 10, in an amount of from 0.1 g to 10 kg per 100 kg of seed.
